# EUROPEAN PATENT APPLICATION

(11) **EP 4 310 091 A1**
(43) Date of publication of application: **24.01.2024**
(21) Application number: 22770598.5
(22) Date of filing: 17.03.2022
(51) Int. Cl.: C07D 498/14, C07D 471/00, C07D 471/02, A61K 31/55, A61P 35/00

(54) **PYRIMIDINE-FUSED CYCLIC COMPOUND, PREPARATION METHOD THEREFOR AND USE THEREOF**

(30) Priority: 17.03.2021 CN 202110285465
(71) Applicant: Genfleet Therapeutics (Shanghai) Inc., Shanghai 201203 (CN); Zhejiang Genfleet Therapeutics Co., Ltd., Shaoxing Binhai New City, Zhejiang 312000 (CN)
(72) Inventor: JIANG, Tao, Shanghai 201203 (CN); ZHOU, Fusheng, Shanghai 201203 (CN); ZHANG, Leitao, Shanghai 201203 (CN); CAI, Lijian, Shanghai 201203 (CN); YAN, Feng, Shanghai 201203 (CN); ZHAO, Jichen, Shanghai 201203 (CN); LAN, Jiong, Shanghai 201203 (CN); LU, Qiang, Shanghai 201203 (CN)
(74) Representative: Elzaburu S.L.P.
(86) International application number: PCT/CN2022/081443
(87) International publication number: WO 2022/194245

(57) **Abstract**

Disclosed are a pyrimidine-fused cyclic compound having an inhibitory effect on KRAS gene mutation, or a pharmaceutically acceptable salt, a stereoisomer, a solvate or a prodrug thereof, and a pharmaceutical composition containing the compound, and the use thereof in the preparation of a drug for treating cancer.

## Description

### TECHNICAL FIELD

The present disclosure relates to the field of medicine, and in particular, to pyrimidine-fused cyclic compounds, preparation methods therefor, and use thereof.

### BACKGROUND

KRAS is a member of 21kD protein of the Ras family of GTP enzyme proteins, and is an essential component for cell signaling. The role of KRAS in malignant tumors and mutations in various tumor types (e.g. G12C mutation, G12D mutation, G12V mutation, etc.) are known to the public, and thus KRAS has become a very attractive target for cancer therapy in the pharmaceutical industry. Compounds that inhibit KRAS activity are highly desirable to researchers in the art and are under sizzling research. At present, breakthroughs have been made in the art for KRAS G12C, e.g. KRAS G12C inhibitors of AMG, MIRATI have shown to beadequate in safety and efficacy. However, there is a continuing interest and effort to develop inhibitors of KRAS, in particular inhibitors for activating KRAS mutants, especially KRAS G12D.

### SUMMARY OF THE INVENTION

The present disclosure provides a novel pyrimidine-fused cyclic compound, which is used as a KRAS G12D inhibitor with high activity, good selectivity, low toxic and side effects, etc.

In a first aspect, the present disclosure provides a compound, or a pharmaceutically acceptable salt, a stereoisomer, a solvate or a prodrug thereof as an inhibitor of KRAS G12D, wherein the compound is a compound shown in formula (I), formula (II-1), formula (II-2), or formula (III).

In an embodiment, the present disclosure provides a compound shown in formula (I), or a pharmaceutically acceptable salt, a stereoisomer, a solvate, or a prodrug thereof: 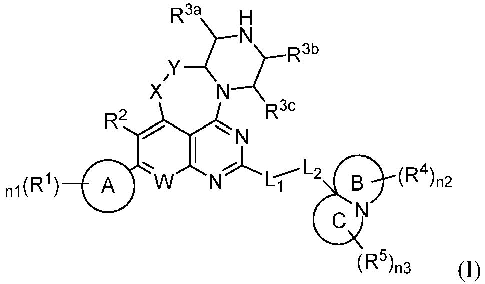 in the formula (I),
X is O or NR¹¹; where R¹¹ is selected from H and C1-C6 alkyl;
Y is CR¹²R¹³, CR¹⁴R¹⁵CR¹⁶R¹⁷, C(O) or C(O)CR¹⁸R¹⁹, where R¹², R¹³, R¹⁴ and R¹⁵ are each independently selected from H, C1-C3 alkyl, C1-C3 deuterated alkyl, and C1-C3 halogenated alkyl; R¹⁶, R¹⁷, R¹⁸, R¹⁹ are each independently selected from H, C1-C3 alkyl and halogen;
W is N or CR²⁰ ; where R²⁰ is H, C1-C6 alkyl, halogen, C1-C6 halogenated alkyl, C1-C6 alkoxyl, cycloalkyl, cycloalkyl-O-, heterocyclyl or heterocyclyl-O-; where the cycloalkyl and the heterocyclyl are each independently optionally substituted with halogen;
ring A is selected from a group consisting of: aryl, heteroaryl;
R¹ is a substituent at any position on ring A, each R¹ is independently selected from: C1-C6 alkyl, halogen, hydroxyl, C1-C6 alkoxyl, C1-C6 halogenated alkyl, C1-C6 halogenated alkoxyl, cyano, NR²¹R²², C(O)NR²³R²⁴, CH₂R²⁵, N=S(O)(C1-C6 alkyl)₂, S(O)C1-C6 alkyl, S (O)₂R²⁶, -S-C1-C6 alkyl, C2-C6 alkenyl, C2-C6 alkynyl, C2-C6 hydroxyalkynyl, C1-C6 cyanoalkyl, triazolyl, -S-C1-C6 halogenated alkyl, C1-C6 hydroxyalkyl, -CH₂C(O)NR²⁷R²⁸, -C2-C6 alkynyl NR²⁹R³⁰, C2-C6 deuterated alkynyl, (C1-C6 alkoxyl) C1-C6 halogenated alkyl- or cycloalkyl; where the cycloalkyl is optionally substituted with halogen or C1-C6 alkyl, where,
R²¹ is H, C1-C6 alkyl, C1-C6 halogenated alkyl, C(O)C1-C6 alkyl or C(O)₂C1-C6 alkyl;
R²² is H, C1-C6 alkyl or C1-C6 halogenated alkyl; or R²¹ and R²², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²³, R²⁴ are each independently H, C1-C6 alkyl or C1-C6 halogenated alkyl; or R²³ and R²⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C6 alkyl; where R²⁵¹, R²⁵², R²⁵³, R²⁵⁴ are each independently H or C1-C6 alkyl; R²⁵¹ and R²⁵², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy; R²⁵³ and R²⁵⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁶ is C1-C6 alkyl, C1-C6 halogenated alkyl or NR²⁶¹R²⁶², where R²⁶¹, R²⁶² are each independently H or C1-C6 alkyl; or R²⁶¹ and R²⁶², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁷, R²⁸ are each independently H, C1-C6 alkyl or C1-C6 halogenated alkyl; or R²⁷ and R²⁸, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁹, R³⁰ are each independently H, C1-C6 alkyl or C1-C6 halogenated alkyl; or R²⁹ and R³⁰, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
n1 is 0, 1, 2 or 3;
R² is H, cyano, halogen, C1-C6 alkyl, C1-C6 alkoxyl, C1-C6 halogenated alkyl, C1-C6 halogenated alkoxyl, C1-C6 deuterated alkoxyl, NR³¹R³², C2-C4 alkynyl or CH₂OR³³; where R³¹, R³², R³³ are each independently hydrogen or C1-C6 alkyl;
R^{3a}, R^{3b}, R^{3c} are each independently hydrogen, halogen, hydroxyl, cyano, C1-C3 alkyl, C1-C3 deuterated alkyl or C1-C3 halogenated alkyl;
L₁ is a bond, O or NR³⁴;
L₂ is C1-C4 alkylene or heteroaryl, where one or two hydrogen atoms on any carbon atom or on any same carbon atom of the C1-C4 alkylene are independently and optionally substituted with deuterium, C1-C6 alkyl, or C1-C6 halogenated alkyl; or two hydrogen atoms on any same carbon atom of the C1-C4 alkylene are optionally simultaneously substituted with -(CH₂)ₘ₃-, -(CH₂)ₘ₁-O-(CH₂)ₘ₂-, -(CH₂)ₘ₁-N-(CH₂)ₘ₂- to form a cyclic substituent; m3 is 1 or 2; each m1 is respectively and independently 0, 1, 2, or 3; each m2 is respectively and independently 0, 1, 2, or 3; and m1 and m2 are not 0 semultaneously;
ring B is a 3-to 6-membered nitrogen-containing heterocyclyl;
ring C is a 3-to 6-membered nitrogen-containing heterocyclyl;
R⁴ is a substituent at any position on ring B; n2 is 0, 1, 2 or 3;
R⁵ is a substituent at any position on ring C; n3 is 0, 1, 2 or 3;
R⁴, R⁵ are defined as follows:
   (a) R⁴, R⁵ are each independently halogen, hydroxyl, C1-C3 hydroxyalkyl, C1-C3 alkyl, C1-C3 halogenated alkyl, C1-C3 alkoxyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxyl) C1-C3 alkyl, (C1-C3 alkyl)C(O), oxo, (C1-C3 halogenated alkyl)C(O)-, -SO₂F, (C1-C3 alkoxyl) C1-C3 alkoxyl, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl) phenyl, -OC(O) heterocyclyl or -CH₂ heterocyclyl; wherein, the phenyl in -OC(O)NH(C1-C3 alkyl)O(C1-C3 alkyl) phenyl or -NHC(O) phenyl is optionally substituted with -C(O)H or OH; the heterocyclyl in -CH₂ heterocyclyl is optionally substituted with oxo; or
   (b) one R⁴ is -CH₂CH₂- or -CH₂CH₂CH₂-, and the R⁴ together with a carbon atom to which it is attached, forms a cyclopropyl or cyclobutyl; the remaining R⁴, R⁵ are each independently halogen, hydroxyl, C1-C3 hydroxyalkyl, C1-C3 alkyl, C1-C3 halogenated alkyl, C1-C3 alkoxyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxyl) C1-C3 alkyl, (C1-C3 alkyl)C(O), oxo, (C1-C3 halogenated alkyl)C(O)-, -SO₂F, (C1-C3 alkoxyl) C1-C3 alkoxyl, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl) phenyl, -OC(O) heterocyclyl or -CH₂ heterocyclyl; wherein, the phenyl in -OC(O)NH(C1-C3 alkyl)O(C1-C3 alkyl) phenyl or -NHC(O) phenyl is optionally substituted with -C(O)H or OH; the heterocyclyl in -CH₂ heterocyclyl is optionally substituted with oxo; or
   (c) one R⁵ is -CH₂CH₂- or -CH₂CH₂CH₂-, and, the R⁵ together with a carbon atom to which it is attached, forms a cyclopropyl or cyclobutyl; the remaining R⁵, and R⁴ are each independently halogen, hydroxyl, C1-C3 hydroxyalkyl, C1-C3 alkyl, C1-C3 halogenated alkyl, C1-C3 alkoxyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxyl) C1-C3 alkyl, (C1-C3 alkyl)C(O), oxo, (C1-C3 halogenated alkyl)C(O)-, -SO₂F, (C1-C3 alkoxyl) C1-C3 alkoxyl, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl) phenyl, -OC(O)heterocyclyl or -CH₂ heterocyclyl; wherein, the phenyl in -OC (O)NH(C1-C3 alkyl)O(C1-C3 alkyl) phenyl or -NHC(O)phenyl is optionally substituted with -C(O)H or OH; the heterocyclyl in -CH₂ heterocyclyl is optionally substituted with oxo; or
   (d) one R⁴ is -CH₂CH₂- or -CH₂CH₂CH₂- and, the R⁴ together with a carbon atom to which it is attached, forms a cyclopropyl or cyclobutyl; one R⁵ is -CH₂CH₂- or -CH₂CH₂CH₂- and, the R⁵ together with a carbon atom to which it is attached, forms a cyclopropyl or cyclobutyl; the remaining R⁴, the remaining R⁵ are each independently halogen, hydroxyl, C1-C3 hydroxyalkyl, C1-C3 alkyl, C1-C3 halogenated alkyll, C1-C3 alkoxyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxyl) C1-C3 alkyl, (C1-C3 alkyl)C(O), oxo, (C1-C3 halogenated alkyl)C(O)-, -SO₂F, (C1-C3 alkoxyl) C1-C3 alkoxyl, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl) phenyl, -OC(O) heterocyclyl or -CH₂ heterocyclyl; wherein, the phenyl in -OC (O)NH(C1-C3 alkyl)O(C1-C3 alkyl) phenyl or -NHC (O) phenyl is optionally substituted with -C(O)H or OH; the heterocyclyl in -CH₂ heterocyclyl is optionally substituted with oxo;
each R³⁴ is respectively and independently hydrogen, C1-C3 alkyl, or C1-C3 halogenated alkyl; or two R³⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy.

In an embodiment, the compound is shown in formula (I-1) or formula (I-2):

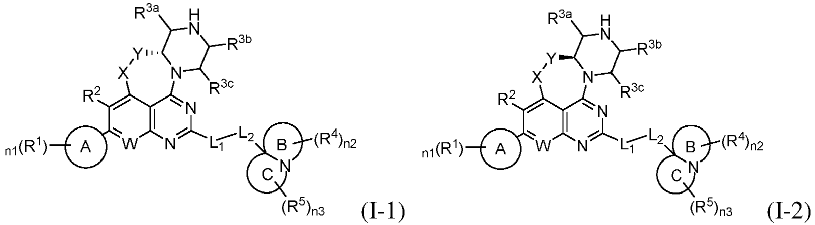

In either formula, X, Y, W, R¹, R², R^{3a}, R^{3b}, R^{3c}, R⁴, R⁵, n1, n2, n3, L₁, L₂, ring A, ring B, ring C are each as defined for formula (I).

In an embodiment, the present disclosure provides a compound shown in formula (II-1) or formula (II-2), or a pharmaceutically acceptable salt, a stereoisomer, a solvate, or a prodrug thereof: 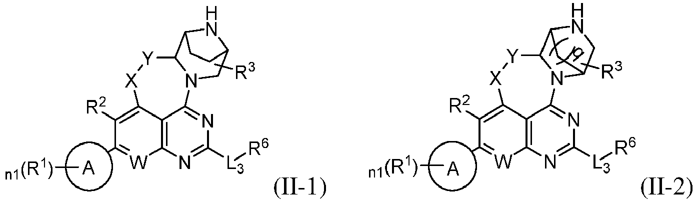 where in formula (II-1) or formula (II-2),
q is 1 or 2;
X is O or NR¹¹; where R¹¹ is selected from H and C1-C6 alkyl;
Y is CR¹²R¹³, CR¹⁴R¹⁵CR¹⁶R¹⁷, C(O) or C(O)CR¹⁸R¹⁹, where R¹², R¹³, R¹⁴ and R¹⁵ are each independently selected from H, C1-C3 alkyl, C1-C3 deuterated alkyl, and C1-C3 halogenated alkyl; R¹⁶, R¹⁷, R¹⁸, R¹⁹ are each independently selected from H, C1-C3 alkyl and halogen;
W is N or CR²⁰; where R²⁰ is H, C1-C6 alkyl, halogen, C1-C6 halogenated alkyl, C1-C6 alkoxyl, cycloalkyl, cycloalkyl-O-, heterocyclyl or heterocyclyl-O-; where the cycloalkyl, the heterocyclyl are each independently optionally substituted with halogen;
ring A is selected from a group consisting of: aryl, heteroaryl;
R¹ is a substituent at any position on ring A, each R¹ is independently selected from: C1-C6 alkyl, halogen, hydroxyl, C1-C6 alkoxyl, C1-C6 halogenated alkyl, C1-C6 halogenated alkoxyl, cyano, NR²¹R²², C(O)NR²³R²⁴, CH₂R²⁵, N=S(O)(C1-C6 alkyl)₂, S(O)C1-C6 alkyl, S(O)₂R²⁶, -S-C1-C6 alkyl, C2-C6 alkenyl, C2-C6 alkynyl, C2-C6 hydroxyalkynyl, C1-C6 cyanoalkyl, triazolyl, -S-C1-C6 halogenated alkyl, C1-C6 hydroxyalkyl, -CH₂C(O)NR²⁷R²⁸, -C2-C6 alkynyl NR²⁹R³⁰, C2-C6 deuterated alkynyl, (C1-C6 alkoxyl) C1-C6 halogenated alkyl- or cycloalkyl; where the cycloalkyl is optionally substituted with halogen or C1-C6 alkyl, where,
R²¹ is H, C1-C6 alkyl, C1-C6 halogenated alkyl, C(O)C1-C6 alkyl or C(O)₂C1-C6 alkyl;
R²² is H, C1-C6 alkyl or C1-C6 halogenated alkyl; or R²¹ and R²², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²³, R²⁴ are each independently H, C1-C6 alkyl or C1-C6 halogenated alkyl; or R²³ and R²⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C6 alkyl; where R²⁵¹, R²⁵², R²⁵³, R²⁵⁴ are each independently H or C1-C6 alkyl; R²⁵¹ and R²⁵², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy; R²⁵³ and R²⁵⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁶ is C1-C6 alkyl, C1-C6 halogenated alkyl or NR²⁶¹R²⁶², where R²⁶¹, R²⁶² are each independently H or C1-C6 alkyl; or R²⁶¹ and R²⁶², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁷, R²⁸ are each independently H, C1-C6 alkyl or C1-C6 halogenated alkyl; or R²⁷ and R²⁸, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁹, R³⁰ are each independently H, C1-C6 alkyl or C1-C6 halogenated alkyl; or R²⁹ and R³⁰, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
n1 is 0, 1, 2 or 3;
R² is H, cyano, halogen, C1-C6 alkyl, C1-C6 alkoxyl, C1-C6 halogenated alkyl, C1-C6 halogenated alkoxyl, C1-C6 deuterated alkoxyl, NR³¹R³², C2-C4 alkynyl or CH₂OR³³; where R³¹, R³², R³³ are each independently hydrogen or C1-C6 alkyl;
R³ is a substituent at any position on a bridge ring, and is hydrogen, halogen, hydroxyl, cyano, C1-C3 alkyl, C1-C3 deuterated alkyl or C1-C3 halogenated alkyl;
L₃ is a bond, O or NR³⁴;
R⁶ is hydrogen, -N(R³⁴)₂, heterocyclyl, C1-C6 alkyl, -L-heterocyclyl, -L-aryl, -L-heteroaryl, -L-cycloalkyl, -L-N(R³⁴)₂, -L-NHC(=NH)NH₂, -L-C(O)N(R³⁴)₂, -L-C1-C6 halogenated alkyl, -L-OR³⁴, -L-(CH₂OR³⁴)(CH₂)ₙOR³⁴, -L-NR³⁴C(O)-aryl, -L-COOH, or -L-C(O)OC1-C6 alkyl; where each of the heterocyclyl, the aryl in the -L-NR³⁴C(O)-aryl, the heterocyclyl in the -L-heterocyclyl, the cycloalkyl in the -L-cycloalkyl may be optionally substituted with one or more R³⁵, or optionally two hydrogen atoms on the same carbon atom are simultaneously substituted with -CH₂CH₂- or -CH₂CH₂CH₂- as cyclopropyl or cyclobutyl; each of the aryl in the -L-aryl, the heteroaryl in the -L-heteroaryl may be optionally substituted with one or more R³⁶;
where each L is respectively and independently C1-C4 alkylene or heteroaryl, where one or two hydrogen atoms on any carbon atom or on any same carbon atom of the C1-C4 alkylene are independently and optionally substituted with deuterium, C1-C6 alkyl, or C1-C6 halogenated alkyl; or two hydrogen atoms on any same carbon atom of the C1-C4 alkylene are optionally simultaneously substituted with -(CH2)m3-, -(CH₂)ₘ₁-O-(CH₂)ₘ₂-, -(CH₂)ₘ₁-N-(CH₂)ₘ₂- to form a cyclic substituent; m3 is 1 or 2; each m1 is respectively and independently 0, 1, 2, or 3; each m2 is respectively and independently 0, 1, 2, or 3; and m1 and m2 are not 0 semultaneously;
each R³⁵ is respectively and independently halogen, hydroxyl, C1-C3 hydroxyalkyl, C1-C3 alkyl, C1-C3 halogenated alkyl, C1-C3 alkoxyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxyl) C1-C3 alkyl, (C1-C3 alkyl)C(O), oxo, (C1-C3 halogenated alkyl)C(O)-, -SO₂F, (C1-C3 alkoxyl) C1-C3 alkoxyl, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl) phenyl, -OC (O) heterocyclyl (e.g. -OC(O)- 3- to 6-membered heterocyclyl) or -CH₂ heterocyclyl (e.g. -CH₂- 3- to 6-membered heterocyclyl); wherein, the phenyl in -OC(O)NH(C1-C3 alkyl)O(C1-C3 alkyl) phenyl or -NHC (O) phenyl is optionally substituted with -C(O)H or OH; the heterocyclyl in -CH₂ heterocyclyl is optionally substituted with oxo;
each R³⁶ is respectively and independently halogen, hydroxyl, HC (O)-, C1-C4 alkyl, C1-C4 alkoxyl, C1-C4 halogenated alkyl, C1-C4 hydroxyalkyl, or -N(R³⁴)₂;
each R³⁴ is respectively and independently hydrogen, C1-C3 alkyl, or C1-C3 halogenated alkyl; or two R³⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy.

In an embodiment, the compound is shown in formula (II-1A), formula (II-1B), formula (II-2A), formula (II-2B): 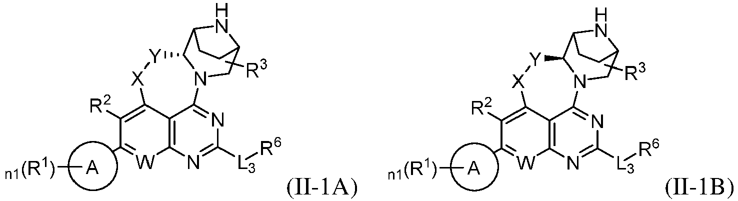 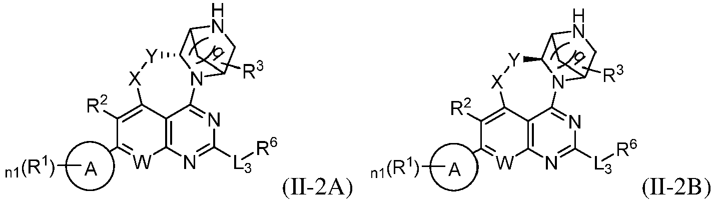 in each formula, X, Y, W, R¹, R², R³, R⁶, n1, L₃, q, and ring A are each as defined for formula (II-1) or formula (II-2).

In an embodiment, the compound is shown in formula (II-1A1), formula (II-1A2), formula (II-1B1), formula (II-1B2), formula (II-1C1), or Formula (II-1C2): 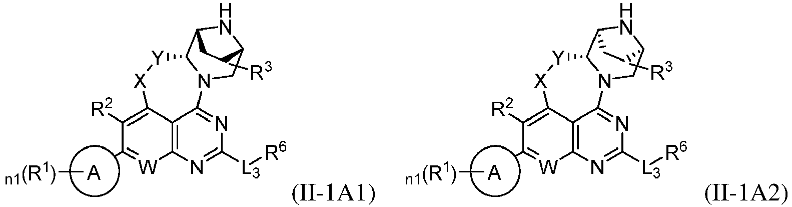 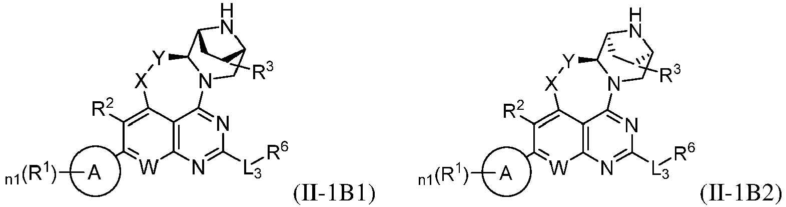 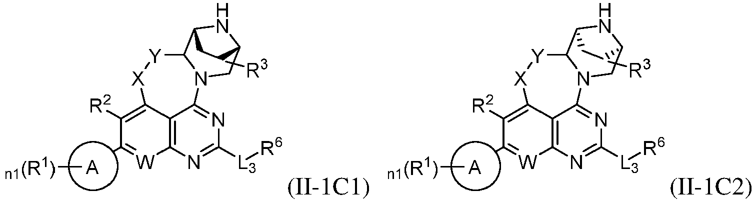 in each formula, X, Y, W, R¹, R², R³, R⁶, n1, L₃, and ring A are each as defined for formula (II-1) or formula (II-2).

In an embodiment, the present disclosure provides a compound shown in formula (III), or a pharmaceutically acceptable salt, a stereoisomer, a solvate, or a prodrug thereof: 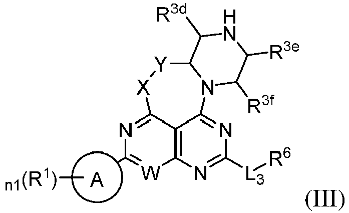 in formula (III),
X is O or NR¹¹; where R¹¹ is selected from H and C1-C6 alkyl;
Y is CR¹²R¹³, CR¹⁴R¹⁵CR¹⁶R¹⁷, C(O) or C(O)CR¹⁸R¹⁹, where R¹², R¹³, R¹⁴ and R¹⁵ are each independently selected from H, C1-C3 alkyl, C1-C3 deuterated alkyl, and C1-C3 halogenated alkyl; R¹⁶, R¹⁷, R¹⁸, R¹⁹ are each independently selected from H, C1-C3 alkyl and halogen;
W is N or CR²⁰; where R²⁰ is H, C1-C6 alkyl, halogen, C1-C6 halogenated alkyl, C1-C6 alkoxyl, cycloalkyl, cycloalkyl-O-, heterocyclyl or heterocyclyl-O-; where the cycloalkyl, the heterocyclyl are each independently optionally substituted with halogen;
ring A is selected from a group consisting of: aryl, heteroaryl;
R¹ is a substituent at any position on ring A, each R¹ is independently selected from: C1-C6 alkyl, halogen, hydroxyl, C1-C6 alkoxyl, C1-C6 halogenated alkyl, C1-C6 halogenated alkoxyl, cyano, NR²¹R²², C(O)NR²³R²⁴, CH₂R²⁵, N=S(O) (C1-C6 alkyl)₂, S (O) C1-C6 alkyl, S (O)₂R²⁶, -S-C1-C6 alkyl, C2-C6 alkenyl, C2-C6 alkynyl, C2-C6 hydroxyalkynyl, C1-C6 cyanoalkyl, triazolyl, -S-C1-C6 halogenated alkyl, C1-C6 hydroxyalkyl, -CH₂C(O)NR²⁷R²⁸, -C2-C6 alkynyl NR²⁹R³⁰, C2-C6 deuterated alkynyl, (C1-C6 alkoxyl) C1-C6 halogenated alkyl- or cycloalkyl; where the cycloalkyl is optionally substituted with halogen or C1-C6 alkyl, where,
R²¹ is H, C1-C6 alkyl, C1-C6 halogenated alkyl, C(O)C1-C6 alkyl or C(O)₂C1-C6 alkyl; R²² is H, C1-C6 alkyl or C1-C6 halogenated alkyl; or R²¹ and R²², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²³, R²⁴ are each independently H, C1-C6 alkyl or C1-C6 halogenated alkyl; or R²³ and R²⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C6 alkyl; where R²⁵¹, R²⁵², R²⁵³, R²⁵⁴ are each independently H or C1-C6 alkyl; R²⁵¹ and R²⁵², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy; R²⁵³ and R²⁵⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁶ is C1-C6 alkyl, C1-C6 halogenated alkyl or NR²⁶¹R²⁶², where R²⁶¹, R²⁶² are each independently H or C1-C6 alkyl; or R²⁶¹ and R²⁶², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁷, R²⁸ are each independently H, C1-C6 alkyl or C1-C6 halogenated alkyl; or R²⁷ and R²⁸, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁹, R³⁰ are each independently H, C1-C6 alkyl or C1-C6 halogenated alkyl; or R²⁹ and R³⁰, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
n1 is 0, 1, 2 or 3;
R ^{3d} , R^{3e}, R^{3f} are each independently hydrogen, halogen, hydroxyl, cyano, C1-C3 alkyl, C1-C3 deuterated alkyl or C1-C3 halogenated alkyl; or
R^{3d} is linked to R^{3e} to form -CHR^{3d1}- or -CHR^{3d2}CHR^{3d3}-; R^{3f} is hydrogen, halogen, hydroxyl, cyano, C1-C3 alkyl, C1-C3 deuterated alkyl or C1-C3 halogenated alkyl; R^{3d1}, R^{3d2}, R^{3d3} are each independently hydrogen, halogen, hydroxyl, cyano, C1-C3 alkyl, C1-C3 deuterated alkyl or C1-C3 halogenated alkyl; or
R^{3d} is linked to R^{3f} to form -CHR^{3f1}- or -CHR^{3f2}CHR^{3f3}-; R^{3e} is hydrogen, halogen, hydroxyl, cyano, C1-C3 alkyl, C1-C3 deuterated alkyl or C1-C3 halogenated alkyl; R^{3f1}, R^{3f2}, R^{3f3} are each independently hydrogen, halogen, hydroxyl, cyano, C1-C3 alkyl, C1-C3 deuterated alkyl or C1-C3 halogenated alkyl;
L₃ is a bond, O or NR³⁴;
R⁶ is hydrogen, -N(R³⁴)₂, heterocyclyl, C1-C6 alkyl, -L-heterocyclyl, -L-aryl, -L-heteroaryl, -L-cycloalkyl, -L-N(R³⁴)₂, -L-NHC(=NH)NH₂, -L-C(O)N(R³⁴)₂, -L-C1-C6 halogenated alkyl, -L-OR³⁴, -L-(CH₂OR³⁴)(CH₂)ₙOR³⁴, -L-NR³⁴C(O)-aryl, -L-COOH, or -L-C(O)OC1-C6 alkyl; where each of the heterocyclyl, the aryl in the -L-NR³⁴C(O)-aryl, the heterocyclyl in the -L-heterocyclyl, the cycloalkyl in the -L-cycloalkyl may be optionally substituted with one or more R³⁵, or optionally two hydrogen atoms on the same carbon atom are simultaneously substituted with -CH₂CH₂- or -CH₂CH₂CH₂- as cyclopropyl or cyclobutyl; each of the aryl in the -L-aryl, the heteroaryl in the -L-heteroaryl may be optionally substituted with one or more R³⁶;
where each L is respectively and independently C1-C4 alkylene or heteroaryl, where one or two hydrogen atoms on any carbon atom or on any same carbon atom of the C1-C4 alkylene are independently and optionally substituted with deuterium, C1-C6 alkyl, or C1-C6 halogenated alkyl; or two hydrogen atoms on any same carbon atom of the C1-C4 alkylene are optionally simultaneously substituted with -(CH₂)ₘ₃-, -(CH₂)ₘ₁-O-(CH₂)ₘ₂-, -(CH₂)ₘ₁-N-(CH₂)ₘ₂- to form a cyclic substituent; m3 is 1 or 2; each m1 is respectively and independently 0, 1, 2, or 3; each m2 is respectively and independently 0, 1, 2, or 3; and m1 and m2 are not 0 semultaneously;
each R³⁵ is respectively and independently halogen, hydroxyl, C1-C3 hydroxyalkyl, C1-C3 alkyl, C1-C3 halogenated alkyl, C1-C3 alkoxyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxyl) C1-C3 alkyl, (C1-C3 alkyl)C(O), oxo, (C1-C3 halogenated alkyl)C(O)-, -SO₂F, (C1-C3 alkoxyl) C1-C3 alkoxyl, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl) phenyl, -OC (O) heterocyclyl (e.g. -OC(O)- 3- to 6-membered heterocyclyl) or -CH₂ heterocyclyl (e.g. -CH₂- 3- to 6-membered heterocyclyl); wherein, the phenyl in -OC (O)NH(C1-C3 alkyl)O(C1-C3 alkyl) phenyl or -NHC (O) phenyl is optionally substituted with -C(O)H or OH; the heterocyclyl in -CH₂ heterocyclyl is optionally substituted with oxo; or
each R³⁶ is respectively and independently halogen, hydroxyl, HC (O)-, C1-C4 alkyl, C1-C4 alkoxyl, C1-C4 halogenated alkyl, C1-C4 hydroxyalkyl, or -N(R³⁴)₂;
each R³⁴ is respectively and independently hydrogen, C1-C3 alkyl, or C1-C3 halogenated alkyl; or two R³⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy.

In an embodiment, the compound is shown in formula (III-1) or formula (III-2): 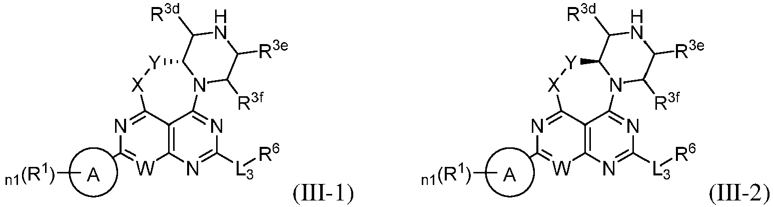 in either formula, X, Y, W, R¹, R^{3d}, R^{3e}, R^{3f}, R⁶, n1, L₃, and ring A are each as defined for formula (III).

In an embodiment, the compound is shown in formula (III-1a), formula (III-1b), formula (III-2a), or formula (III-2b):

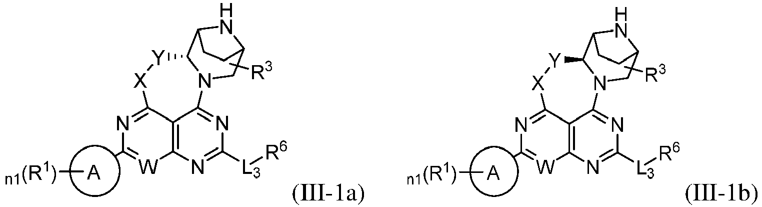

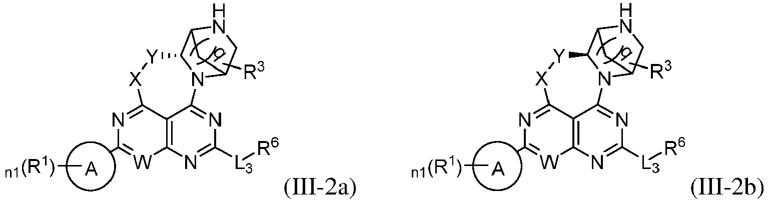

In each formula, q is 1 or 2; R³ is a substituent at any position on a bridge ring, and is hydrogen, halogen, hydroxyl, cyano, C1-C3 alkyl, C1-C3 deuterated alkyl or C1-C3 halogenated alkyl; X, Y, W, ring A, R¹, n1, L₃, R⁶ are as defined for formula (III).

In an embodiment, the compound is shown in formula (III-1a1), formula (III-1a2), formula (III-1b1), formula (III-1b2), formula (III-1c1), or formula (III-1c2): 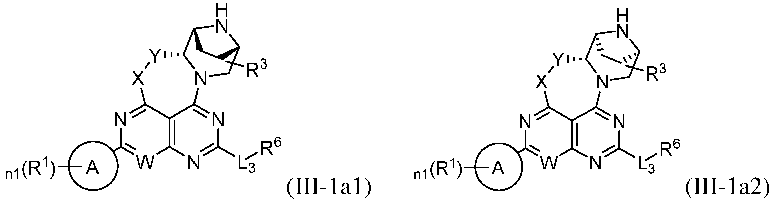 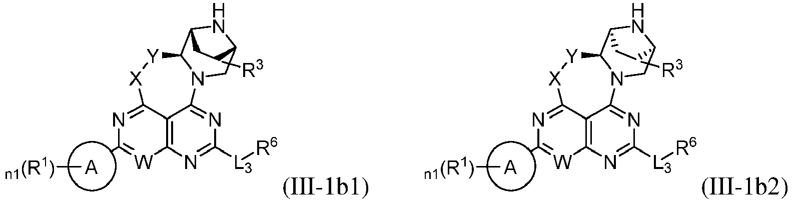 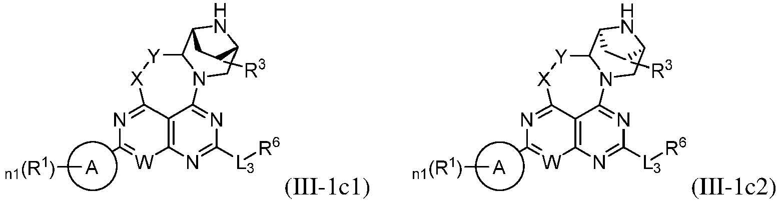 in each formula, R³ is a substituent at any position on a bridge ring, and is hydrogen, halogen, hydroxyl, cyano, C1-C3 alkyl, C1-C3 deuterated alkyl or C1-C3 halogenated alkyl; X, Y, W, ring A, R¹, n1, L₃, R⁶ are as defined for formula (III).

In an embodiment, in each formula, R³ is hydrogen.

In an embodiment, in each formula, Y is -CH₂-.

In an embodiment, in each formula, the cycloalkyl is C3-C20 cycloalkyl. Preferably, the cycloalkyl is C3-C12 cycloalkyl (more preferably, C3-C8 monocyclic cycloalkyl), C5-C20 spirocycloalkyl, C5-C20 fused cycloalkyl or C5-C20 bridged cycloalkyl.

In an embodiment, in each formula, the heterocyclyl is 3- to 20-membered heterocyclyl. Preferably, the heterocyclyl is a monocyclic heterocyclyl (e.g. a 3-to 8-membered monocyclic heterocyclyl), a 5-to 20-membered spiro heterocyclyl, a 5-to 20-membered fused heterocyclyl, and a 5-to 20-membered bridged heterocyclyl.

In an embodiment, in each formula, the aryl is C6-C14 aryl. Preferably, the aryl is a monocyclic aryl, a non-fused polycyclic aryl, and an aromatic fused polycyclic.

In an embodiment, in each formula, the heteroaryl is 5- to 14-membered heteroaryl. Preferably, the heteroaryl is a monocyclic heteroaryl (e.g. a 5-or 6-membered monocyclic heteroaryl), a fused bicyclic heteroaryl (e.g. an 8-to 10-membered bicyclic heteroaryl), or a fused tricyclic heteroaryl.

In an embodiment, W is N or CR²⁰; where R²⁰ is hydrogen, fluorine, methyl, methoxyl or cyclopropoxyl.

In an embodiment, X is O.

In an embodiment, Y is CH₂.

In an embodiment, Y is selected from a group consisting of:

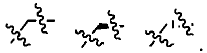

In an embodiment, R² is fluorine, chlorine, hydroxyl, methoxyl, or cyano.

In an embodiment, ring A is phenyl, naphthyl, pyridyl, pyrimidinyl, pyrazinyl, pyridazinyl, pyrrolyl, pyrazolyl, imidazolyl, 1,2,3,4-tetrahydronaphthyl, 2,3-dihydro-1H-indenyl, isoquinolinyl, indazolyl, or benzo[d] [1,3]dioxolane.

In an embodiment, ring A is selected from a group consisting of: 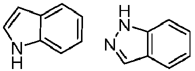 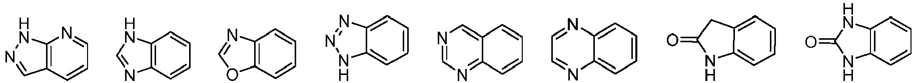 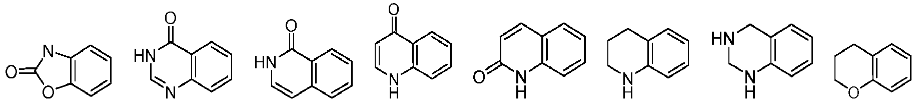 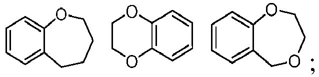 and these groups may be attached to the remainder of a molecule through any suitable ring atom.

In an embodiment, R¹ is a substituent at any position on ring A; n1 is 0, 1, 2 or 3;
each R¹ is respectively and independently selected from: C1-C3 alkyl, halogen, hydroxyl, C1-C3 alkoxyl, C1-C3 halogenated alkyl, C1-C3 halogenated alkoxyl, cyano, NR²¹R²², C(O)NR²³R²⁴, CH₂R²⁵, N=S(O) (C1-C3 alkyl)₂, S (O) C1-C3 alkyl, S (O)₂R²⁶, -S-C1-C3 alkyl, C2-C4 alkenyl, C2-C4 alkynyl, C2-C4 hydroxyalkynyl, C1-C3 cyanoalkyl, triazolyl, -S-C1-C3 halogenated alkyl, C1-C3hydroxyalkyl, -CH₂C(O)NR²⁷R²⁸, -C2-C4 alkynyl NR²⁹R³⁰, C2-C4 deuterated alkynyl, (C1-C3 alkoxyl) C1-C3 halogenated alkyl- or cycloalkyl; where the cycloalkyl is optionally substituted with halogen or C1-C3 alkyl, where
R²¹ is H, C1-C3 alkyl, C1-C3 halogenated alkyl, C(O)C1-C3 alkyl or C(O)₂C1-C3 alkyl; R²² is H, C1-C3 alkyl or C1-C3 halogenated alkyl; or R²¹ and R²², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²³, R²⁴ are each independently H, C1-C3 alkyl or C1-C3 halogenated alkyl; or R²³ and R²⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C3 alkyl; where R²⁵¹, R²⁵², R²⁵³, R²⁵⁴ are each independently H or C1-C3 alkyl; R²⁵¹ and R²⁵², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy; R²⁵³ and R²⁵⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁶ is C1-C3 alkyl, C1-C3 halogenated alkyl or NR²⁶¹R²⁶², where R²⁶¹, R²⁶² are each independently H or C1-C3 alkyl; or R²⁶¹ and R²⁶², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁷, R²⁸ are each independently H, C1-C3 alkyl or C1-C3 halogenated alkyl; or R²⁷ and R²⁸, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁹, R³⁰ are each independently H, C1-C3 alkyl or C1-C3 halogenated alkyl; or R²⁹ and R³⁰, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy.
In an embodiment, L₂ is methylene or ethylidene or propylidene; where one or two hydrogen atoms on any carbon atom or on any same carbon atom of the methylene or ethylidene or propylidene are independently and optionally substituted with deuterium or C1-C3 alkyl; or two hydrogen atoms on any same carbon atom of the methylene or ethylidene or propylidene are optionally substituted simultaneously with -CH₂- or -CH₂CH₂- to form a cyclopropyl or cyclobutyl.

In an embodiment, R^{3a}, R^{3b}, R^{3c} are each independently hydrogen, fluorine, methyl or methoxyl.

In an embodiment, 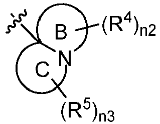 is selected from a group consisting of: 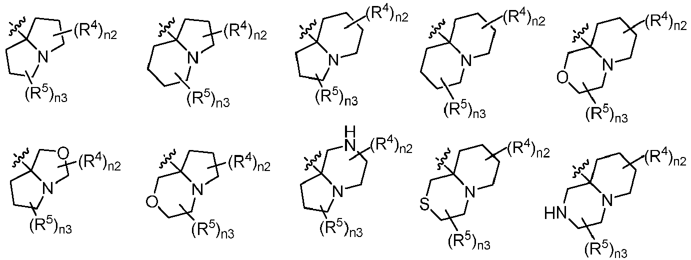 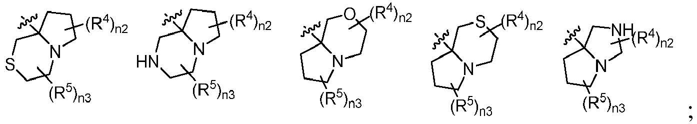 in each formula, each R⁴, R⁵, n2, n3 is independently as defined for formula (I).

In an embodiment, 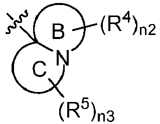 is selected from a group consisting of:

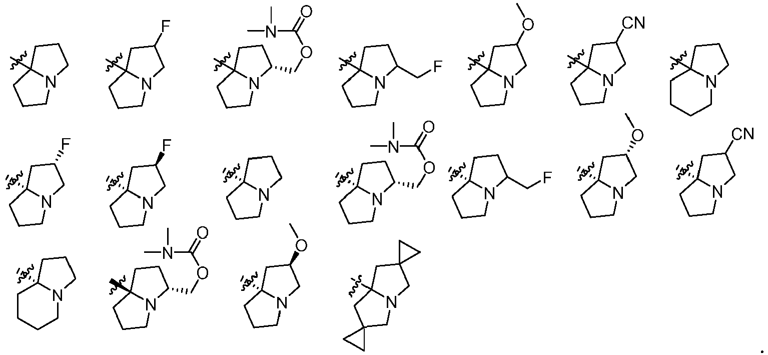

In an embodiment, R³ is hydrogen, fluorine, methyl or methoxyl.

In an embodiment, R ^{3d}, R^{3e}, R^{3f} are each independently hydrogen, fluorine, methyl or methoxyl; or

R^{3d} is linked to R^{3e} to form -CHR^{3d1}- or -CHR^{3d2}CHR^{3d3}; R^{3f} is hydrogen, fluorine, methyl or methoxyl; R^{3d1}, R^{3d2}, R^{3d3} are each independently hydrogen, fluorine, methyl or methoxyl; or

R^{3d} is linked to R^{3f} to form -CHR^{3f1}- or -CHR^{3f2}CHR^{3f3}; R^{3e} is hydrogen, fluorine, methyl or methoxyl; R^{3f1}, R^{3f2}, R^{3f3} are each independently hydrogen, fluorine, methyl or methoxyl.

In an embodiment, L is methylene or ethylidene or propylidene, where one or two hydrogen atoms on any carbon atom or on any same carbon atom of the methylene or ethylidene or propylidene are independently and optionally substituted with deuterium or C1-C3 alkyl; or two hydrogen atoms on any same carbon atom of the methylene or ethylidene or propylidene are optionally substituted simultaneously with -CH₂- or -CH₂CH₂- to form a cyclopropyl or cyclobutyl.

In an embodiment, R⁶ is hydrogen, -N(R³⁴)₂, 3- to 20-membered heterocyclyl, C1-C6 alkyl, -L-3- to 20-membered heterocyclyl, -L-C6-C14 aryl, -L-5- to 14-membered heteroaryl, -L-C3-C20 cycloalkyl, -L-N(R³⁴)₂, -L-NHC(=NH)NH₂, -L-C(O)N(R³⁴)₂, -L-C1-C6 halogenated alkyl, -L-OR³⁴, -L-(CH₂OR³⁴)(CH₂)ₙOR³⁴, -L-NR³⁴C(O)-C6-C14 aryl, -L-COOH, or -L-C(O)OC1-C6 alkyl; where the 3-to 20-membered heterocyclyl, C6-C14 aryl in the -L-NR³⁴C(O)-C6-C14 aryl; wherein the C3-C20 cycloalkyl may be optionally substituted with one or more R³⁵, or optionally two hydrogen atoms on the same carbon atom are simultaneously substituted with -CH₂CH₂- or -CH₂CH₂CH₂- as cyclopropyl or cyclobutyl; the C6-C14 aryl in -L-C6-C14 aryl or the 5-to 14-membered heteroaryl in the -L-5-to 14-membered heteroaryl may be optionally substituted with one or more R³⁶; L, R³⁴, R³⁵, R³⁶ are each as defined above.

In an embodiment, R⁶ is hydrogen, -N(R³⁴)₂, 3- to 8-membered monocyclic heterocyclyl, 5-to 20-membered spiro heterocyclyl, 5- to 20-membered fused heterocyclyl, 5- to 20-membered bridged heterocyclyl, C1-C6 alkyl, -L-3- to 8-membered monocyclic heterocyclyl, -L-5- to 20-membered spiro heterocyclyl, -L-5- to 20-membered fused heterocyclyl, -L-5- to 20-membered bridged heterocyclyl, -L-phenyl, -L-naphthyl, -L-5 to 14-membered heteroaryl, -L-C3-C12 cycloalkyl, -L-C5-C20 spirocycloalkyl, C5-C20 fused cycloalkyl, -L-C5-C20 bridged cycloalkyl, -L-N(R³⁴)₂, -L-NHC(=NH)NH₂, -L-C(O)N(R³⁴)₂, -L-C1-C6 haloalkyl, -L-OR³⁴, -L-(CH₂OR³⁴)(CH₂)ₙOR³⁴, -L-NR³⁴C(O)-phenyl, -L-NR³⁴C(O)-naphthyl, -L-COOH, or -L-C(O)OC1-C6 alkyl; where the 3-to 8-membered monocyclic heterocyclyl, 5-to 20-membered spiro heterocyclyl, 5-to 20-membered fused heterocyclyl, 5-to 20-membered bridged heterocyclyl, C3-C12 cycloalkyl, C5-C20 spirocycloalkyl, C5-C20 fused cycloalkyl, C5-C20 bridged cycloalkyl, phenyl in the -L-NR³⁴C(O)-phenyl, the naphthyl in the -L-NR³⁴C(O)-naphthyl may be optionally substituted with one or more R³⁵ or optionally two hydrogen atoms on the same carbon atom are simultaneously substituted with -CH₂CH₂- or -CH₂CH₂CH₂- as cyclopropyl or cyclobutyl; the phenyl in the -L-phenyl, the naphthyl in the -L-naphthyl, the 5-to 14-membered heteroaryl in the -L-5-to 14-membered heteroaryl may be optionally substituted with one or more R³⁶; L, R³⁴, R³⁵, R³⁶ are each as defined above.

In an embodiment, R⁶ is hydrogen or -N(R³⁴)₂. Preferably, each R³⁴ is respectively and independently hydrogen or C1-C3 alkyl; or one R³⁴ is hydrogen and the other R³⁴ is C1-C3 alkyl.

In an embodiment, R³⁴ is hydrogen, C1-C3 alkyl or C1-C3 cyanoalkyl.

In an embodiment, the C1-C6 alkyl is methyl, ethyl, isopropyl, or isobutyl.

In an embodiment, L is methylene or ethylidene or propylidene, where one or two hydrogen atoms on any carbon atom or on any same carbon atom of the methylene or ethylidene or propylidene are independently and optionally substituted with deuterium or C1-C3 alkyl; or two hydrogen atoms on any same carbon atom of the methylene or ethylidene or propylidene are optionally substituted simultaneously with -CH₂- or -CH₂CH₂- to form a cyclopropyl or cyclobutyl.

In an embodiment, each R⁶ is respectively and independently 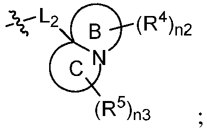 R⁴, R⁵, L₂, ring B, ring C, n2, n3 are as defined for each group in formula (I).

In an embodiment, the heterocyclyl in R⁶ is respectively and independently hexahydro-1H-pyrrolizinyl, hexahydro-3H-pyrrolizin-3-one, hexahydro-1H-pyrrolo[2,1-c] [1,4]oxazinyl, octahydroindolizinyl, hexahydropyrrolizinyl 4(1H)-oxide, azetidinyl, pyrrolidinyl, pyrrolidin-2-one, oxetanyl, piperidinyl, 1-azabicyclo[2.2.1]heptyl, morpholinyl, oxa-5-azabicyclo[2.2.1]hept-5-yl, thiopyranyl, 6-oxa-2-azaspiro[3.4]octyl, 7-oxa-2-azaspiro[3.5]nonyl, 2',3'-dihydrospiro[cyclopropane-1,1'-indenyl], (2S)-1-azabicyclo[2.2.1]heptan-2-yl or tetrahydrofuranyl; each group as described above is independently optionally substituted with one or more R³⁵, or optionally two hydrogen atoms on the same carbon atom are simultaneously substituted with -CH₂CH₂- or -CH₂CH₂CH₂- as cyclopropyl or cyclobutyl.

In an embodiment, R⁶ is -L-heterocyclyl; the heterocyclyl is hexahydro-1H-pyrrolizinyl.

In an embodiment, R⁶ is -L-heterocyclyl; the heterocyclyl is hexahydro -1H-pyrrolizinyl substituted with one R³⁵, or is hexahydro-1H-pyrrolizinyl, wherein two hydrogen atoms on the same carbon atom are simultaneously substituted with -CH₂CH₂- or -CH₂CH₂CH₂- as cyclopropyl or cyclobutyl; where R³⁵ is halogen, hydroxyl, C1-C3 hydroxyalkyl, C1-C3 halogenated alkyl, C1-C3 alkyl, C1-C3 alkoxyl, phenyl, pyrazolyl, or -CH₂OC(O)N(R³⁴)₂.

In an embodiment, the halogen is fluorine.

In an embodiment, the heterocyclyl is hexahydro-1H-pyrrolizinyl further substituted with two additional R³⁵; the other two R³⁵ are each independently C1-C3 alkyl.

In an embodiment, R⁶ is -L-heterocyclyl; the heterocyclyl is azetidinyl substituted with one R³⁵, or is azetidinyl in which two hydrogen atoms on the same carbon atom are simultaneously substituted with CH₂CH₂- or -CH₂CH₂CH₂- as cyclopropyl or cyclobutyl; the R³⁵ is C1-C3 alkyl.

In an embodiment, R⁶ is -L-heterocyclyl; the heterocyclyl is pyrrolidinyl substituted with one R³⁵, or is pyrrolidinyl in which two hydrogen atoms on the same carbon atom are simultaneously substituted with -CH₂CH₂- or -CH₂CH₂CH₂- as cyclopropyl or cyclobutyl; where R³⁵ is hydroxyalkyl, halogenated alkyl, C1-C3 alkyl, alkoxyl, aryl C1-C3 alkyl, -phenyl, -O-phenyl, and-NHC(O)phenyl; where the aryl of the aryl C1-C3 alkyl, the phenyl, or the phenyl in the -O-phenyl or the phenyl in the -NHC (O) phenyl are each independently optionally substituted with one or more R³⁶.

In an embodiment, the phenyl in the phenyl, the -O-phenyl or the -NHC (O) phenyl is substituted with -SO₂F.

In an embodiment, R⁶ is -L-heterocyclyl; the heterocyclyl is pyrrolidinyl substituted with two R³⁵, where one R³⁵ is C1-C3 alkyl and the other R³⁵ is C1-C3 alkoxyl or halogen.

In an embodiment, R⁶ is -L-heterocyclyl; the heterocyclyl is pyrrolidin-2-one substituted with one R³⁵, or is pyrrolidin-2-one, wherein two hydrogen atoms on the same carbon atom are simultaneously substituted with -CH₂CH₂- or -CH₂CH₂CH₂- as cyclopropyl or cyclobutyl; where R³⁵ is C1-C3 alkyl.

In an embodiment, R⁶ is -L-heterocyclyl; the heterocyclyl is piperidinyl substituted with one R³⁵, or is piperidinyl in which two hydrogen atoms on the same carbon atom are simultaneously substituted with -CH₂CH₂- or -CH₂CH₂CH₂- as cyclopropyl or cyclobutyl; where R³⁵ is acetyl, (C1-C3alkoxyl) C1-C3alkoxyl or -C(O)CH₂Cl.

In an embodiment, R⁶ is -L-heterocyclyl; the heterocyclyl is morpholinyl or oxa-5-azabicyclo[2.2.1]hept-5-yl.

In an embodiment, R⁶ is -L-heteroaryl; the heteroaryl is optionally substituted with one or more R³⁶.

In an embodiment, R⁶ is -L-heteroaryl; L is methylene or ethylidene or propylidene, where one or two hydrogen atoms on any carbon atom or on any same carbon atom of the methylene or ethylidene or propylidene are independently and optionally substituted with deuterium or C1-C3 alkyl; or two hydrogen atoms on any same carbon atom of the methylene or ethylidene or propylidene are optionally substituted simultaneously with -CH₂- or -CH₂CH₂- to form a cyclopropyl or cyclobutyl; the heteroaryl is pyridinyl, pyrazolyl, imidazolyl, triazolyl, 4,5,6,7-tetrahydro-1H-indazolyl, benzimidazolyl, imidazo[1,2-a]pyridinyl or pyrimidinyl, each group as described above is optionally substituted with one or more R³⁶.

In an embodiment, R⁶ is -L-heteroaryl; L is methylene or ethylidene or propylidenel where one or two hydrogen atoms on any carbon atom or on any same carbon atom of the methylene or ethylidene or propylidene are independently and optionally substituted with deuterium or C1-C3 alkyl; or two hydrogen atoms on any same carbon atom of the methylene or ethylidene or propylidene are optionally substituted simultaneously with -CH₂- or -CH₂CH₂- to form a cyclopropyl or cyclobutyl; the heteroaryl is pyridyl substituted with one R³⁶, where R³⁶ is halogen, C1-C4 alkyl, -N(R⁵)₂ or C1-C4 alkoxyl.

In an embodiment, R⁶ is -L-heteroaryl; L is methylene or ethylidene or propylidene, where one or two hydrogen atoms on any carbon atom or on any same carbon atom of the methylene or ethylidene or propylidene are independently and optionally substituted with deuterium or C1-C3 alkyl; or two hydrogen atoms on any carbon atom of the methylene or ethylidene or propylidene are optionally substituted simultaneously with -CH₂- or -CH₂CH₂- to form a cyclopropyl or cyclobutyl; the heteroaryl is pyrazolyl substituted with one R³⁶, where R³⁶ is C1-C4 alkyl or -N(R³⁴)₂.

In an embodiment, R⁶ is -L-heteroaryl; L is methylene or ethylidene or propylidene, where one or two hydrogen atoms on any carbon atom or on any same carbon atom of the methylene or ethylidene or propylidene are independently and optionally substituted with deuterium or C1-C3 alkyl; or two hydrogen atoms on any carbon atom of the methylene or ethylidene or propylidene are optionally substituted simultaneously with -CH₂- or -CH₂CH₂- to form a cyclopropyl or cyclobutyl; the heteroaryl is imidazolyl substituted with one R³⁶, where R³⁶ is C1-C4 alkyl, C1-C4 halogenated alkyl or C1-C4 hydroxyalkyl.

In an embodiment, R⁶ is -L-heteroaryl; L is methylene or ethylidene or propylidenel where one or two hydrogen atoms on any carbon atom or on any same carbon atom of the methylene or ethylidene or propylidene are independently and optionally substituted with deuterium or C1-C3 alkyl; or two hydrogen atoms on any same carbon atom of the methylene or ethylidene or propylidene are optionally substituted simultaneously with -CH₂- or -CH₂CH₂- to form a cyclopropyl or cyclobutyl; the heteroaryl is triazolyl substituted with one R³⁶, where R³⁶ is C1-C4 alkyl.

In an embodiment, R⁶ is -L-aryl; the aryl is optionally substituted with one or more R³⁶.

In an embodiment, R⁶ is -L-cycloalkyl; the cycloalkyl is optionally substituted with one or more R³⁵, or optionally two hydrogen atoms on the same carbon atom are simultaneously substituted with -CH₂CH₂- or -CH₂CH₂CH₂- as cyclopropyl or cyclobutyl; each R35 is as defined above.

In an embodiment, R⁶ is -L-N(R³⁴)₂; where L is C1-C4 alkylene, where one or two hydrogen atoms on any carbon atom or on any same carbon atom of the C1-C4 alkylene are independently and optionally substituted with deuterium or C1-C3 alkyl; or two hydrogen atoms on any same carbon atom of the C1-C4 alkylene are optionally substituted simultaneously with -CH₂- or -CH₂CH₂- to form a cyclopropyl or cyclobutyl.

In an embodiment, R⁶ is -L-N(R³⁴)₂; where L is methylene, ethylidene, or propylidene, where one or two hydrogen atoms on any carbon atom or on any same carbon atom of the methylene, ethylidene, or propylidene are independently and optionally substituted with deuterium or C1-C3 alkyl; or two hydrogen atoms on any same carbon atom of the methylene, ethylidene or propylidene are optionally substituted simultaneously with -CH₂- or -CH₂CH₂- to form a cyclopropyl or cyclobutyl group; each R³⁴ is independently selected from C1-C3 alkyl.

in an embodiment, R⁶ is -L-NC(=NH)-NH₂; where L is C1-C4 alkylene, where one or two hydrogen atoms on any carbon atom or on any same carbon atom of the C1-C4 alkylene are independently and optionally substituted with deuterium or C1-C3 alkyl; or two hydrogen atoms on any same carbon atom of the C1-C4 alkylene are optionally substituted simultaneously with -CH₂- or -CH₂CH₂- to form a cyclopropyl or cyclobutyl.

In an embodiment, L is ethylidene or propylidene.

In an embodiment, R⁶ is -L-C1-C6 halogenated alkyl; where L is C1-C4 alkylene, where one or two hydrogen atoms on any carbon atom or on any same carbon atom of the C1-C4 alkylene are independently and optionally substituted with deuterium or C1-C3 alkyl; or two hydrogen atoms on any same of the carbon atom of the C1-C4 alkylene are optionally substituted simultaneously with -CH₂- or -CH₂CH₂- to form a cyclopropyl or cyclobutyl.

in an embodiment, R⁶ is -L-OR³⁴; where L is C1-C4 alkylene, where one or two hydrogen atoms on any carbon atom or on any same carbon atom of the C1-C4 alkylene are independently and optionally substituted with deuterium or C1-C3 alkyl; or two hydrogen atoms on any same carbon atom of the C1-C4 alkylene are optionally substituted simultaneously with -CH₂- or -CH₂CH₂- to form a cyclopropyl or cyclobutyl.

In an embodiment, R⁶ is -L-(CH₂OR³⁴)(CH₂)ₙOR³⁴; where L is C1-C4 alkylene; where one or two hydrogen atoms on any carbon atom or on any same carbon atom of the C1-C4 alkylene are independently and optionally substituted with deuterium or C1-C3 alkyl; or two hydrogen atoms on any same carbon atom of the C1-C4 alkylene are optionally substituted simultaneously with -CH₂- or -CH₂CH₂- to form a cyclopropyl or cyclobutyl.

In an embodiment, R⁶ is -L-NR³⁴C(O)-aryl; where L is C1-C4 alkylene, where one or two hydrogen atoms on any carbon atom or on any same carbon atom of the C1-C4 alkylene are independently and optionally substituted with deuterium or C1-C3 alkyl; or two hydrogen atoms on any same of the same carbon atoms of the C1-C4 alkylene are optionally substituted simultaneously with -CH₂- or -CH₂CH₂- to form a cyclopropyl or cyclobutyl.

In an embodiment, R6 is -L-heterocyclyl; L is C1-C4 alkylene; where two hydrogen atoms on any same carbon atom of the C1-C4 alkylene are independently and optionally substituted with deuterium or C1-C6 alkyl; or two hydrogen atoms on any one same carbon atom of the C1-C4 alkylene are optionally substituted simultaneously with -CH₂- or -CH₂CH₂- to form a cyclopropyl or cyclobutyl.

In an embodiment, L is methylene or ethylidene or propylidene; where two hydrogen atoms on any same carbon atom of the methylene or ethylidene or propylidene are independently and optionally substituted with deuterium or C1-C3 alkyl; or two hydrogen atoms on any same carbon atom of the methylene or ethylidene or propylidene are optionally substituted simultaneously with -CH₂- or -CH₂CH₂- to form a cyclopropyl or cyclobutyl.

In an embodiment, W is CR²⁰; R²⁰ is cyclopropyl, cyclopropyl-O-, cyclobutyl, cyclobutyl-O-, cyclopentyl, cyclopentyl-O-, tetrahydrofuranyl, tetrahydrofuran-O-; where the cyclopropyl, cyclobutyl, cyclopentyl, tetrahydrofuranyl are each independently substituted with halogen.

In an embodiment, W is CR²⁰; R²⁰ is hydrogen, halogen, C1-C6 alkyl, C3-C6 cycloalkyl, C3-C6 cycloalkyl-O-, 3-to 6-membered heterocyclyl or 3-to 6-membered heterocyclyl-O-; wherein the C3-C6 cycloalkyl, the 3-to 6-membered heterocyclyl are each independently substituted with halogen.

In an embodiment, R²⁰ is halogen or C1-C3 alkyl.

In an embodiment, R²⁰ is fluorine.

In an embodiment, R²⁰ is methyl.

In an embodiment, L₂ or L is methylene or ethylidene or propylidene; where two hydrogen atoms on any same carbon atom of the methylene or ethylidene or propylidene are independently and optionally substituted with deuterium or C1-C3 alkyl; or two hydrogen atoms on any same carbon atom of the methylene or ethylidene or propylidene are optionally substituted simultaneously with -CH₂- or -CH₂CH₂- to form a cyclopropyl or cyclobutyl.

In an embodiment, 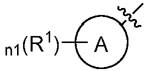 is phenyl substituted with one, two or three R¹, where each R¹ is respectively and independently halogen, hydroxyl, ethynyl, methyl, methyl-O-, methyl-S-, trifluoromethyl, trifluoromethyl-O-, amino, isopropyl, or cyclopropyl; the cyclopropyl is optionally substituted with halogen or methyl.

In an embodiment, 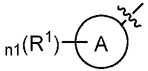 is naphthyl substituted with one, two or three R¹, where each R¹ is respectively and independently halogen, hydroxyl, ethynyl, methyl, methyl-O-, methyl-S-, trifluoromethyl, trifluoromethyl-O-, amino, isopropyl, or cyclopropyl; the cyclopropyl is optionally substituted with halogen or methyl.

In an embodiment, 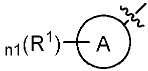 is naphthyl substituted with one, two or three R¹, where each R¹ is respectively and independently halogen, hydroxyl, ethynyl, methyl, ethyl, difluoromethyl, or hydroxyl substituted propyl.

In an embodiment, 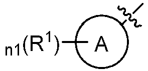 is heteroaryl optionally substituted with one or more R¹.

In an embodiment, 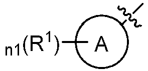 is pyridinyl substituted with one, two or three R¹, where each R⁸ is respectively and independently halogen, hydroxyl, ethynyl, methyl, methyl-O-, methyl-S-, trifluoromethyl, trifluoromethyl-O-, amino, isopropyl, or cyclopropyl; the cyclopropyl is optionally substituted with halogen or methyl.

In an embodiment, 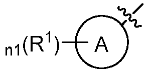 is isoquinolinyl, indazolyl or benzo[d][1,3]dioxolane optionally substituted with one or more R¹.

In an embodiment, 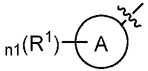 is isoquinolinyl substituted with one, two or three R¹, where each R⁸ is respectively and independently halogen, hydroxyl, ethynyl, methyl, methyl-O-, methyl-S-, trifluoromethyl, trifluoromethyl-O-, amino, isopropyl, or cyclopropyl; the cyclopropyl is optionally substituted with halogen or methyl.

In an embodiment, 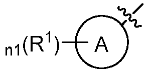 is indazolyl substituted with one, two, or three R¹, where each R⁸ is respectively and independently C1-C3 alkyl.

In an embodiment, 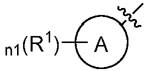 is benzo[d][1,3]dioxolane substituted with two R¹ groups, where each R⁸ is respectively and independently selected from halogen.

In an embodiment, 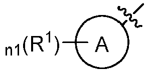 is selected from a group consisting of:

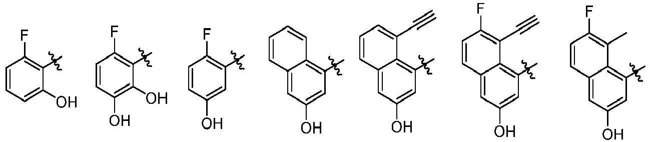

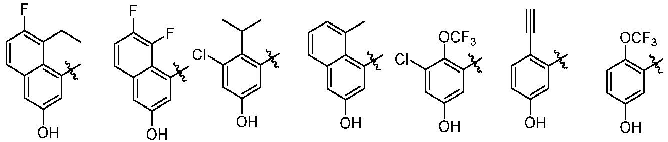

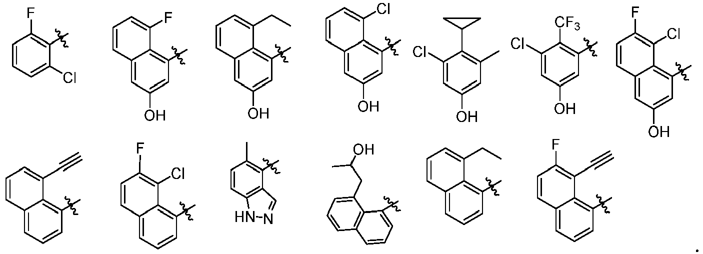

In an embodiment, the -L₃-R⁶ is selected from a group consisting of: 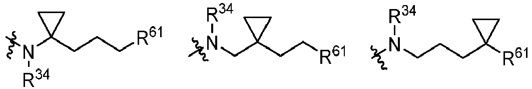 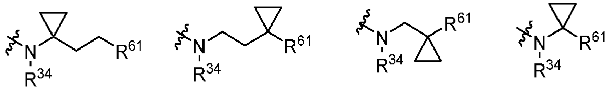 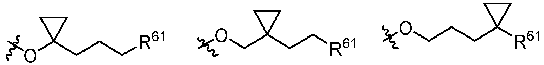 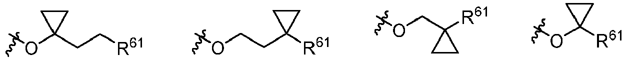 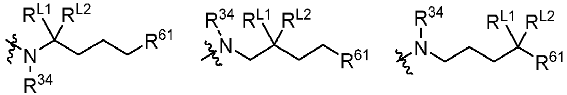 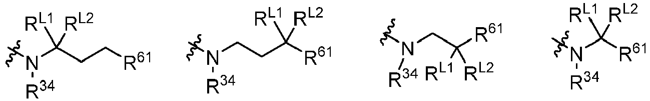 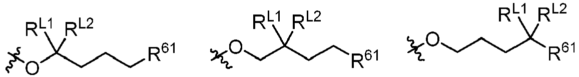 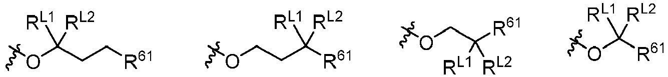 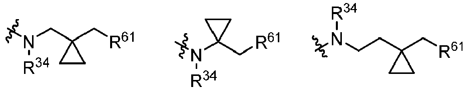 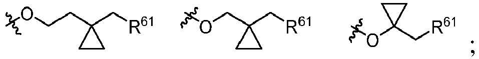 in each formula, R^{L1}, R^{L2} are each independently hydrogen, deuterium, C1-C6 alkyl, or C1-C6halogenated alkyl; R⁶¹ is heterocyclyl, aryl, heteroaryl, cycloalkyl, -N(R³⁴)₂, -NHC(=NH)NH₂, -C(O)N(R³⁴)₂, -C1-C6 halogenated alkyl, -OR³⁴, -(CH₂OR³⁴)(CH₂)ₙOR³⁴, -NR³⁴C(O)-aryl, -COOH, or -C(O)OC1-C6 alkyl; where each of the heterocyclyl, the aryl in the -NR³⁴C(O)- aryl, and the cycloalkyl may be optionally substituted with one or more R³⁵, or optionally two hydrogen atoms on the same one carbon atom are simultaneously substituted with -CH₂CH₂- or -CH₂CH₂CH₂- as a cyclopropyl or cyclobutyl group; each of the aryl, the heteroaryl may be optionally substituted with one or more R³⁶; R³⁴, R³⁵, R³⁶ are each as defined above. Preferably, in each of the above-mentioned formulae, R³⁴ in N-R³⁴ is H.

In an embodiment, the -L₃-R⁶ is selected from a group consisting of: 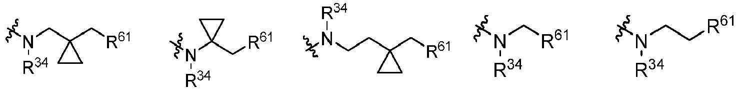 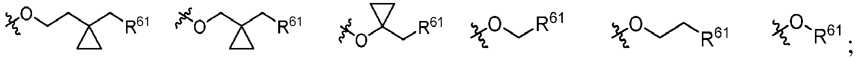 in each formula, R⁶¹ is heterocyclyl, aryl, heteroaryl, cycloalkyl, -N(R³⁴)₂, -NHC(=NH)NH₂, -C(O)N(R³⁴)₂, -C1-C6 halogenated alkyl, -OR³⁴, -(CH₂OR³⁴)(CH₂)ₙOR³⁴, -NR³⁴C(O)-aryl, -COOH, or -C(O)OC1-C6 alkyl; where the heterocyclyl must be substituted with one or more R³⁵, or optionally two hydrogen atoms on the same one carbon atom are simultaneously substituted with -CH₂CH₂- or -CH₂CH₂CH₂- as cyclopropyl or cyclobutyl; each of the aryl in the -NR³⁴C(O)-aryl and the cycloalkyl may be optionally substituted with one or more R³⁵, or optionally two hydrogen atoms on the same one carbon atom are simultaneously substituted with -CH₂CH₂- or -CH₂CH₂CH₂- as a cyclopropyl or cyclobutyl group; each of the aryl, the heteroaryl may be optionally substituted with one or more R³⁶; R³⁴, R³⁵, R³⁶ are as defined above and each is as defined above. Preferably, in each of the above-mentioned formulae, R³⁴ in N-R³⁴ is H.

In an embodiment, R⁶¹ is selected from a group consisting of:

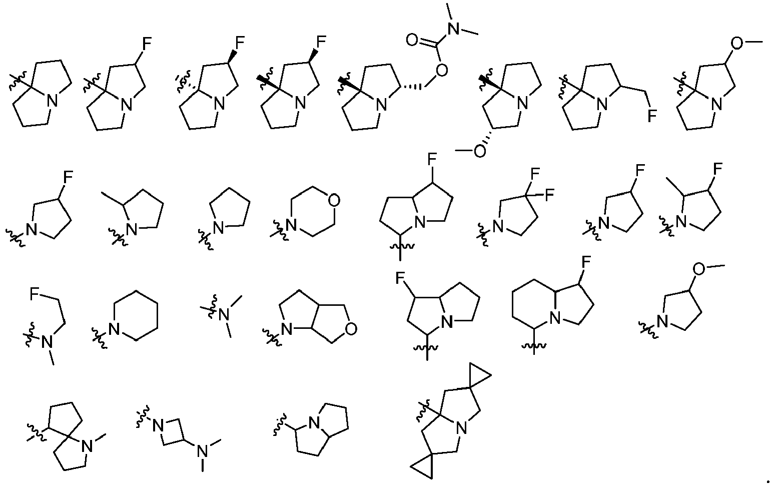

In an embodiment, R⁶¹ is hexahydro-1H-pyrrolizinyl.

In an embodiment, R⁶¹ is hexahydro-1H-pyrrolizinyl substituted with one R³⁵; where R³⁵ is halogen, hydroxyl, C1-C3 hydroxyalkyl, C1-C3 halogenated alkyl, C1-C3 alkyl, C1-C3 alkoxyl, phenyl, pyrazolyl, or -CH₂OC(O)N(R³⁴)₂; each R³⁴ is respectively and independently hydrogen, C1-C3 alkyl, or C1-C3 halogenated alkyl; or two R³⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxyl, trifluoromethoxy.

In an embodiment, the compound is selected from the compounds in Table A.

In an embodiment, the compound is selected from the compounds in example 7 and Table B.

In an embodiment, the compound is selected from the compounds in examples 1 to 6, 8 to 12 and Table C.

In an embodiment, the compound is selected from the compounds in Table (I).

**Table (I)**

| | | | |
|---|---|---|---|
| Z1 | 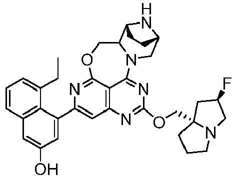 | Z2 | 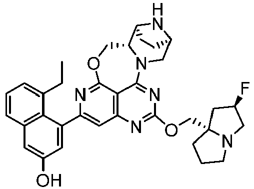 |
| Z308 | 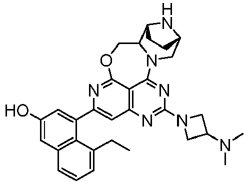 | Z170 | 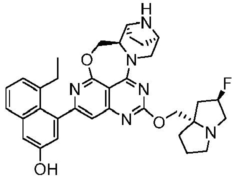 |
| Z140-1 | 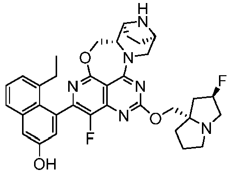 | Z350 | 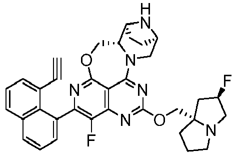 |
| Z381 | 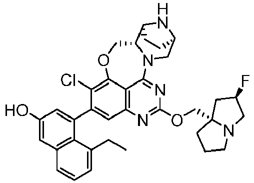 | Z131-1 | 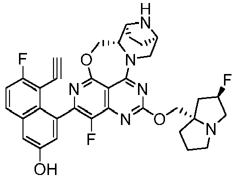 |
| Z260-1 | 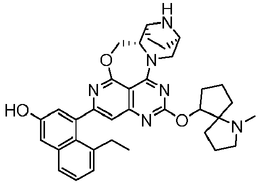 | Z382 | 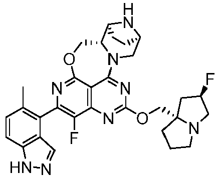 |
| Z383 | 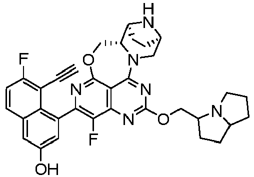 | Z385 | 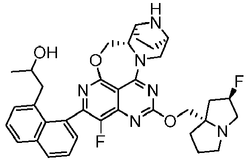 |

In a second aspect, the present disclosure provides a pharmaceutical composition including the compound, or pharmaceutically acceptable salt, stereoisomer, solvate or prodrug thereof according to the first aspect in the above; and a pharmaceutically acceptable carrier.

As used herein, the term "pharmaceutically acceptable carrier" refers to any formulation capable of delivering an effective amount of the active substance of the present disclosure without interfering with the biological activity of the active substance and without toxic and side effects to a host or subject, or a carrier representative of carrier media, including water, oils, vegetables and minerals, cream bases, lotion bases, ointment bases and the like. Such bases include suspending agents, viscosity enhancers, transdermal enhancers and the like. Their formulations are well known to those skilled in the fields of cosmetics or topical pharmaceuticalagents.

In embodiments of the present disclosure, the pharmaceutical composition may be administered in any way: orally, spray inhalation, rectally, nasally, buccally, topically, parenterally, such as subcutaneous, intravenous, intramuscular, intraperitoneal, intrathecal, intraventricular, intrasternal and intracranial injection or infusion, or with the aid of an explanted reservoir. Oral administration, intraperitoneal administration or intravenous administration are preferred. When administered orally, the compounds of the present disclosure may be prepared in any orally acceptable formulation, including but not limited to, tablets, capsules, aqueous solutions or aqueous suspensions. Carriers for tablets generally include lactose and corn starch. In addiiton, lubricants such as magnesium stearate may be added. Diluents used in capsule formulations typically include lactose and dried corn starch. Aqueous suspensions are generally prepared by mixing the active ingredients with suitable emulsifiers and suspending agents. If desired, some sweeteners, flavoring agents or colorants may also be added to the above-mentioned oral formulations. when administered topically, in particular when to affected surfaces or organs that are readily accessible by topical application, such as eye, skin or lower intestinal neurological diseases, the compounds of the present disclosure may be can be prepared into different topical agents according to different aflfected surfaces or organs. When administered topically to eyes, the compounds of the present invention can be formulated into micronized suspensions or solutions with isotonic sterile salines at a certain pH with or without the addition of preservatives such as benzyl alkanol chlorides as carriers. For eye use, the compounds can also be prepared into ointments such as Vaseline ointments. When administered topically to the skin, the compounds of the present disclosure can be prepared into suitable ointment, lotion or cream formulations, with the active ingredients being suspended or dissolved in one or more carriers. Carriers that can be used in ointment formulations include, but are not limited to mineral oils, liquid Vaseline, white Vaseline, propylene glycol, polyethylene oxide, polypropylene oxide, emulsified wax, and water. Carriers that can be used in lotions or creams include but are not limited to mineral oils, sorbitan monostearate, Tween 60, cetyl esters wax, hexadecen-aryl alcohol, 2-octyldodecanol, benzyl alcohol, and water. The compounds of the present invention may also be administered in the form of sterile injections, including sterile injectable water or oil suspensions or sterile injectable solutions. Carriers and solvents that can be used include water, Ringer's solution, and isotonic sodium chloride solution. In addition, sterile non-volatile oils can also be used as solvents or suspending media, eg, monoglycerides or diglycerides.

Another aspect of the present disclosure provides use of the compound as described above, or the pharmaceutically acceptable salt, the stereoisomer, the solvate or the prodrug thereof according to the first aspect or the pharmaceutical composition according to the second aspect in preparation of medicaments for preventing and/or treating a disease or condition, the disease or condition is a KRAS G12D associated disease or disorder.

As used herein, "KRAS G12D" refers to a mutant form of a mammalian KRAS protein that contains an amino acid substitution of an aspartic acid for a glycine at amino acid position 12. The assignment of amino acid codon and residue positions for human KRAS is based on the amino acid sequence identified by UniProtKB/Swiss-Prot P01116: Variantp.Gly12Asp.

As used herein, "KRAS G12D inhibitor" refers to a compound of the present disclosure, as described herein, represented by formula (I), formula (II-1), formula (II-1), or Formula (III). These compounds can negatively regulate or inhibit all or part of the enzymatic activity of KRAS G12D.

As used herein, a "KRAS G12D associated disease or disorder" refers to a disease or disorder associated with or mediated by, or having a KRAS G12D mutation. A non-limiting example of a KRAS G12D associated disease or disorder is a KRAS G12D associated cancer.

Another aspect of the present disclosure provides a method for treating a KRAS G12D associated cancer including the steps of: administering to a subject in need thereof a therapeutically effective amount of the compound or pharmaceutically acceptable salt, stereoisomer, solvate or prodrug thereof according to the first aspect of the present disclosure; or administering to a subject in need thereof a therapeutically effective amount of the pharmaceutical composition according to the second aspect of the present disclosure.

In an embodiment, the KRAS G12D associated cancer includes, but is not limited to, lung cancer, prostate cancer, breast cancer, brain cancer, skin cancer, cervical cancer, testicular cancer, and the like. In an embodiment, the KRAS G12D associated cancer includes, but is not limited to, astrocytoma, breast cancer, cervical cancer, colorectal cancer, endometrial cancer, esophageal cancer, gastric cancer, head and neck cancer, hepatocellular cancer, laryngeal cancer, lung cancer, oral cancer, ovarian cancer, prostate cancer, thyroid cancer, sarcomas and the like. In an embodiment, the KRAS G12D associated cancer includes, but is not limited to, cancers of cardiac sites such as sarcomas (angiosarcomas, fibrosarcomas, rhabdomyosarcomas, liposarcomas), myxomas, rhabdomyomas, fibromas, lipomas, teratomas, and the like; cancers of the lung, for example, bronchial carcinoma (squamous cell, undifferentiated small cell, undifferentiated large cell, adenocarcinoma), alveolar (bronchiolar) carcinoma, bronchial adenoma, sarcoma, lymphoma, chondroma hamartoma, mesothelioma; cancers of the gastrointestinal tract, for example, esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small intestine (adenocarcinoma, carcinoid tumors), kaposi's sarcoma, leiomyoma, hemangioma, lipoma, neurofibroma, fibroma), large intestine (adenocarcinoma, tubular adenoma, villous adenoma, hamartoma, leiomyoma); cancers of the genitourinary tract, for example, kidney (adenocarcinoma, wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratoma), choriocarcinoma, sarcoma, interstitial cell carcinoma, fibroma, fibroadenoma, adenomatoid tumors, lipoma); cancers of the liver, for example liver cancer (hepatocellular carcinoma), cholangiocarcinoma, hepatoblastoma, angiosarcoma, hepatocellular adenoma, hemangioma; biliary: gallbladder cancer, ampulla cancer, bile duct cancer; cancers of bone sites, e.g. osteogenic sarcoma (osteosarcoma), fibrosarcoma, malignant fibrous histiocytoma, chondrosarcoma, ewing's sarcoma, malignant lymphoma (reticulum cell sarcoma), multiple myeloma, malignant giant cell tumor, chordoma, osteochondroma (osteochondral chondroma), benign chondroma, benign chondromatous osteoma, and giant cell tumor; cancers of the nervous system, for example, skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma, glioblastoma multiforme, glioblastoma, congenital tumors), spinal cord neurofibroma, meningioma, glioma, sarcoma); gynecological cancers, for example, uterus (endometrial carcinoma), cervix (cervical carcinoma, precancerous cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); hematologic cancers, e.g. blood (myeloid leukemia (acute) and chronic), acute lymphocytic leukemia, chronic lymphocytic leukemia, myeloproliferative diseases, multiple myeloma, myelodysplastic syndrome), hodgkin's disease, non-Hodgkin' s lymphoma (malignant lymphoma); cancers of the skin site, for example, malignant melanoma, basal cell carcinoma, squamous cell carcinoma, kaposi's sarcoma, moles dysplastic nevi, lipoma, hemangioma, dermatofibroma, keloids, psoriasis; and cancers of the adrenal sites, e.g. neuroblastoma; and the like.

In an embodiment, the KRAS G12D associated cancer is lung cancer.

In an embodiment, the KRAS G12D associated cancer is selected from a group consisting of: non-small cell lung cancer, small cell lung cancer, colorectal cancer, rectal cancer, or pancreatic cancer.

Another aspect of the present disclosure provides a method for treating cancer in a subject in need thereof, the method including: (a) determining that the cancer is associated with a KRAS G12D mutation (e.g. a KRAS G12D associated cancer); and
(b) administering to the subject a therapeutically effective amount of the compound, or a pharmaceutically acceptable salt, stereoisomer, solvate or prodrug thereof according to the first aspect of the present disclosure; or administering to the subject a therapeutically effective amount of the pharmaceutical composition according to the second aspect of the present disclosure.

In an embodiment, the administration is accomplished by a route selected from: parenteral, intraperitoneal, intradermal, intracardiac, intraventricular, intracranial, intracerebrospinal, intraluminal, intrasynovial, intrathecal, intramuscular injection, intravitreal injection, intravenous injection, intraarterial injection, oral, buccal, sublingual, transdermal, topical, intratracheal, rectal, subcutaneous and topical administrations.

Another aspect of the present disclosure provides a method for inhibiting the activity of KRAS G12D in a cell, including the steps of: contacting the cell with the compound, or pharmaceutically acceptable salt, stereoisomer, solvate or prodrug thereof according to the first aspect of the present disclosure; or contacting the cell with the pharmaceutical composition according to the second aspect of the present disclosure.

Another aspect of the present disclosure provides use of the compound, or pharmaceutically acceptable salt, stereoisomer, solvate or prodrug thereof according to the first aspect described above, or the pharmaceutical composition according to the second aspect described above, in preparation of an KRAS G12D inhibitor.

As used herein, the term "subject" refers to an animal, particularly a mammal, perferably human.

As used herein, the term "effective amount" or "therapeutically effective amount" refers to a sufficient amount of a non-toxic drug or medicament that can achieve the expected effect. In embodiments of the present disclosure, twhen treating a patient according to the present invention, the amount of a drug is given depending on many factors, such as the specific dosage regimen, the disease or condition type and its severity, and the peculiarity (e.g. body weight) of the subject or host in need of treatment.e.g., body weight) of the subject or host in need of treatment. However, in accordance with the particular ambient conditions, including, for example, the adopted specific drug and administration route, the treatment condition, and the treated subject or host, the administered dosage may be conventionally determined by the known method in the art.Usually, for dosages used for treating an adult, the administered dosage is typically in a range of 0.02-5000 mg/day, for example, about 1-1500 mg/day. The desired dose can be conveniently shown as a single dose, or divided doses administered simultaneously (or in a short time) or at appropriate intervals, for example, two, three, four or more divided doses per day. It will be understood by those skilled in the art that, although the above-mentioned dosage range is given, the specific effective amount may be appropriately adjusted according to the patient's condition in conjunction with the physician' s diagnosis.

As used herein, the term "pharmaceutically acceptable salt" refers to a salt of a compound of the present disclosure that is pharmaceutically acceptable and has the pharmacological activity of the parent compound. Such salts include: acid addictive salts formed with inorganic acids such as nitric acid, phosphoric acid, carbonic acid and the like, or organic acids, such as propionic acid, hexanoic acid, cyclopentanoic acid, glycolic acid, pyruvic acid, gluconic acid, stearic acid, muconic acid and the like, or salts formed by replacing acidic protrons present on the parent compoundwith metal ions, such as alkali metal ion or alkaline earth metal ion; or a coordination compound formed with organic bases such as ethanolamine and the like. The pharmaceutically acceptable salts of the present disclosure can be synthesized from the parent compound which contains acidic groups or basic groups by conventional chemical methods. Generally, such salts are prepared by: reacting these compounds in the form of free acids or bases with a stoichiometric amount of an appropriate base or acid in water or an organic solvent or a mixture of the two. Generally, nonaqueous media such as ether, ethyl acetate, ethanol, isopropanol, or acetonitrile are preferred. In addition to the form of salts, the compounds provided herein exist in the form of prodrug. Prodrugs of the compounds described herein readily undergo chemical changes under physiological conditions and thus transformed into the compounds of the present disclosure. In addition, prodrugs can be converted to the compounds of the present disclosure by chemical or biochemical methods in an in vivo environment.

As used herein, the term "solvate" refers to a substance formed by a compound of the present invention combined with a pharmaceutically acceptable solvent. Pharmaceutically acceptable solvents include acetic acid and the like. Solvates include stoichiometric solvates and non-stoichiometric solvates. Some compounds of the present invention can be present in a non-solvated or solvated form. In general, the solvated form is equivalent to unsolvated form and is intended to be encompassed within the scope of the present disclosure.

As used herein, the term "stereoisomer" includes conformational isomers and configurational isomers, wherein the configurational isomers primarily include cis-trans isomers and optical isomers. The compounds described herein may be present in the form of stereoisomers, and thus encompass all possible stereoisomeric forms, including but not limited to cis-trans isomers, tautomers, enantiomers, diastereomers, atropisomers, and the like, as well as any combination or any mixture of the aforementioned stereoisomers, such as meso-forms, racemates, equal mixtures of atropisomers, and the like. The compounds of the present disclosure may also be present in the form of any combination or any mixture of the aforementioned stereisomers, for example, a mixture of equal amounts of a mesomer, a raceme, and an atropisomer, for example, a single enantiomer, a single diastereomer or a mixture of them, or a single atropisomer or a mixture thereof. When the compounds described herein contain olefinic double bonds, unless specified otherwise, they include cis-isomers, trans-isomers and any combination thereof. Atropisomers of the present disclosure are stereoisomers based on axial or planar chirality resulting from restricted intramolecular rotation. As a drug, a stereoisomer having excellent activity is preferable. The compounds of the present disclosure have optical isomers derived from asymmetric carbon and the like, and if necessary, a single isomer can be resolved by methods known in the art, for example, crystallization or chiral chromatography and the like. Isomeric compounds obtained by the resolution can be assigned with any specific structure for each isomer.

As used herein, the term "C1-C6 alkyl" refers to straight and branched aliphatic groups consisting of 1 to 6 carbon atoms, preferably, C1-C4 alkyl or C1-C3 alkyl. Examples of alkyl include, but are not limited to, methyl, ethyl, propyl, isopropyl, butyl, isobutyl, sec-butyl, tert-butyl, pentyl, and hexyl.

As used herein, the term "C1-C6 haloalkyl" refers to C1-C6 alkyl in which one or more hydrogen are substituted with halogen. The definition of alkyl is as previously described, preferably, C1-C3 halogenated alkyl or C1-C4 halogenated alkyl. Examples of halogenated alkyl include, but are not limited to, trifluoromethyl, difluoromethyl, and monofluoromethyl.

As used herein, the term "C1-C6 deuterated alkyl" refers to C1-C6 alkyl in which one or more hydrogen are substituted with a deuterium atom. The definition of alkyl is as previously described, preferably, C1-C3 deuterated alkyl or C1-C4 deuterated alkyl. Examples of halogenated alkyl include, but are not limited to, trifluoromethyl, difluoromethyl, and monofluoromethyl.

As used herein, the term "C1-C6 alkoxyl" refers to an -O-C1-C6 alkyl, preferably, C1-C3 alkoxyl or C1-C4 alkoxyl. The definition of alkyl is as previously described.

As used herein, the term "C1-C6 halogenated alkyl" refers to an O-C1-C6 halogenated alkyl, preferably, C1-C3 halogenated alkoxyl or C1-C4 halogenated alkoxyl. The definition of halogenated alkyl is as previously described.

As used herein, the term "C1-C6 deuterated alkyl" refers to an O-C1-C6 deuterated alkyl, preferably C1-C3 deuterated alkoxyl or C1-C4 deuterated alkoxyl. The definition of deuterated alkyl is as previously described.

As used herein, the term "C1-C6 cyanoalkyl" refers to a-C1-C6 alkyl-cyano, preferably, C1-C3 cyanoalkyl or C1-C4 cyanoalkyl. The defintion of alkyl is as previously described. As used herein, the term "C2-C6 alkynyl" refers to straight and branched aliphatic groups consisting of 2-6 carbon atoms having 1 or 2 carbon-carbon triple bonds, preferably, C2-C4-alkynyl or C2-C3-alkynyl. Examples of alkynyl include, but are not limited to, ethynyl, propynyl, butynyl, and the like.

As used herein, the term "C2-C6 alkenyl" refers to straight and branched aliphatic groups consisting of 2-6 carbon atoms having 1 or 2 carbon-carbon double bonds, preferably, C2-C4 alkenyl or C2-C3 alkenyl. Examples of alkenyl include, but are not limited to, ethenyl, propenyl, butenyl, and the like.

As used herein, the term "C1-C6 hydroxyalkyl" refers to -C1-C6 alkyl-hydroxyl (OH), preferably, C1-C3 hydroxyalkyl (i.e. -C1-C3 alkyl-hydroxy) or C1-C4 hydroxyalkyl (i.e. -C1-C4 alkyl-hydroxy). The definition of alkyl is as previously described.

As used herein, the term "C2-C6 hydroxyalkynyl" refers to-C2-C6 alkynyl-OH. Alkynyl is as defined above, preferably, C2-C4 hydroxyalkynyl or C2-C3 hydroxyalkynyl.

As used herein, the term "C2-C6 hydroxyalkenyl" refers to -C2-C6 alkenyl-OH. Alkenyl is as defined above, preferably, C2-C4 hydroxyalkenyl or C2-C3 hydroxyalkenyl.

As used herein, the term "C1-C4 alkylene" refer to C1-C4 alkyl, as defined above, that is positioned between and serves to connect two other portions of the molecule. Typical alkylene includes, but is not limited to, methylene, ethylene, propylene, and butylene.

As used herein, the terms "cycloalkyl" and "cycloalkyl ring" are used interchangeably to refer to saturated monocyclic or polycyclic hydrocarbyls, including, for example, monocyclic cycloalkyl, spirocycloalkyl, fused cycloalkyl, and bridged cycloalkyl. In the present disclosure, the ring carbon atoms of the cycloalkyl may be optionally substituted with 1, 2 or 3 oxo groups to form a cyclic ketone structure. For example, the term "C3-C20 cycloalkyl" refers to cycloalkyl having 3 to 20 ring carbon atoms, including monocyclic cycloalkyl, spirocycloalkyl, fused cycloalkyl, and bridged cycloalkyl,preferably, C3-C12 cycloalkyl (more preferably C3-C8 monocyclic cycloalkyl), C5-C20 spirocycloalkyl, C5-C20 fused cycloalkyl or C5-C20 bridged cycloalkyl.

The term "C3-C8 monocyclic cycloalkyl" refers to saturated monocyclic hydrocarbyls having 3 to 8 ring carbon atoms, preferably C3-C6 monocyclic cycloalkyl or C4-C6 monocyclic cycloalkyl, more preferably C3, C4, C5 or C6 monocyclic cycloalkyl. Specific examples of monocyclic cycloalkyl include, but are not limited to, cyclopropyl, cyclobutyl, cyclopentyl, cyclohexyl, cycloheptyl, cyclooctyl, and the like.

As used herein, the terms "spirocycloalkyl" and "spirocycloalkyl ring" refer to polycyclic hydrocarbyls formed by sharing one carbon atom (referred to as a spiro atom) between two or more monocyclic rings. Spirocycloalkyl is classified as monospirocycloalkyl, dispirocycloalkyl and polyspirocycloalkyl according to the number of spiro atoms shared from ring to ring. The term "C5-C20 spirocycloalkyl" refers to polycyclic hydrocarbyls having 5 to 20 ring carbon atoms, wherein the monocyclic ring sharing a spiro atom is a C3-C8 monocyclic cycloalkyl ring; preferably, C6-C14 spirocycloalkyl; more preferably, C6-C14 mono spirocycloalky; further preferably C7-C11 spirocycloalkyl; sill more preferably, a 7- to 11- membered mono spirocycloalkyl; most preferably, C7 (C4 monocyclic cycloalkyl ring/C4 monocyclic cycloalkyl ring), C8 (C4 monocyclic cycloalkyl ring/C5 monocyclic cycloalkyl ring), C9 (C4 monocyclic cycloalkyl ring/C6 monocyclic cycloalkyl ring, C5 monocyclic cycloalkyl ring/C5 monocyclic cycloalkyl ring), C10 (C5 monocyclic cycloalkyl ring/C6 monocyclic cycloalkyl ring) or C11 (C6 monocyclic cycloalkyl ring/C6 monocyclic cycloalkyl ring) monocyclic spirocycloalkyl. Specific examples of spirocycloalkyl include, but are not limited to:

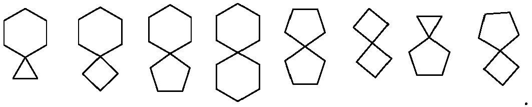

These spirocycloalkyl may be attached to the remainder of the molecule through any one of the ring atoms.

As used herein, the terms "fused cycloalkyl" and "fused cycloalkyl ring" refer to two or more monocyclic hydrocarbyls formed by sharing a pair of adjacent carbon atoms. Bicyclic, tricyclic, tetracyclic or polycyclic fused cycloalkyl can be classified according to the number of rings formed. The term "C5-C20 fused cycloalkyl" refers to polycyclic hydrocarbyls having 5 to 20 ring carbon atoms, wherein the monocyclic ring sharing apair of adjacent carbon atoms is a C3-C8 monocyclic cycloalkyl ring, preferably C6-C14 fused cycloalkyl, more preferably C6-C14 doubly fused cycloalkyl, more preferably C7-C10 fused cycloalkyl, more preferably C7-C10 doubly fused cycloalkyl, most preferably C8 (C5 monocyclic cycloalkyl ring is fused to a C5 monocyclic cycloalkyl ring), C9 (C5 monocyclic cycloalkyl ring is fused to a C6 monocyclic cycloalkyl ring) or C10 (C6 monocyclic cycloalkyl ring is fused to a C6 monocyclic cycloalkyl ring) doubly fused cycloalkyl. Specific examples of fused cycloalkyl include, but are not limited to:

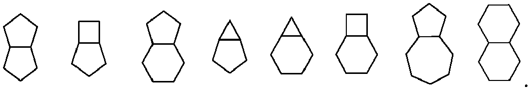

These fused cycloalkyl may be attached to the remainder of the molecule through any one of the ring atoms.

As used herein, the terms "bridged cycloalkyl" and "bridged cycloalkyl ring" refer to a polycyclic hydrocarbyl formed between two or more monocyclic rings by sharing two carbon atoms that are not directly connected. Bicyclic, tricyclic, tetracyclic or polycyclic bridged cycloalkyl can be classified according to the number of constituent rings. The term "C5-C20 bridged cycloalkyl" refers to a polycyclic hydrocarbyl having 5 to 20 ring carbon atoms in which any two rings share two carbon atoms that are not directly connected, preferably C6-C14 bridged cycloalkyl, more preferably C7-C10 bridged cycloalkyl. Specific examples of bridged cycloalkyl include, but are not limited to:

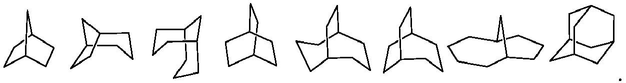

These bridged cycloalkyl may be attached to the remainder of the molecule through any one of the ring atoms.

For example, examples of cycloalkyl herein include, but are not limited to, cyclopropyl, cyclobutyl, cyclopentyl, cyclopentenyl, cyclohexyl, cyclohexenyl, cycloheptyl, cyclooctyl, bicyclo[1.1.1]pentyl.

As used herein, the term "cycloalkoxyl" refers to an -O-cycloalkyl, wherein cycloalkyl is as previously defined.

As used herein, the terms "heterocyclyl" and "heterocyclyl ring" are used interchangeably, and refer to saturated or partially unsaturated monocyclic or polycyclic hydrocarbyls, including, for example, monocyclic heterocyclyl, spiro heterocyclyl, fused heterocyclyl, and bridged heterocyclyl. Ring carbon atoms of the heterocyclyls described in the presetn disclosure may be optionally substituted with 1, 2, or 3 oxo groups to form a structure of cyclic ketone, cyclic lactone, or cyclic lactam. For example, the term "3-to 20-membered heterocyclyl" refers to a saturated or partially unsaturated monocyclic or polycyclic hydrocarbyls having 3 to 20 ring atoms, wherein the one or more (preferably 1, 2, 3 or 4) ring atoms are heteroatoms selected from nitrogen, oxygen or S(=O)ₘ' (where m' is an integer of from 0 to 2), but excludes the ring moieties O-O-, -O-S- or -S-S-, the remaining ring atoms being carbon. When the ring atom is a nitrogen atom, it may be substituted or unsubstituted (i.e. N or NR, R being hydrogen or a further substituent as already defined herein). The 3-to 20-membered heterocyclyl described in the present disclosure includes a monocyclic heterocyclic (e.g. a 3-to 8-membered monocyclic heterocyclyl), a 5-to 20-membered spiro heterocyclyl, a 5-to 20-membered fused heterocyclyl and a 5-to 20-membered bridged heterocyclyl.

As used herein, the term "3-to 20-membered nitrogen-containing heterocyclyl" refers to a saturated or partially unsaturated monocyclic hydrocarbyl having 3 to 20 ring atoms containing at least 1 nitrogen atom, wherein the group is attached to the remainder of the molecule through the nitrogen atom; and the group optionally also contains one or two other heteroatoms selected from nitrogen, oxygen or S(=O)ₘ' and used as ring atoms, wherein m' is an integer of from 0 to 2. The 3-to 20-membered nitrogen-containing heterocyclyl described herein includes a monocyclic heterocyclyl (e.g. a 3-to 8-membered monocyclic nitrogen-containing heterocyclyl), a 5-to 20-membered nitrogen-containing spiro heterocyclyl, a 5-to 20-membered nitrogen-containing fused heterocyclyl, and a 5-to 20-membered nitrogen-containing bridged heterocyclyl.

As used herein, the terms "3-to 8-membered monocyclic heterocyclyl" and "3-to 8-membered monocyclic heterocyclyl ring" refer to a saturated or partially unsaturated monocyclic hydrocarbyl having 3 to 8 ring atoms, wherein 1, 2 or 3 are heteroatoms selected from nitrogen, oxygen or S(=O)ₘ' , wherein m' is an integer of from 0 to 2, preferably 3-to 6-membered monocyclic heterocyclyl having 3 to 6 ring atoms, wherein 1 or 2 are heteroatoms, more preferably 4 to 6 membered monocyclic heterocyclyls having 4 to 6 ring atoms, wherein 1 or 2 are heteroatoms, more preferably 5-or 6-membered monocyclic heterocyclyls having 5 or 6 ring atoms, wherein 1 or 2 are heteroatoms. When the heteroatom is a nitrogen atom, the nitrogen atom may be substituted or unsubstituted (i.e. N or NR, R being hydrogen or a further substituent as already defined herein). When the heteroatom is a sulfur atom, the sulfur atom may be optionally oxidized (i.e. S(=O)ₘ', m' being an integer of from 0 to 2). Ring carbon atoms of the monocyclic heterocyclyls may be optionally substituted with 1, 2, or 3 oxo groups to form a cyclic ketone, cyclic lactone, or cyclic lactam structure. Specific examples of monocyclic heterocyclyls include, but are not limited to, aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, tetrahydropyrrolidine, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholin-1,1-dioxide, tetrahydropyran, 1,2-dihydroazetidine, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4 (3H)-one, pyrimidin-4 (1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrole-2,5-dione, furan-2(3H)-one, furan-2(5H)-one, 1,3-dioxol-2-one, oxazol-2(3H)-one, 1,3-dihydro-2H-imidazol-2-one, furan -2,5-dione, 3,6-dihydropyridin-2(1H)-one, pyridine-2,6-(1H, 3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine, 1,2,3,4-tetrahydropyrimidine and the like.

As used herein, the term "3-to 6-membered nitrogen-containing heterocyclyl" refers to a saturated or partially unsaturated monocyclic hydrocarbyl having 3 to 6 ring atoms containing at least 1 nitrogen atom as a ring atom, wherein the group may be attached to other parts of the molecule either through the nitrogen atom or through other ring atoms of the group; and the group optionally also contains one or two other heteroatoms selected from nitrogen, oxygen or S(=O)ₘ' and used as ring atoms, wherein m' is an integer of from 0 to 2. Specific examples may be selected from 3 to 8 membered monocyclic heterocyclyls including, but not limited to, tetrahydropyrrolyl, oxazolidinyl, isoxazolidinyl, piperidinyl, piperazinyl, morpholinyl, thiomorpholinyl, azetidinyl and the like.

As used herein, the terms "spiroheterocyclyl" and "spiroheterocyclyl ring" refer to polycyclic heterocyclyls formed by sharing one carbon atom (referred to as a spiro atom) between two or more saturated or partially unsaturated monocyclic rings, wherein one or more (e.g. 1, 2, or 3) ring atoms are heteroatoms selected from nitrogen, oxygen, or S(=O)ₘ' (where m' is an integer of from 0 to 2) and the remaining ring atoms are carbon. When the heteroatom is a nitrogen atom, the nitrogen atom may be substituted or unsubstituted (i.e. N or NR, R being hydrogen or a further substituent as already defined herein). Each single ring may contain one or more double bonds, but none of the rings have a completely conjugated π-electron system. Spiroheterocyclyls are classified as mono-, bi- or poly-spiroheterocyclyls depending on the number of spiro atoms shared between the rings. The term "5-to 20-membered spiroheterocyclyl" refers to a spiroheterocyclyl having 5 to 20 ring atoms, wherein one of the monocyclic rings that share a spiro atom is a 3-to 8-membered monocyclic heterocyclyl ring and the other monocyclic ring is a 3-to 8-membered monocyclic heterocyclyl ring or a 3-to 8-membered monocyclic cycloalkyl ring, preferably 6-to 14-membered spiro heterocyclyls having 6 to 14 ring atoms, wherein 1 or 2 are heteroatoms, more preferably 7- to 11- membered spiro heterocyclyls having 7 to 11 ring atoms, wherein 1 or 2 are heteroatoms, most preferably 7-membered (4-membered monocyclic heterocyclyl ring/4-membered monocyclic heterocyclyl ring or 4-membered monocyclic heterocyclyl ring/4-membered monocyclic cycloalkyl or 4-membered monocyclic cycloalkyl ring/4-membered monocyclic heterocyclyl ring), 8-membered (4-membered monocyclic heterocyclyl ring/5-membered monocyclic heterocyclyl ring), 9-membered (4-membered monocyclic heterocyclyl ring/6-membered monocyclic heterocyclyl ring, 5-membered monocyclic heterocyclyl ring/5-membered monocyclic heterocyclyl ring), 10-membered (5-membered monocyclic heterocyclyl ring/6-membered monocyclic heterocyclyl ring) or 11-membered (6-membered monocyclic heterocyclyl ring/6-membered monocyclic heterocyclyl ring) mono-spiro heterocyclyl. Specific examples of spiroheterocyclyls include, but are not limited to:

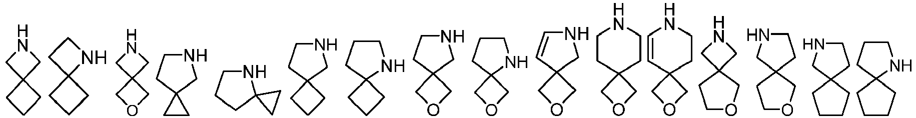

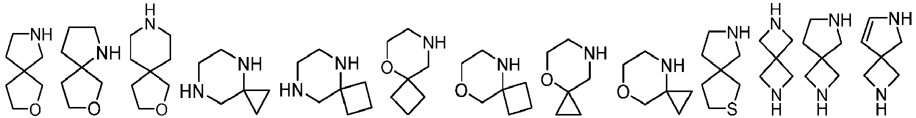

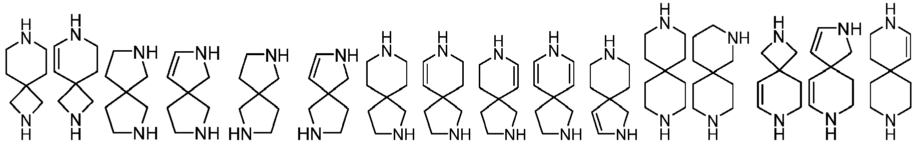

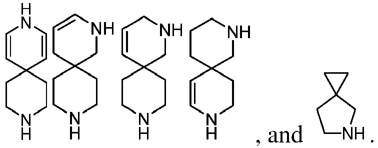

These spiroheterocyclyls may be attached to the remainder of the molecule through any suitable ring atom.

As used herein, the term "5-to 20-membered nitrogen-containing spiroheterocyclyl" refers to a spiroheterocyclyl having 5 to 20 ring atoms containing at least 1 nitrogen atom, wherein the group is attached to the remainder of the molecule through the nitrogen atom; and the group optionally also contains as ring atoms one or two or three further heteroatoms selected from nitrogen, oxygen or S(=O)ₘ', wherein m' is an integer of from 0 to 2.

As used herein, the terms "fused heterocyclyl" and "fused heterocyclyl ring" refer to a polycyclic heterocyclyl in which two or more saturated or partially unsaturated monocyclic rings are formed by sharing a pair of adjacent ring atoms, wherein one or more (e.g. 1, 2, or 3) ring atoms are heteroatoms selected from nitrogen, oxygen, or S(=O)ₘ' (where m' is an integer of from 0 to 2), the remaining ring atoms being carbon. When the heteroatom is a nitrogen atom, the nitrogen atom may be substituted or unsubstituted (i.e. N or NR, R being hydrogen or a further substituent as already defined herein). Each single ring may contain one or more double bonds, but none of the rings have a completely conjugated π-electron system. The shared a pair of adjacent ring atoms may be C-C or N-C. Bicyclic, tricyclic, tetracyclic or polycyclic fused heterocyclyls can be classified according to the number of constituent rings. The term "5-to 20-membered fused heterocyclyl" refers to fused heterocyclyls having 5 to 20 ring atoms, wherein the single ring sharing a pair of adjacent ring atoms is a 3-to 8-membered monocyclic heterocyclyl ring, preferably 6-to 14-membered fused heterocyclyls having 6 to 14 ring atoms, wherein 1 or 2 are heteroatoms, more preferably 6- to 10- membered fused heterocyclyls having 6 to 10 ring atoms, wherein 1 or 2 are heteroatoms;, more preferably 8- to 10- membered fused heterocyclyls having 8 to 10 ring atoms, wherein1 or 2 are heteroatoms, most preferably 8-membered (a 5-membered monocyclic heterocyclyl ring fused to a 5-membered monocyclic heterocyclyl ring), 9-membered (a 5-membered monocyclic heterocyclyl ring fused to a 6-membered monocyclic heterocyclyl ring) or 10-membered (a 6-membered monocyclic heterocyclyl ring fused to a 6-membered monocyclic heterocyclyl ring) bicyclic fused heterocyclyls. Specific examples of fused heterocyclyl groups include, but are not limited to:

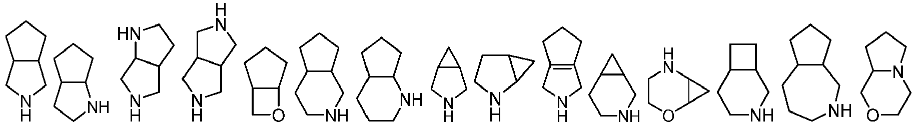

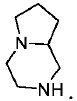

These fused heterocyclic groups may be attached to the remainder of the molecule through any suitable ring atom.

As used herein, the term "5-to 20-membered nitrogen-containing fused heterocyclyl" refers to a fused heterocyclyl having 5 to 20 ring atoms containing at least 1 nitrogen atom, wherein the group is attached to the remainder of the molecule through the nitrogen atom; and the group optionally also contains as ring atoms one or two or three further heteroatoms selected from nitrogen, oxygen or S(=O)ₘ', wherein m' is an integer of from 0 to 2.

As used herein, the terms "bridged heterocyclyl" and "bridged heterocyclyl ring" refer to a polycyclic heterocyclyl in which two or more saturated or partially unsaturated monocyclic rings are formed by sharing two ring atoms that are not directly connected, wherein one or more (e.g. 1, 2, or 3) ring atoms are heteroatoms selected from nitrogen, oxygen, or S(=O)ₘ' (where m' is an integer of from 0 to 2) and the remaining ring atoms are carbon. Bicyclic, tricyclic, tetracyclic or polycyclic bridged cycloalkyl can be classified according to the number of constituent rings. The term "5-to 20-membered bridged heterocyclyl" refers to a saturated or partially unsaturated polycyclic heterocyclic groups having 5 to 20 ring atoms in which any two rings share two ring atoms that are not directly connected, each monocyclic ring may contain one or more double bonds, but no ring has a completely conjugated π-electron system, preferably 6- to 14-membered bridged heterocyclyls, more preferably 7- to 10-membered bridged heterocyclyls. Specific examples of bridged heterocyclyls include, but are not limited to:

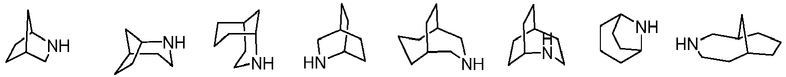

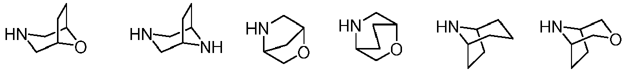

These bridged heterocyclyls may be attached to the remainder of the molecule through any suitable ring atom.

As used herein, the term "5-to 20-membered nitrogen-containing bridged heterocyclyl" refers to a bridged heterocyclyl having 5 to 20 ring atoms containing at least 1 nitrogen atom, wherein the group is attached to the remainder of the molecule through the nitrogen atom; and the group optionally also contains as ring atoms one or two or three further heteroatoms selected from nitrogen, oxygen or S(=O)ₘ', wherein m' is an integer of from 0 to 2.

In the present disclosure, each of the above-mentioned heterocyclyls may be optionally substituted, and when substituted, the substituent is preferably one or more of the substituents described in the present disclosure.

Specifically, specific examples of heterocyclyls described herein include, but are not limited to, epoxyl, azetidinyl, tetrahydrofuranyl, tetrahydropyranyl, pyrrolidinyl, pyrrolidonyl, piperidinyl, piperazinyl, imidazolyl, imidazopyridinyl, thiazolyl, oxazolidinyl, oxazolidinedionyl, decahydroquinolinyl, piperidonyl, morphinyl, azabicyclohexyl, azabicycloheptyl, azabicyclooctyl, azabicyclononyl, azabicyclodecyl, azaspiroheptyl, azaspirooctyl, azaspirononyl, azaspirodecyl, tetrahydrospiro[cyclopropane-1,2'-pyrrolizinyl, hexahydro-1H-pyrrolizinyl, hexahydro-1H-pyrrolo [2, 1-c] [1,4] oxazinyl, octahydroindolizinyl, oxaspiroheptyl, oxaspirooctyl, oxaspirononyl, oxaspirodecyl, diazaspirononyl, oxabicyclohexyl, oxabicycloheptyl, oxabicyclooctyl, hexahydropyrrolizinyl 4(1H)-oxide.

As used herein, the term "aryl" refers to an all-carbon monocyclic, all-carbon non-fused polycyclic (rings being linked by a covalent bond, rather than fused), or all-carbon fused polycyclic (i.e. rings sharing a pair of adjacent carbon atoms) groups, where the group has at least aromatic ring, i.e., has a conjugated π-electron system. For example, the term "C6-C14 aryl" refers to aryls having 6 to 14 ring atoms, preferably C6-C10 aryl. The C6-C14 aryl in the present disclosure includes monocyclic aryl, non-fused polycyclic aryl and aromatic fused polycyclices, where examples of monocyclic aryl include phenyl, and examples of the non-fused polycyclic aryl include biphenyl and the like.

In the present disclosure, when the C6-C14 aryl is aromatic fused polycycles, the aromatic fused polycycles may be a polycyclic group formed by a monoaryl ring fused with one or more monoaryl rings, non-limiting examples thereof include naphthyl, anthryl, and the like.

In some embodiments of the disclosure, when the C6-C14 aryl group is an aromatic fused polycycles, the aromatic fused polycycles may also be a polycyclic group formed by a monoaryl ring (e.g. phenyl) fused with one or more non-aromatic rings, wherein the ring linked to the parent structure is an aromatic ring or a non-aromatic ring. The non-aromatic ring includes, but is not limited to, 3-to 6-membered monocyclic heterocyclyl rings (preferably a 5-or 6-membered monocyclic heterocyclyl ring, where the ring carbon atoms may be substituted with 1 to 2 oxo groups to form a cyclic lactam or cyclic lactone structure), a 3-to 6-membered monocyclic cycloalkyl ring (preferably a 5-or 6-membered monocyclic cycloalkyl ring, wherein the ring carbon atoms may be substituted with 1 or 2 oxo groups to form a cyclic ketone structure). Polycycles in which a monoring is fused to one or more non-aromatic rings as described above may be attached to other groups or to the parent structure via a nitrogen or carbon atom, the ring to which the parent structure is attached being a monoaryl ring or a non-aromatic ring.

In the present disclosure, the above-mentioned kinds of aryls may be substituted or unsubstituted, and when substituted, the substituent is preferably one or more of the substituent groups described in the present disclosure.

As used herein, the term "aryl" refers to a C6-C14 aromatic radical comprising 1 to 3 aromatic rings. It may be optionally substituted with one or more R⁶ or one or more R⁷. In an embodiment, the aryl is C6-C10 aryl. Examples of aryl include, but are not limited to, phenyl, naphthyl, anthracenyl, fluorenyl, and dihydrobenzofuranyl. "Aryl" also refers to a bicyclic or tricyclic ring system wherein one or both rings in the aryl, respectively, may be saturated or partially saturated. If the aryl contains two saturated rings, the two saturated rings may be fused ring systems or spiro ring systems. Examples of aryl comprising two saturated rings (and being a spiro ring system) include the following:

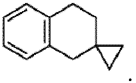

As used herein, the term "aryl C1-C6 alkyl" or "aralkyl" is meant to include one aryl covalently linked to one alkyl. The aryl is as defined above and alkyl is as defined above, and any or all of aryl or alkyl may be independently optionally substituted or unsubstituted. An example of the aralkyl is (C6-C10) aryl (C1-C6) alkyl-. Specific examples include, but are not limited to, benzyl, phenethyl and naphthylmethyl. An example of a substituted aryl C1-C6 alkyl is where the alkyl is substituted with hydroxyalkyl.

The term "heteroaryl", as used herein, refers to a monocyclic or fused polycyclic (i.e. sharing adjacent pairs of ring atoms, which may be C-C or N-C) group wherein the nitrogen and sulfur atoms may optionally be oxidized and the nitrogen atom may optionally be quaternized, with ring atoms being substituted with at least one heteroatom independently selected from nitrogen, oxygen, or sulfur. The heteroaryl has 6, 10 or 14 π electrons shared, with at least one ring in the group being aromatic. For example, the term "5- to 14-membered heteroaryl" refers to a heteroaryl having 5 to 14 ring atoms, wherein 1, 2, 3 or 4 are heteroatoms selected from nitrogen, oxygen or S(=O)ₘ', wherein m' is an integer of from 0 to 2, preferably 5- to 10-membered heteroaryls having 5 to 10 ring atoms, wherein 1, 2, 3 or 4 are heteroatoms. In the present disclosure, the 5- to 10-membered heteroaryl is a monocyclic heteroaryl (e.g. a 5-or 6-membered monocyclic heteroaryl), a fused bicyclic heteroaryl (e.g. an 8-to 10-membered bicyclic heteroaryl), or a fused tricyclic heteroaryl.

As used herein, the term "5- or 6-membered monocyclic heteroaryl" refers to a monocyclic heteroaryl having 5 or 6 ring atoms, wherein 1, 2 or 3 are heteroatoms selected from nitrogen, oxygen or S(=O)ₘ', wherein m' is an integer of from 0 to 2. Specific examples of monocyclic heteroaryl include, but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine, and the like.

As used herein, the term "8- to 10-membered bicyclic heteraryl" refers to a fused bicyclic heteroaryl having 8 to 10 ring atoms, wherein 1, 2, 3, 4 or 5 are heteroatoms selected from nitrogen, oxygen or S(=O)ₘ', wherein m' is an integer of from 0 to 2. The fused bicyclic heteroaryl can be either a bicyclic group (preferably a 9-or 10-membered bicyclic heteroaryl ring) formed by a monoaryl ring (e.g. phenyl) fused to a monocyclic heteroaryl ring (preferably a 5-or 6-membered monocyclic heteroaryl ring), or a bicyclic group formed by a monocyclic heteroaryl ring (preferably a 5-or 6-membered monocyclic heteroaryl ring) fused with a monocyclic heteroaryl ring (preferably a 5-or 6-membered monocyclic heteroaryl ring).

Any adjacent two ring atoms, including C-C, N-C, N-N, attached on the monocyclic heteroaryl ring described above can be fused to cycloalkyl, heterocyclyl, aryl, or heteroaryl, such as monocyclic cycloalkyl, monocyclic heterocyclyl, monocyclic aryl, 5-or 6-membered monocyclic heteroaryl ring, as defined herein, to form a fused polycyclic ring. The two ring atoms attached on the monocyclic heteroaryl ring forming a fused ring with other rings are preferably C-C, including, but not limited to, the following forms:

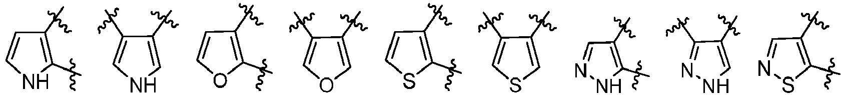

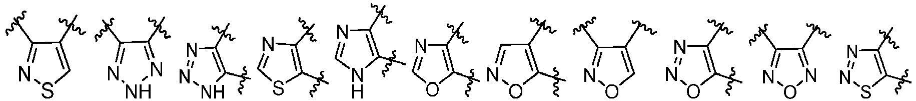

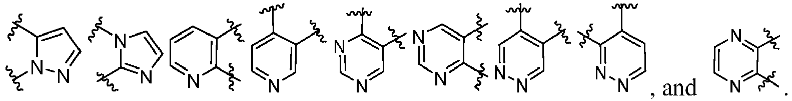

The above-mentioned groups are attached to the remainder of the molecule through the ring atom identified with " 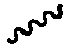 ".

Non-limiting examples of 8-to 10-membered bicyclic heteroaryls include: benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine, imidazo[1,2-b]pyridazine and the like.

Specific examples of bicyclic heteroaryls include, but are not limited to: 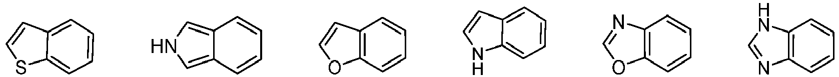 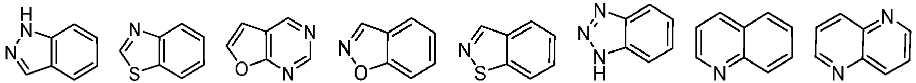 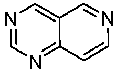 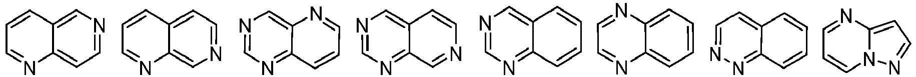 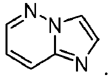 These groups may be attached to the remainder of the molecule through any suitable ring atom. The ring linked to the parent structure can be a monocyclic heteroaryl ring or a phenyl ring.

In some embodiments of the present disclosure, the fused bicyclic heteroaryl or fused tricyclic heteroaryl may be a polycyclic group formed by a monocyclic heteroaryl ring (preferably a 5-or 6-membered monocyclic heteroaryl ring) fused with one or more non-aromatic rings, wherein the ring linked to the parent structure is a monocyclic heteroaryl ring or a non-aromatic ring. The non-aromatic ring includes, but not limited to, a 3-to 6-membered monocyclic heterocyclyl ring (preferably a 5-or 6-membered monocyclic heterocyclyl ring, where the ring carbon atoms may be substituted with 1 to 2 oxo groups to form a cyclic lactam or cyclic lactone structure), a 3-to 6-membered monocyclic cycloalkyl ring (preferably a 5-or 6-membered monocyclic cycloalkyl ring, wherein the ring carbon atoms may be substituted with 1 or 2 oxo groups to form a cyclic ketone structure) etc. Polycycle in which a monocyclic heteraryl ring is fused to one or more non-aromatic rings as described above may be attached to other groups or to the parent structure via a nitrogen or carbon atom, the ring linked to the parent structure is a monocyclic heteraryl ring or a non-aromatic ring.

In the present disclosure, the above-mentioned kinds of heteraryls may be substituted or unsubstituted, and when substituted, the substituent is preferably one or more of the substituted groups described in the present disclosure.

As used herein, the term "acetyl" refers to -C(O)CH₃.

### DETAIL DESCRIPTION OF THE EMBODIMENTS

The compounds of the present disclosure can be prepared by a plurality of synthesis methods well known to those skilled in the art, including the specific embodiments set forth below, embodiments derived therefrom in combination with other chemical synthesis methods, and equivalents thereof well known to those skilled in the art. Preferred embodiments include, but are not limited to embodiments of the present disclosure. The part relevant to synthesis methods of intermediates or starting compounds herein is present separately in the preparative example illustrated in a example. It is not repeated in the remaining embodiments, if the same intermediate or starting compound is used.

The present disclosure will now be described in more detail by way of embodiments, but not intended to any disadvantageous limitation thereof. While the present disclosure has been described in detail and also disclosed specific embodiments thereof, it will be apparent to those skilled in the art that, various changes and modifications can be made regarding detailed embodiments of the present disclosure therein without departing from the spirits and scope of the present disclosure. Where specific conditions are not illustrated in the examples, they are carried out according to conventional conditions or those conditions suggested by the manufacturer. The used reagents or instruments without indicating the manufacturer, are conventional products commercially available.

Regarding high-performance liquid chromatogragraphy (HPLC), the following conditions may be adopted in the following examples, unless otherwise specified:
HPLC (formic acid method): column type: Waters XBridge C18, 190*250 mm, 5 µm; mobile phase system: A: 20 0.1% formic acid; B: preparative grade acetonitrile; flow rate: 15 ml/min; B%=20%-100%; column temperature: room temperature.
HPLC (ammonium bicarbonate method): column type: Waters XBridge C18, 190*250 mm, 5 µm; mobile phase system: A: 0.1% aqueous ammonium bicarbonate; B: preparative grade acetonitrile; flow Rate: 15 ml/min; B%=20%-100%; column temperature: room temperature.

### Preparative Example 1: Synthesis of tert-butyl (1R,5S)-2-(hydroxymethyl)-3,8-diazabicyclo[3.2.1]octane-8-carboxylate (A1)

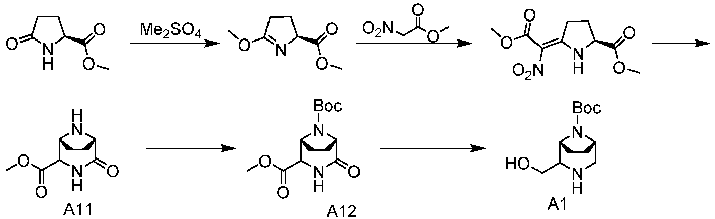

Step 1: (S)-Methyl 5-oxopyrrolidine-2-carboxylate (200 g, 1397 mmol) and dimethyl sulfate (134 mL) were added to a 1 L single-neck flask. Upon completion of the addition, the system reacted at 56°C for 18 h, and the reaction was monitored by TLC for completion. The mixture was cooled to room temperature, and triethylamine (292 mL) was added dropwise and stirred for 30 minutes. 400 mL of water was added, and the obtained mixture was extracted with ethyl acetate (400 mL×3). The organic phase was washed with saturated brine (400 mL), dried over anhydrous sodium sulfate, and filtered. The filtrate was rotoevaporated to obtain (S)-methyl 5-methoxyl-3,4-dihydro-2H-pyrrole-2-carboxylate (150 g, yield: 68.4%), which was a yellow oily compound. ES-API:[M+1]⁺=158.1.

Step 2: (S)-methyl 5-methoxy-3,4-dihydro-2H-pyrrole-2-carboxylate (150 g, 954.3 mmol) and methyl nitroacetate (123.8 g, 1040.2 mmol) were added to a 1 L single-neck flask. Upon completion of the addition, the system reacted at 60°C for 40 hours, and the reaction was monitored by nuclear magnetic resonance for completion. The reaction mixture was purified by column chromatography (DCM/EA=50/1) to obtain (S,Z)-methyl 5-(2-methoxy-1-nitro-2-oxoethylidene)pyrrolidine-2-carboxylate (80.0 g, yield: 34.2%), which was yellow solid. ES-API:[M+1]⁺=245.1.

Step 3: (S,Z)-methyl 5-(2-methoxy-1-nitro-2-oxoethylidene)pyrrolidine-2-carboxylate (20.0 g, 81.9 mmol), palladium on carbon (2.0 g) and methanol (600 mL) were added to a 1 L single-neck flask. Upon completion of the addition, the reaction system was replaced with hydrogen for 3 times and reacted at 60°C for 5 days. The reaction was monitored by LC-MS for completion, and the feeding was repeated for 4 batches. The mixture was filtered, and the filtrate was concentrated to obtain methyl (1R,5S)-4-oxo-3,8-diazabicyclo[3.2.1]octane-2-carboxylate (A11, 60 g, crude product). ES-API:[M+1]⁺=185.1

Step 4: The compound methyl (1R,5S)-4-oxo-3,8-diazabicyclo[3.2.1]octane-2-carboxylate (A11, 60.0 g, 325 mmol), triethylamine (65.9 g, 651.5 mmol) and dichloromethane (600 mL) were added to a 1 L single-neck bottle. The reaction system was cooled to 0°C, and di-tert-butyl dicarbonate (106.6 g, 488.6 mmol) was added. Upon completion of the addition, the system reacted at room temperature overnight, and the reaction was monitored by LC-MS for completion. Water (1 L) was added, and the obtained mixture was extracted with dichloromethane (1 L). The organic phase was rotoevaporated. The crude product was purified by column chromatography (dichlorohexane/methanol=20:1) to obtain 8-(tert-butyl) 2-methyl (1R,5S)-4-oxo-3,8-diazabicyclo[3.2.1]octane-2,8-dicarboxylate (A12, 70.0 g, yield: 75.8%) which was a yellow solid. ES-API:[M-Boc+1]⁺=285.1.

Step 5: compound 8-(tert-butyl) 2-methyl (1R,5S)-4-oxo-3,8-diazabicyclo[3.2.1]octane-2,8-dicarboxylate (A12, 20.0 g, 70.3 mmol) and tetrahydrofuran (300 mL) were added to a 500 mL three-neck flask. The reaction system was cooled to 0°C, and lithium aluminum tetrahydride (5.87 g, 154.7 mmol) was added in batches (temperature controlled below 5°C). Upon completion of the addition, the system reacted at room temperature overnight, and the reaction was monitored by LC-MS for completion. The reaction system was cooled to 0°C, and the reaction was quenched with water. The reaction mixture was stirred for 1 h and filtered. The filter cake was rinsed with dichloromethane/methanol (10:1), and the filtrate was concentrated to obtain a crude product. The crude product was prepared by reverse phase HPLC to obtain tert-butyl (1R,5S)-2-(hydroxymethyl)-3,8-diazabicyclo[3.2.1]octane-8-carboxylate (A1, 5.8 g, yield: 32.2%) which was yellow solid. ES-API:[M-Boc+1]⁺=243.2.

### Preparative Example 2: Synthesis of isomer (A1-1) and isomer (A1-2)

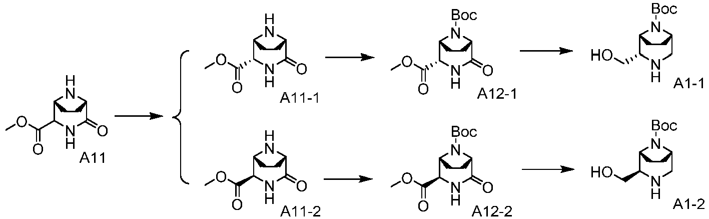

Intermediate A11 was resolved by referring to a resolution method in reference "Tetrahedron Letters, 45 (2004)1481-1483" to obtain methyl (1R,2R,5S)-4-oxo-3,8-diazabicyclo[3.2.1]octane-2-carboxylate (A11-2) and methyl (1R,2S,SS)-4-oxo-3,8-diazabicyclo[3.2.1]octane-2-carboxylate (A11-1). Starting from the two isomers A11-1 and A11-2, respectively, (1R,2R,5S)-2-hydroxymethyl)-3,8-diazabicyclo[3.2.1]octane-8-tert-butyl carboxylate (A1-2) and (1R,2S,5S)-2-(hydroxymethyl)-3,8-diazabicyclo[3.2.1]octane-8-tert-butyl carboxylate (A1-1) were synthesized by referring to step 4 and step 5 of Preparative Example 1, respectively. For A1-2, ¹H NMR (400 MHz, DMSO-d6): δ = 4.57 (t, J = 5.6 Hz, 1H), 3.94 (br s, 2H), 3.17 (br t, J = 5.6 Hz, 2H), 2.72 (br d, J = 10.4 Hz, 2H), 2.51-2.57 (m, 1H), 2.02-2.17 (m, 1H), 1.45-1.83 (m, 4H), 1.40 (s, 9H). ES-API:[M+1]⁺=243.2. For A1-1, ¹H NMR (400 MHz, CDCl₃): δ 4.28-4.02 (m, 2H), 3.53 (d, *J* = 7.2 Hz, 2H), 3.06-3.03 (m, 1H), 2.75-2.43 (m, 4H), 2.07-1.96 (m, 2H), 1.86-1.76 (m, 2H), 1.46 (s, 9H). ES-API:[M+1]⁺=243.2.

### Preparative Example 3: Synthesis of isomer (A2-1) and isomer (A2-2)

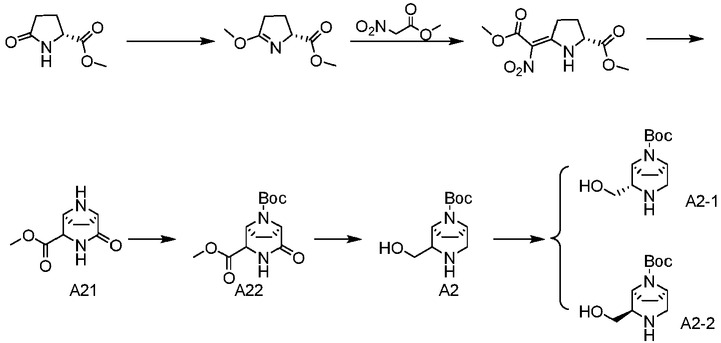

Step 1: (R)-Methyl 5-oxopyrrolidine-2-carboxylate (395.3 g, 2.762 mmol) was dissolved in 3500 mL of dichloromethane, and trimethyloxonium tetrafluoroborate (612.697 g, 4.142 mol, 1.5 eq) was added in batches at 5°C. The system reacted at 25°C for 24 hr, and the reaction was monitored by TLC for completion. The reaction solution was slowly poured into 5000 mL of saturated aqueous sodium carbonate solution, with a pH of 8-9. The solution was separated, and the obtained aqueous phase was extracted with 1500 mL of dichloromethane. The organic phases were combined, washed with saturated brine, dried over anhydrous magnesium sulfate, filtered, and rotoevaporated to obtain (R)-methyl 5-methoxy-3,4-dihydro-2H-pyrrole-2-carboxylate (406.7 g, 2.588 mol, yield: 93.702%) as a light yellow oily liquid. ES-API:[M+1]⁺=158.1.

Step 2: (R)-methyl 5-methoxy-3,4-dihydro-2H-pyrrole-2-carboxylate (406.7 g, 2.588 mol) was added to methyl nitroacetate (308.133 g, 2.588 mol, 1 eq). The system reacted at 60°C for 48 hr, and the reaction was monitored by nuclear magnetic resonance for completion. Column chromatography (petroleum ether:ethyl acetate=4:1-2:1) was performed to obtain 250.0 g of crude yellow oily liquid. The crude product was beaten, purified, filtered, and dried to obtain (R)-methyl 5-(2-methoxy-1-nitro-2-oxoethylidene)pyrrolidine-2-carboxylate (146.2 g, 598.687 mmol, yield: 23.136%) as a light yellow solid. ES-API:[M+1]⁺=245.1.

Step 3: In a 5L autoclave, (R)-methyl 5-(2-methoxy-1-nitro-2-oxoethylidene)pyrrolidine-2-carboxylate (146.2 g, 598.687 mmol) was dissolved in methanol (1500 mL) and tetrahydrofuran (1500 mL), and Pd(OH)₂/C(15.00 g, 20% purity) was added. The reaction system was replaced with hydrogen for 3 times and reacted at 40°C under 0.4 MPa for 72 hr. Hydrogen was replenished several times during the reaction. The reaction system was cooled to room temperature. The reaction solution was filtered and rotoevaporated to obtain methyl (1S,5R)-4-oxo-3,8-diazabicyclo [3.2.1]octane-2-carboxylate (A21, 110.3 g, crude product) which was yellow oily liquid. ES-API:[M+1]⁺=185.1.

Step 4: The crude product methyl (1S,5R)-4-oxo-3,8-diazabicyclo[3.2.1]octane-2-carboxylate (A21, 10.00 g, 54.291 mmol) was dissolved in water (50 mL) and tetrahydrofuran (50 mL). Na₂CO₃ (5.754 g, 54.291 mmol) was added and (Boc)₂O (11.849 g, 54.291 mmol) was added dropwise at 10°C. The system reacted at 20°C for 12 hr, and the reaction was monitored by TLC for completion. The reaction solution was filtered, and the filter cake was washed with 50 mL of ethyl acetate. The solution was separated, and the aqueous layer was extracted with 50 mL of ethyl acetate. The organic phases were combined and rotoevaporated, and column chromatography (petroleum ether: ethyl acetate=2:1~1:1) was preformed to obtain 8-(tert-butyl) 2-methyl (1S,5R)-4-oxo-3,8-diazabicyclo[3.2.1]octane-2,8-dicarboxylate (A22, 3.61 g, 12.698 mmol, yield: 23.388%) which was white solid. ES-API:[M-Boc+1]⁺=285.1.

Step 5: 8-(tert-butyl) 2-methyl (1S,5R)-4-oxo-3,8-diazabicyclo[3.2.1]octane-2,8-dicarboxylate (A22, 2.00 g, 7.035 mmol) was dissolved in tetrahydrofuran (20 mL), and lithium aluminum tetrahydride (400.489 mg, 10.552 mmol, 1.5 eq) was added in batches at 0°C. Upon completion of the addition, the system reacted at 20°C for 12 hr, and the reaction was monitored by TLC for completion. At 0°C, 0.4 mL of water, 0.4 mL of 15% sodium hydroxide solution and 1.2 mL of water were added dropwise successively to the reaction solution. Upon completion of the addition, anhydrous magnesium sulfate was added and stirred for 10 min. The obtained mixture was filtered and concentrated to obtain a crude product. The crude product was prepared by reverse phase HPLC to obtain tert-butyl (1S,5R)-2-hydroxymethyl)-3,8-diazabicyclo[3.2.1]octane-8-carboxylate (A2, 0.65 g, yield: 38%) as a light yellow solid. ES-API:[M-Boc+1]⁺=243.2.

Step 6: tert-butyl (1S,5R)-2(hydroxymethyl)-3,8-diazabicyclo[3.2.1]octane-8-carboxylate (A2, 100 mg) was chirally resolved [column type: CHIRALPAK-AY, 4.6*250 mm, 5 um, mobile phase: hexane: EtOH (0.1% diethylamine)=95:5 (v/v), flow rate: 1 mL/min, column temperature=37°C, detection wavelength: 210 nm] to obtain tert-butyl (1S,2S,5R)-2(hydroxymethyl)-3,8-diazabicyclo[3.2.1]octane-8-carboxylate (A2-1, 58 mg, peak 2, retention time: 10.391 min) and tert-butyl (1S,2R,5R)-2-(hydroxymethyl)-3,8-diazabicyclo[3.2.1]octane-8-carboxylate (A2-2, 12 mg, peak 1, retention time: 9.374 min). For A2-1, ¹H NMR (400 MHz, DMSO-*d6*): δ 4.57 (br t, J = 5.2 Hz, 1H), 3.94 (br s, 2H), 3.18 (br t, J = 5.2 Hz, 2H), 2.73 (br d, J = 10.4 Hz, 2H), 2.52-2.59 (m, 1H), 1.47-1.84 (m, 4H), 1.40 ppm (s, 9H). ES-API:[M+1]⁺=243.2. For A2-2, ¹H NMR (400 MHz, CDCl₃): δ 4.28-4.00 (m, 2H), 3.54 (d, *J* = 7.6 Hz, 2H), 3.06-3.03 (m, 1H), 2.76-2.43 (m, 4H), 2.08-1.96 (m, 2H), 1.87-1.77 (m, 2H), 1.46 (s, 9H). ES-API:[M+1]⁺=243.2.

### Example 1: Synthesis of 5-ethyl-4-((6R,9S)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)naphthalen-2-ol (Z1)

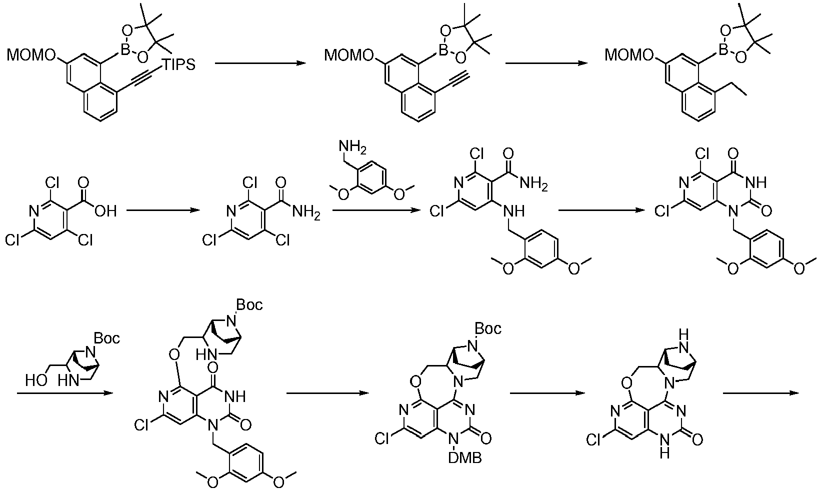

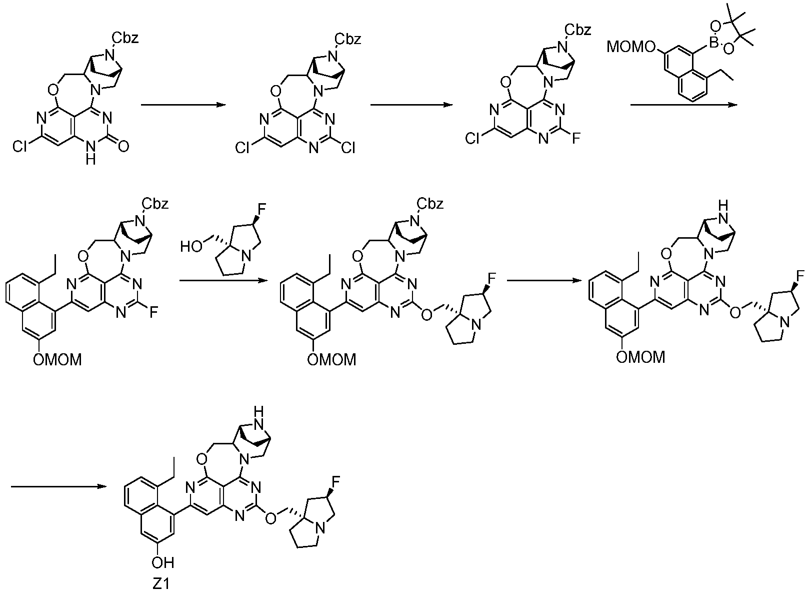

Step 1: Cesium fluoride (3.0 g, 20.24 mmol) was added to a solution of triisopropyl ((6-methoxymethoxy)-8-(4,4,5,5-tetramethyl-1,3,2-dioxaborolan-2-yl)naphthalen-1-yl)eth ynyl)silane (500 mg, 1.012 mmol) in N,N-dimethylformamide (5.0 mL), and the system reacted at room temperature for 2 hours. After completion of the reaction, the reaction mixture was extracted with ethyl acetate (100 mL×2). The ethyl acetate phases were combined, washed with saturated brine (100 mL×3), dried over anhydrous sodium sulfate, filtered, and rotoevaporated to obtain 2-(8-ethynyl-3-(methoxymethoxy)naphthalen-1-yl)-4,4,5,5-tetramethyl-1,3,2-dioxaborina ne (558 mg, crude product). ES-API:[M+H]⁺=339.3.

Step 2: Palladium on carbon with a mass fraction of 10% (60 mg) was added to a solution of 2-(8-ethynyl-3-(methoxymethoxy)naphthalen-1-yl)-4,4,5,5-tetramethyl-1,3,2-dioxaborina ne (558 mg, crude product) in methanol (10.0 mL). The reaction system was replaced with hydrogen for 4 times and reacted in the presence of protective hydrogen at room temperature for 2 hours. After completion of the reaction, the reaction mixture was filtered, and the solvent was rotoevaporated under reduced pressure to obtain 2-(8-ethyl-3-(methoxymethoxy)naphthalen-1-yl)- 4,4,5,5-tetramethyl-1,3,2-dioxaborinane (284 mg, 2-step overall yield: 82%). ES-API:[M+1]⁺=343.3.

Step 3: 2,4,6-Trichloronicotinic acid (5.0 g, 22.22 mmol) was dissolved in dichloromethane (40.0 mL) at 0°C. Oxalyl chloride (6.0mL) was added dropwise to the above-mentioned system and then 8 drops of N,N-dimethylformamide was added. The reaction reaction system was stirred at room temperature for 1 hour. After 1 hour, the solvent was rotoevaporated under reduced pressure, and a solution of dry tetrahydrofuran (30.0 mL) was added. The obtained solution was cooled to 0~5°C and added dropwise to a mixed solution of aqueous ammonia (30.0 mL) and tetrahydrofuran (30.0 mL), stirred at room temperature for 1 hour, and extracted with ethyl acetate (100 mL×2). The ethyl acetate phases were combined, washed with saturated brine (100 mL×1), dried over anhydrous sodium sulfate, filtered, and rotoevaporated to obtain 2,4,6-trichloronicotinamide (6.0 g, crude product). ES-API:[M+H]⁺=224.9/226.9.

Step 4: In a 500 mL three-neck round-bottom flask, (2,4-dimethoxyphenyl)methanamine (4.45 g, 26.66 mmol) was dissolved in dried dioxane (90.0 mL). After cooling to 0~5°C in an ice-water bath, N,N-diisopropylethyl amine (8.61 g, 66.66 mmol) was added in a nitrogen atmosphere, and the system reacted at this temperature for about 10-15 minutes. The 2,4,6-trichloronicotinamide (6.0 g, crude product) was dissolved in dried dioxane (20 mL) and added dropwise to the above-mentioned solution. The reaction was continually carried out at 50°C for 5 hours and monitored by LCMS for completion. The reaction solution was poured into ice water (400 mL), extracted with ethyl acetate (200 mL×1), dried over anhydrous sodium sulfate, filtered, and rotoevaporated. The crude product was purified by column chromatography [petroleum ether:ethyl acetate=100:0~50:50, (v/v)] to obtain 2,6-dichloro-4((2,4-dimethoxybenzyl)amino)nicotinamide (4.16 g, 2-step overall yield: 52%). ES-API:[M+H]⁺= 356.1/358.1.

Step 5: 2,6-dichloro-4-((2,4-dimethoxybenzyl)amino)nicotinamide (3.723 g, 10.47 mmol) and dry tetrahydrofuran (60 mL) were added to a 500 mL three-necked round-bottom flask at room temperature. After cooling to 0~5°C in an ice-water bath, sodium hydride (838 mg, 20.95 mmol) was added in batches, and the system reacted at this temperature for 20 minutes. N,N'-carbonyldiimidazole (5.09 g, 31.41 mmol) was added to dry tetrahydrofuran (30 mL) and added dropwise to the above-mentioned solution. Upon completion of the addition, the system reacted at this temperature for 0.5-1 hour, and the reaction was monitored by LCMS for completion. The reaction solution was poured into ice water (300 mL), adjusted to pH 7-8 with 6M aqueous hydrochloric acid under an ice-water bath condition, and filtered to obtain 5,7-dichloro-1-(2,4-dimethoxybenzyl)pyrido[4,3-d]pyrimidine-2,4(1H,3H)-dione (3.7 g, yield: 92.7%). ES-API:[M+H]⁺=382.0/384.0.

Step 6: Dry tetrahydrofuran (100 mL) was added to a 250 mL single-neck flask at room temperature followed by tert-butyl (1R,5S)-2-(hydroxymethyl)-3,8-diazabicyclo[3.2.1] octane-8-carboxylate (A1, 358.0 mg, 1.480 mmol). After cooling to 0~5°C in an ice-water bath, sodium hydride (1.20 mg, 2.960 mmol) was added, and the system reacted for 10-20 minutes in a nitrogen atmosphere. The 5,7-dichloro-1-(2,4-dimethoxybenzyl)pyrido[4,3-d]pyrimidine- 2,4(1H,3H)-dione (565.0 mg, 1.480 mmol) was added in batches. The reaction was continually carried out at 0~5°C for 20-30 minutes and monitored by LC-MS for completion. The reaction solution was poured into ice water (300 mL), adjusted to pH 7-8 with 6M aqueous hydrochloric acid, and extracted with ethyl acetate (100 mL×2). The ethyl acetate phases were combined, washed with saturated brine (100 mL×1), dried over anhydrous sodium sulfate, filtered, and rotoevaporated to obtain tert-butyl (1R,5S)-2-(((7-chloro-1-(2,4-dimethoxybenzyl)-2,4-dioxa-1,2,3,4-tetrahydropyrido[4,3-d]pyrimidin-5-yl)oxy)methyl)-3,8-diazabicyclo[3.2.1] octane-8-carboxylate (850 mg, yield: 97%). ES-API:[M+H]⁺=588.3.

Step 7: Dry N,N-dimethylformamide (20 mL) and tert-butyl (1R,5S)-2-(((7-chloro-1-(2,4-dimethoxybenzyl)-2,4-dioxa-1,2,3,4-tetrahydropyrido[4,3-d] pyrimidin-5-yl)oxy)methyl)-3,8-diazabicyclo[3.2.1]octane-8-carboxylate (850 mg, 1.448 mmol) were added to a 250 mL single-neck flask at room temperature. 1-Propylphosphonic anhydride (5.0 g, 7.856 mmol) was added, and the system reacted at room temperature for 5 minutes. 1,8-Diazabicyclo[5.4.0]undec-7-ene (1.14 g, 7.488 mmol) was added dropwise, and the system reacted at room temperature for 1-2 hours. After completion of the reaction, the above-mentioned solution was slowly added dropwise to 100 mL of ice water. A large amount of solid was precipitated, filtered, and rotoevaporated under reduced pressure to obtain a target compound, tert-butyl (6R,9S)-2-chloro-13-3,4-dimethoxybenzyl)-12-oxo-5a,6,7,8,9,10,12,13-octahydro-5H-4-o xa-3,10a,11,13,14-pentaaza-6,9-methanonaphtho[1,8-ab]heptene-14-carboxylate (620 mg, yield: 75.2%). ES-API: [M+H]⁺=570.3.

Step 8: tert-butyl (6R,9S)-2-chloro-13-(3,4-dimethoxybenzyl)-12-oxo-5a,6,7,8,9,10,12,13-octahydro-5H-4-oxa-3,10a,11,13,14-pentaaza-6,9-methanonaphtho[1,8-ab]heptene-14-carboxylate (420 mg, 0.7380 mmol) was added to trifluoroacetic acid (5 mL) and stirred at 55°C for 1 hour. After completion of the reaction, the solvent was rotoevaporated under reduced pressure to obtain (6R,9S)-2-chloro-5a,6,7,8,9, 10-hexahydro-5H-4-oxa-3, 10a, 11, 13,14-pentaaza-6,9-methox ynaphtho[1,8-ab]hepten-12(13H)-one (500 mg, crude product). ES-API: [M+H]⁺=320.1. Step 9: Sodium carbonate (350 mg, 3.282 mmol) was added to a mixed solution of (6R,9S)-2-chloro-5a,6,7,8,9, 10-hexahydro-5H-4-oxa-, 10a, 11, 13,14-pentaaza-6,9-methano naphtho[1,8-ab]hepten-12(13H)-one (350 mg, 1.094 mmol) in tetrahydrofuran (5.0 mL) and water (5.0 mL). After cooling in an ice-water bath, carbobenzoxy succinimide (354 mg, 1.422 mmol) was added, and the system reacted at room temperature for 3 hours. After completion of the reaction, the reaction solution was added with dichloromethane (80 mL), washed with saturated aqueous sodium bicarbonate solution (100 mL) and saturated brine (80 mL), dried over anhydrous sodium sulfate, and concentrated. The crude product was purified by a flash silica gel column (ethyl acetate/petroleum ether: 0-50%) to obtain benzyl (6R,9S)-2-chloro- 12-oxa-5a,6,7,8,9,10,12,13-octahydro-5H-4-oxa-3, 10a, 11, 13,14-pentaaz a-6,9-methanonaphtho[1,8-ab]heptene-14-carboxylate (460 mg, yield: 93%). ES-API: [M+H]⁺=454.1.

Step 10: Phosphorus oxychloride (465 mg, 3.039 mmol) was added to a solution of benzyl (6R,9S)-2-chloro-12-oxa-5a,6,7,8,9,10,12,13-octahydro-5H-4-oxa-3,10a,11,13,14-pentaaz a-6,9-methanonaphtho[1,8-ab]heptene-14-carboxylate (460 mg, 1.013 mmol) in toluene (3.0 mL) followed by N,N-diisopropylethyl amine (392.0 mg, 3.039 mmol) at 0°C, and the system reacted at 125°C for 12 h. After completion of the reaction, the reaction mixture was cooled to room temperature, and the solvent was rotoevaporated under reduced pressure to obtain benzyl (6R,9S)-2,12-dichloro-5a,6,7,8,9,10-hexahydro-5H-4-oxa-3,10a,11,13,14-pentaaza-6,9-me thanonaphtho[1,8-ab]heptene-14-carboxylate (520 mg, crude product). ES-API: [M+H]⁺=472.2.

Step 11: Potassium fluoride (500 mg, 8.492 mmol) was added to a solution of benzyl (6R,9S)-2,12-dichloro-5a,6,7,8,9,10-hexahydro-5H-4-oxa-3,10a,11,13,14-pentaaza-6,9-me thanonaphtho[1,8-ab]heptene-14-carboxylate (200 mg, 0.4246 mmol) in N,N-dimethylformamide (3.0 mL), and the system reacted at 120°C in a nitrogen atmosphere for 4 hours. After completion of the reaction, the reaction mixture was cooled to room temperature and extracted with ethyl acetate (50 mL×2). The ethyl acetate phases were combined, washed with saturated brine (50 mL×4), dried over anhydrous sodium sulfate, filtered, and rotoevaporated to obtain benzyl (6R,9S)-2-chloro- 12-fluoro-5a,6,7,8,9, 10-hexahydro-5H-4-oxa-3, 10a, 11, 13,14-pentaaza-6, 9-methanonaphtho[1,8-ab]heptene-14-carboxylate (130 mg, yield: 67.4%). ES-API: [M+H]⁺=456.1.

Step 12: benzyl (6R,9S)-2-chloro-12-fluoro-5a,6,7,8,9,10-hexahydro-5H-4-oxa-3,10a,11,13,14-pentaaza-6, 9-methanonaphtho[1,8-ab]heptene-14-carboxylate (140 mg, 0.3076 mmol) was dissolved in tetrahydrofuran/water (2 mL/0.5 mL). The 2-(8-ethyl-3-(methoxymethoxy)naphthalen-1-yl)-4,4,5,5-tetramethyl-1,3,2-dioxaborinane (250 mg, 0.7309 mmol), potassium phosphate (250 mg, 1.179 mmol) and [n-butyl di(1-adamantyl)phosphino](2-amino-1,1'-biphenyl-2-yl)palladium (II) methanesulfonate (40.0 mg, 0.05494 mmol) were added. The reaction system was replaced with nitrogen for 4 times and reacted under microwave at 80°C for 1 hour. The reaction solution was cooled to room temperature and extracted with ethyl acetate (80 mL) and water (60 mL). The organic phase was dried over anhydrous sodium sulfate and filtered, and the filtrate was rotoevaporated under reduced pressure. The crude product was purified by a flash silica gel column (ethyl acetate/petroleum ether: 0-70%) to obtain a product of benzyl (6R,9S)-2-(8-ethyl-3-(methoxymethoxy)naphthalen-1-yl)-12-fluoro-5a,6,7,8,9,10-hexahyd ro-5H-4-oxa-3,10a,11,13,14-pentaaza-6,9-methanonaphtho[1,8-ab]heptene-14-carboxylate (144 mg, yield: 73%). ES-API:[M/2+1]⁺=636.3.

Step 13: Sodium hydride (22 mg, 0.5428 mmol) was added to a solution of ((2R,7aS)-2-fluorotetrahydro-1H-pyrrolizin-7a(5H)-yl)methanol (43.0 mg, 0.2714 mmol) in tetrahydrofuran (5.0 mL) under an ice-water bath condition, and the system reacted at room temperature for 0.5 hours. The benzyl (6R,9S)-2-(8-ethyl-3-(methoxymethoxy) naphthalen-1-yl)-12-fluoro-5a,6,7,8,9,10-hexahydro-5H-4-oxa-3,10a,11,13,14-pentaaza-6, 9-methano[1,8-ab]heptene-14-carboxylate (145 mg, 0.1357 mmol) was added to the above-mentioned reaction system, and the mixture reacted at room temperature for 1~2 hours. After completion of the reaction, ice water (50 mL) was added to the system, and the obtained mixture was extracted with dichloromethane (50 mL× 2). The dichloromethane phases were combined, dried over anhydrous sodium sulfate, and filtered. The filtrate was rotoevaporated under reduced pressure. The crude product was purified by a flash silica gel column (methanol/dichloromethane: 0-10%) to obtain benzyl (6R,9S)-2-(8-ethyl-3-(methoxymethoxy)naphthalen-1-yl)-12-(((2R,7aS)-2-fluorotetrahydr o-1H-pyrrolizin-7a(5H)-yl)methoxy)-5a,6,7,8,9,10-hexahydro-5H-4-oxa-3,10a,11,13,14-p entaaza-6,9-methanonaphtho[1,8-ab]heptene-14-carboxylate (170 mg, crude product). ES-API:[M/2+1]⁺=775.3.

Step 14: Palladium on carbon with a mass fraction of 10% (500 mg) was added to a solution of (6R,9S)-2-(8-ethyl-3-(methoxymethoxy)naphthalen-1-yl)-12-(((2R,7aS)-2-fluorotetrahydr o-1H-pyrrolizin-7a(5H)-yl)methoxy)-5a,6,7,8,9,10-hexahydro-5H-4-oxa-3,10a,11,13,14-p entaaza-6,9-methanonaphtho[1,8-ab]heptene-14-benzyl carboxylate (170 mg, crude product) in methanol (10.0 mL). The reaction system was replaced with hydrogen for 4 times and reacted at room temperature under a hydrogen atmosphere for 2 hours to obtain (6R,9S)-2-(8-ethyl-3-(methoxymethoxy)naphthalen-1-yl)-12-(((2R,7aS)-2-fluorotetrahydr o-1H-pyrrolizin-7a(5H)-yl)methoxy)-5a,6,7,8,9,10-hexahydro-5H-4-oxa-3,10a,11,13,14-p entaaza-6,9-methanonaphtho[1,8-ab]heptene (162 mg, crude product). ES-API:[M/2+1]⁺=7641.3.

Step 15: (6R,9S)-2-(8-ethyl-3-(methoxymethoxy)naphthalen-1-yl)-12-(((2R,7aS)-2-fluorotetrahydr o-1H-pyrrolizin-7a(5H)-yl)methoxy)-5a,6,7,8,9,10-hexahydro-5H-4-oxa-3,10a,11,13,14-p entaaza-6,9-methanonaphtho[1,8-ab]heptene (162 mg, crude product) was dissolved in acetonitrile (6.0 mL). 4M Hydrochloric acid/dioxane solution (2.0 mL, 8.0 mmol) was added under an ice-water bath condition, and the system reacted in an ice-water bath for 0.5 hours. After completion of the reaction, the solvent was rotoevaporated under reduced pressure. Dichloromethane (20 mL) was added, and triethylamine (3.0 mL) was added under an ice-water bath condition. After stirring for 10 minutes, the obtained mixture was extracted with dichloromethane (100 mL×1) and water (50 mL×1). The organic phase was dried over anhydrous sodium sulfate and filtered. The filtrate was rotoevaporated under reduced pressure. The crude product was purified by preparative HPLC (formic acid method) to obtain 5-ethyl-4-((6R,9S)-12-(((2R,7aS)-2-fluorotetrahydro-1H-pyrrolizin-7a(5H)-yl)methoxy)-5 a,6,7,8,9,10-hexahydro-5H-4-oxa-3,10a,11,13,14-pentaaza-6,9-methanonaphtho[1,8-ab]he ptalen-2-yl)naphthalen-2-ol (Z1, formate, 4.69 mg, 3-step overall yield: 5%). ES-API:[M+1]⁺=597.3. ¹H NMR (500 MHz, CD₃OD) δ 8.48 (s, 1H), 7.58 (d, *J* = 8.0 Hz, 1H), 7.34 (t, *J* = 8.0 Hz, 1H), 7.25-6.91 (m, 4H), 5.50-5.31 (m, 1H), 5.11-4.97 (m, 1H), 4.70-4.30 (m, 4H), 4.20-4.10 (m, 1H), 3.85-3.73 (m, 2H), 3.65-3.40 (m, 3H), 3.29-3.14 (m, 2H), 2.56-1.75 (m, 12H), 1.00-0.85 (m, 3H).

### Example 2: Synthesis of 5-ethyl-4-((5aS,6S,9R)-12-(((2R,7aS)-2-fluorotetrahydro-1H-pyrrolizin-7a(5H)-yl)met hoxy)-5a,6,7,8,9,10-hexahydro-5H-4-oxa-3,10a,11,13,14-pentaaza-6,9-methanonaphth o[1,8-ab]hepten-2-yl)naphthalen-2-ol (Z2)

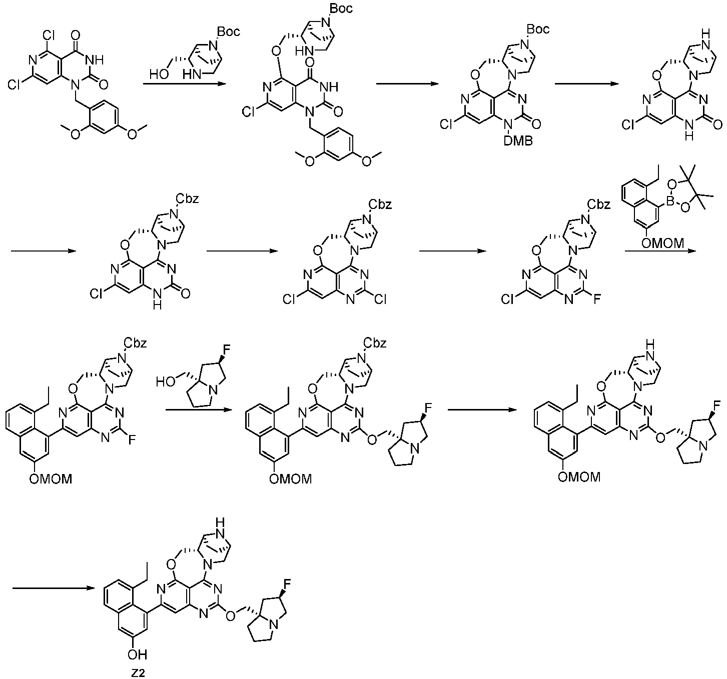

Step 1: Dry tetrahydrofuran (100 mL) was added to a 250 mL single-neck flask at room temperature followed by tert-butyl (1S,2S,5R)-2-(hydroxymethyl)-3,8-diazabicyclo[3.2.1]octane-8-carboxylate (A2-1, 358.0 mg, 1.480 mmol). After cooling to 0~5°C in an ice-water bath, sodium hydride (1,20 mg, 2.960 mmol) was added, and the system reacted for 10-20 minutes in a nitrogen atmosphere. 5,7-Dichloro-1-(2,4-dimethoxybenzyl)pyrido[4,3-d]pyrimidine-2,4(1H,3H)-dione (565.0 mg, 1.480 mmol) was added in batches. The reaction was continually carried out at 50°C for 20~30 minutes and monitored by LC-MS for completion. The reaction solution was poured into ice water (300 mL), adjusted to pH 7~8 with 6M aqueous hydrochloric acid under an ice-water bath condition, and extracted with ethyl acetate (100 mL×2). The ethyl acetate phases were combined, washed with saturated brine (100 mL×1), dried over anhydrous sodium sulfate, filtered, and rotoevaporated to obtain tert-butyl (1S,2S,5R)-2-(((7-chloro-1-(2,4-dimethoxybenzyl)-2,4-dioxa-1,2,3,4-tetrahydropyrido[4,3 -d]pyrimidin-5-yl)oxy)methyl)-3,8-diazabicyclo[3.2.1]octane-8-carboxylate (850 mg, yield: 97%). ES-API:[M+H]⁺=588.3.

Step 2: Dry N,N-dimethylformamide (20 mL) and tert-butyl (1S,2S,5R)-2-(((7-chloro-1-(2,4-dimethoxybenzyl)-2,4-dioxa-1,2,3,4-tetrahydropyrido[4,3-d]pyrimidin-5-yl)oxy)meth yl)-3,8-diazabicyclo[3.2.1]octane-8-carboxylate (850 mg, 1.448 mmol) were added to a 250 mL single-neck flask at room temperature. 1-Propylphosphonic anhydride (5.0 g, 7.856 mmol) was then added, and the system reacted at room temperature for 5 minutes. 1,8-Diazobisspiro[5.4.0]undec-7-ene (1.14 g, 7.488 mmol) was added dropwise, and the system reacted at room temperature for 1~2 hours. After completion of the reaction, the above-mentioned solution was slowly added dropwise to ice water (100 mL). A large amount of solid was precipitated and filtered. The filter cake was rotoevaporated under reduced pressure to obtain tert-butyl (5aS,6S,9R)-2-chloro-13-3,4-dimethoxybenzyl)-12-oxo-5a,6,7,8,9,10,12,13-octahydro-5H-4-oxa-3,10a,11,13,14-pentaaza-6,9-methanonap htho[1,8-ab]heptene-14-carboxylate (620 mg, yield: 73%). ES-API: [M+H]⁺=570.3.

Step 3: tert-butyl (5aS,6S,9R)-2-chloro-13-(2,4-dimethoxybenzyl)-12-oxo-5a,6,7,8,9,10,12,13-octahydro-5H-4-oxa-3,10a,11,13,14-pentaaza-6,9-methanonaphtho[1, 8-ab]heptene-14-carboxylate (420 mg, 0.7380 mmol) was added to trifluoroacetic acid (5.0 mL) and stirred at 55°C for 1 hour. After completion of the reaction, the solvent was rotoevaporated under reduced pressure to obtain (5aS,6S,9R)-2-chloro-5a,6,7,8,9,10-hexahydro-5H-4-oxa-3,10a, 11,13,14-pentaaza-6,9-methanonaphtho[1,8-ab]hepten-12(13H)-one (500 mg, crude product). ES-API: [M+H]⁺=320.1.

Step 4: Sodium carbonate (350 mg, 3.282 mmol) was added to a mixed solution of (5aS,6S,9R)-2-chloro-5a,6,7,8,9,10-hexahydro-5H-4-oxa-3,10a,11,13,14-pentaaza-6,9-met hanonaphtho[1,8-ab]hepten-12(13H)-one (350 mg, 1.094 mmol) in tetrahydrofuran (5.0 mL) and water (5.0 mL). The reaction system was cooled in an ice-water bath. Carbobenzoxy succinimide (354 mg, 1.422 mmol) was added, and the system reacted at room temperature for 3 hours. After completion of the reaction, the reaction solution was added with dichloromethane (80 mL), washed with saturated aqueous sodium bicarbonate solution (100 mL) and saturated brine (80 mL), dried over anhydrous sodium sulfate, and concentrated. The crude product was purified by a flash silica gel column (ethyl acetate/petroleum ether: 0-50%) to obtain benzyl (5aS,6S,9R)-2-chloro-12-oxa-5a,6,7,8,9,10,12,13-octahydro-5H-4-oxa-3, 10a, 11, 13,14-pentaaza-6,9-methanonap htho[1,8-ab]heptene-14-carboxylate (460 mg, yield: 93%). ES-API: [M+H]⁺=454.1.

Step 5: Phosphorus oxychloride (465 mg, 3.039 mmol) and N,N-diisopropylethyl amine (392.0 mg, 3.039 mmol) were added successively to a solution of benzyl (5aS,6S,9R)-2-chloro-12-oxa-5a,6,7,8,9,10,12,13-octahydro-5H-4-oxa-3, 10a, 11, 13,14-pentaaza-6,9-methanonap htho[1,8-ab]heptene-14-carboxylate (460 mg, 1.013 mmol) in toluene (3.0 mL) at 0°C, and the system reacted at 125°C for 12 hours. After completion of the reaction, the reaction mixture was cooled to room temperature, and the solvent was rotoevaporated under reduced pressure to obtain benzyl (5aS,6S,9R)-2,12-dichloro-5a,6,7,8,9, 10-hexahydro-5H-4-oxa-3, 10a, 11, 13,14-pentaaza-6, 9-methanonaphtho[1,8-ab]heptene-14-carboxylate (520 mg, crude product). ES-API: [M+H]⁺=472.2.

Step 6: Potassium fluoride (500 mg, 8.492 mmol) was added to a solution of benzyl (5aS,6S,9R)-2,12-dichloro-5a,6,7,8,9,10-hexahydro-5H-4-oxa-3,10a,11,13,14-pentaaza-6, 9-methanonaphtho[1,8-ab]heptene-14-carboxylate (200 mg, 0.4246 mmol) in N,N-dimethylformamide (3.0 mL), and the system reacted at 120°Cfor 4 hours in a nitrogen atmosphere. After completion of the reaction, the reaction mixture was cooled to room temperature and extracted with ethyl acetate (50 mL×2). The ethyl acetate phases were combined, washed with saturated brine (50 mL×4), dried over anhydrous sodium sulfate, filtered, and rotoevaporated to obtain benzyl (5aS,6S,9R)-2-chloro- 12-fluoro-5a,6,7,8,9, 10-hexahydro-5H-4-oxa-3, 10a, 11, 13,14-pentaa za-6,9-methanonaphtho[1,8-ab]heptene-14-carboxylate (130 mg, yield: 62%). ES-API: [M+H]⁺=456.1.

Step 7: benzyl (5aS,6S,9R)-2-chloro-12-fluoro-5a,6,7,8,9,10-hexahydro-5H-4-oxa-3,10a,11,13,14-pentaaza-6,9-methanonaphtho[1,8-ab]heptene-14-carboxylate (140 mg, 0.3076 mmol) was dissolved in tetrahydrofuran/water (2 mL/0.5 mL). 2-(8-Ethyl-3-(methoxymethoxy)naphthalen-1-yl)-4,4,5,5-tetramethyl-1,3,2-dioxaborolane (250 mg, 0.7309 mmol), potassium phosphate (250 mg, 1.179 mmol) and [n-butyl di(1-adamantyl)phosphino](2-amino-1,1'-biphenyl-2-yl)palladium (II) methanesulfonate (40.0 mg, 0.05494 mmol) were added. The reaction system was replaced with nitrogen 4 times and reacted under microwave at 80°C for 1 hour. The reaction solution was cooled to room temperature and extracted with ethyl acetate (80 mL) and water (60 mL). The organic phase was dried over anhydrous sodium sulfate and filtered. The filtrate was rotoevaporated under reduced pressure. The crude product was purified by a flash silica gel column (ethyl acetate/petroleum ether: 0-70%) to obtain benzyl (SaS,6S,9R)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methanonaphth o[1,8-ab]heptene-14-carboxylate (144 mg, yield: 73%). ES-API:[M/2+1]⁺=636.3.

Step 8: Sodium hydride (22 mg, 0.5428 mmol) was added to a solution of ((2R,7aS)-2-fluorotetrahydro-1H-pyrrolizin-7a(5H)-yl)methanol (43.0 mg, 0.2714 mmol) in tetrahydrofuran (5.0 mL) under an ice-water bath condition, and the system reacted at room temperature for 0.5 hours. After 0.5 hours, the benzyl (5aS,6S,9R)-2-(8-ethyl-3-(methoxymethoxy) naphthalen-1-yl)-12-fluoro-5a,6,7,8,9,10-hexahydro-5H-4-oxa-3,10a,11,13,14-pentaaza-6, 9-methanonaphtho[1,8-ab]heptene-14-carboxylate (145 mg, 0.1357 mmol) was added to the above-mentioned reaction system, and the mixture reacted at room temperature for 1~2 hours. After completion of the reaction, ice water (50 mL) was added to the system, and the obtained mixture was extracted with dichloromethane (50 mL×2). The dichloromethane phases were combined, dried over anhydrous sodium sulfate, and filtered. The filtrate was rotoevaporated under reduced pressure and purified by a flash silica gel column (methanol/dichloromethane: 0-10%) to obtain benzyl (SaS,6S,9R)-2-(8-ethyl-3-(methoxymethoxy)naphthalen-1-yl) -12-(((2R,7aS)-2-fluorotetrahydro-1H-pyrrolizin-7a(5H)-yl)methoxy)-5a,6,7,8,9,10-hexah ydro-5H-4-oxa-3,10a,11,13,14-pentaaza-6,9-methanonaphtho[1,8-ab]heptene-14-carboxyl ate (170 mg, crude product). ES-API:[M/2+1]⁺=775.3.

Step 9: Palladium on carbon with a mass fraction of 10% (500 mg) was added to a solution of benzyl (SaS,6S,9R)-2-(8-ethyl-3-(methoxymethoxy)naphthalen-1-yl)-12-(((2R,7aS)-2-fluorotetrahydro-1H-pyrrolizin-7a(5H)-yl)methoxy)-5a,6,7,8,9,10-hexahydro -5H-4-oxa-3,10a,11,13,14-pentaaza-6,9-methanonaphtho[1,8-ab]heptene-14-carboxylate (170 mg, crude product) in methanol (10.0 mL). The reaction system was replaced with hydrogen for 4 times and reacted at room temperature under a hydrogen atmosphere for 2 hours to obtain (5aS,6S,9R)-2-(8-ethyl-3-(methoxymethoxy)naphthalen-1-yl)-12-(((2R,7aS)-2-fluorotetra hydro- 1H-pyrrolizin-7a(5H)-yl)methoxy)-5a,6,7,8,9, 10-hexahydro-5H-4-oxa-3, 10a, 11, 13, 14-pentaaza-6,9-methanonaphtho[1,8-ab]heptene (162 mg, crude product). ES-API: [M/2+1]⁺=7641.3.

Step 10: (5aS,6S,9R)-2-(8-ethyl-3-(methoxymethoxy)naphthalen-1-yl)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene (162 mg, crude product) was dissolved in acetonitrile (6.0 mL). 4M Hydrochloric acid/dioxane solution (2.0 mL, 8.0 mmol) was added under an ice-water bath condition, and the system reacted in an ice-water bath for 0.5 hours. After completion of the reaction, the solvent was rotoevaporated under reduced pressure. Dichloromethane (20 mL) was added and then triethylamine (3.0 mL) was added under an ice-water bath condition. After stirring for 10 minutes, the obtained mixture was extracted with dichloromethane (100 mL×1) and water (50 mL×1). The dichloromethane phase was dried over anhydrous sodium sulfate and filtered. The filtrate was rotoevaporated under reduced pressure. The crude product was purified by high-performance liquid chromatography (ammonium bicarbonate method) to obtain 5-ethyl-4-((5aS,6S,9R)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaz a-6,9-methanonaphtho[1,8-ab]heptalen-2-yl)naphthalen-2-ol (Z2, 4.65 mg, 3 steps yield: 2.73%). ES-API:[M+1]⁺=597.3. ¹H NMR (500 MHz, CD₃OD) δ 7.57 (d, *J* = 8.2 Hz, 1H), 7.32 (t, *J* = 7.5 Hz, 1H), 7.27-7.09 (m, 3H), 7.06-6.90 (m, 1H), 5.43-5.18 (m, 1H), 5.01 (dd, *J* = 31.4, 13.5 Hz, 1H), 4.58 (d, *J* = 11.5 Hz, 1H), 4.53-4.38 (m, 1H), 4.25 (d, *J* = 10.4 Hz, 1H), 4.19 (d, *J* = 10.4 Hz, 1H), 4.16-4.04 (m, 1H), 3.71 (s, 1H), 3.63 (s, 1H), 3.27-3.17 (m, 4H), 3.01 (td, *J* = 9.6, 5.5 Hz, 1H), 2.55-2.08 (m, 5H), 2.05-1.71 (m, 7H), 0.97 (t, *J* = 7.5 Hz, 1H), 0.90 (t, *J* = 7.4 Hz, 2H).

### Example 3: Synthesis of 4-((6R,9S)-12-(3-(dimethylamino)azetidin-1-yl)-5a,6,7,8,9,10-hexahydro-5H-4-oxa-3,10a,11,13,14-pentaaza-6,9-methanonaphtho[1,8-ab]hepten-2-y l)-5-ethylnaphthalen-2-ol (Z308)

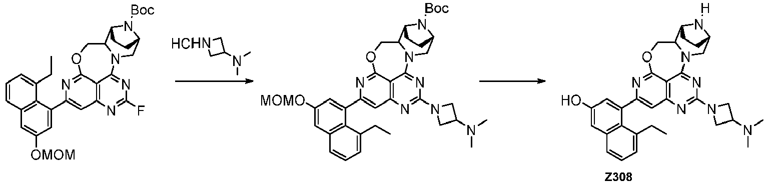

Step 1: N,N-Diisopropylethyl amine (430 mg, 3.322 mmol) was added to a solution of N,N-dimethylazetidin-3-amine hydrochloride (45.0 mg, 0.332 mmol) in dioxane (5.0 mL) followed by tert-butyl (6R,9S)-2-(8-ethyl-3-(methoxymethoxy)naphthalen-1-yl)-12-fluoro-5a,6,7,8,9,10-hexahydro-5H-4-oxa-3,10a,11,13,14-pentaaza-6,9-methoxynaphtho[1,8-ab]h eptene-14-carboxylate (100 mg, 0.1660 mmol) under an ice-water bath condition, and the system reacted at 110°C for 2 hours. After 2 hours, ice water (50 mL) was added to the system, and the obtained mixture was extracted with dichloromethane (50 mL×2). The dichloromethane phases were combined, dried over anhydrous sodium sulfate, and filtered. The filtrate was rotoevaporated under reduced pressure and purified by a flash silica gel column (methanol/dichloromethane: 0-10%) to obtain tert-butyl (6R,9S)-12-(3-(dimethylamino)azetidin-1-yl)-2-(8-ethyl-3-(methoxymethoxy)naphthalen-1-yl)-5a,6,7,8,9,10-hexahydro-5H-4-oxa-3,10a,11,13,14-pe ntaaza-6,9-methanonaphtho[1,8-ab]heptene-14- carboxylate (110 mg, yield: 97%). ES-API:[M+1]⁺=682.3.

Step 2: tert-butyl (6R,9S)-12-(3-(dimethylamino)azetidin-1-yl)-2-(8-ethyl-3-(methoxymethoxy) naphthalen-1-yl)-5a,6,7,8,9,10-hexahydro-5H-4-oxa-3,10a,11,13,14-pentaaza-6,9-methano naphtho[1,8-ab]heptene-14-carboxylate (110 mg, 0.1612 mmol) was dissolved in acetonitrile (6.0 mL). 4M Hydrochloric acid/dioxane solution (2.0 ml, 8.0 mmol) was added under an ice-water bath condition, and the system reacted in an ice-water bath for 0.5 hours. After completion of the reaction, the solvent was rotoevaporated under reduced pressure. Dichloromethane (20 mL) was added and then triethylamine (3.0 mL) was added under an ice-water bath condition. After stirring for 10 minutes, the obtained mixture was extracted once with dichloromethane (100 mL) and water (50 mL). The dichloromethane phase was dried over anhydrous sodium sulfate and filtered. The filtrate was rotoevaporated under reduced pressure to obtain a crude product. The crude product was purified by high-performance liquid chromatography (ammonium bicarbonate method) to obtain 4-((6R,9S)-12-(3-(dimethylamino)azetidin-1-yl)-5a,6,7,8,9,10-hexahydro-5H-4-oxa-3, 10a, 11, 13,14-pentaaza-6,9-methanonaphtho[1,8-ab]heptalen-2-yl)-5-ethylnap hthalen-2-ol (Z308, 14.92 mg, yield: 16.8%). ES-API:[M+1]⁺=538.3.

### Example 4: Synthesis of 5-ethyl-4-((SaR,6S,9R)-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaz a-6,9-methanonaphtho[1,8-ab]hepten-2-yl)naphthalen-2-ol (Z170)

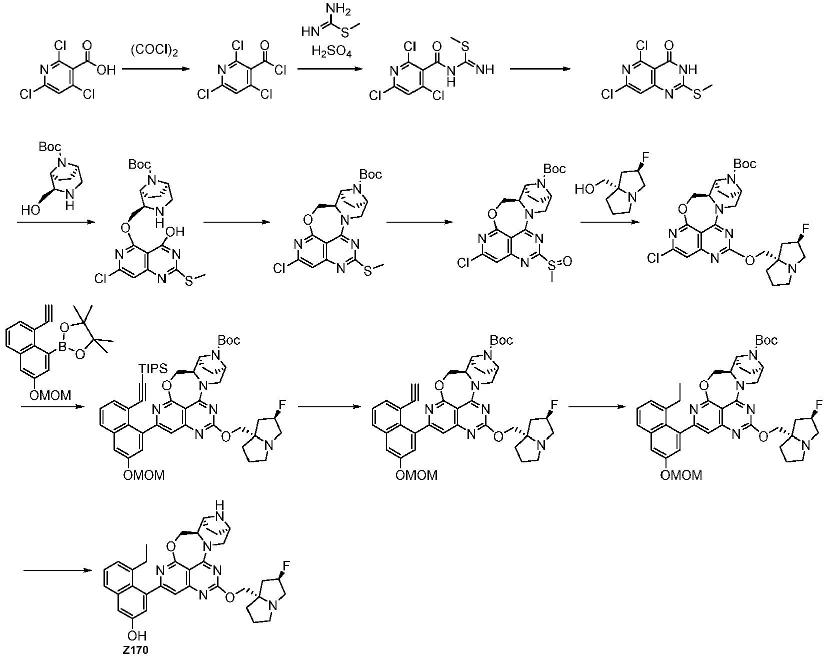

Step 1: 2-Chloro-2-oxoacetyl chloride (0.374 mL, 4.415 mmol) was slowly added to a mixture of 2,4,6-trichloropyridine-3-carboxylic acid (200 mg, 0.883 mmol) and N,N-dimethylformamide (0.02 mL) in dichloromethane (5 mL) at 0°C. The reaction mixture was stirred at room temperature for 1 hour and concentrated to obtain a yellow oily crude product 2,4,6-trichloropyridine-3-carbonyl chloride (216 mg, 0.882 mmol, yield: 99.87%), which was directly used for the next step.

Step 2: Methyl carbamimidothioate (6456.50 mg, 34.301 mmol) and sodium bicarbonate (7204.09 mg, 85.753 mmol) were stirred in tetrahydrofuran (40 mL) and water (70 mL) at room temperature for 10 minutes. The reaction system was cooled to 0°C, and a solution of 2,4,6-trichloropyridine-3-carbonyl chloride (2100 mg, 8.575 mmol) in tetrahydrofuran (40 mL) was added dropwise and stirred at 0°C for 30 minutes. The obtained mixture was then extracted with ethyl acetate. The organic layer was washed with brine, dried over anhydrous sodium sulfate, filtered, and concentrated to obtain methyl (2,4,6-trichloronicotinoyl)carbamimidothioate which was yellow solid (2500 mg, 8.373 mmol, yield: 97.64%). ES-API:[M+1]⁺=297.3.

Step 3: methyl (2,4,6-trichloronicotinoyl)carbamimidothioate (2000 mg, 6.698 mmol) and N,N-diisopropylethyl amine (2.775 mL, 16.746 mmol) were sealed in dioxane (25 mL) and heated at 100°C for 16 hours in a nitrogen atmosphere. The reaction solution was concentrated until the volume was ~5 mL. Water (15 mL) and 2M aqueous hydrochloric acid (3 mL) were added to the residue. After a precipitate formed, the mixture was filtered. The filter cake was washed with water (10 mL) and dried under reduced pressure to obtain 5,7-dichloro-2-(methylthio)pyrido[4,3-d]pyrimidin-4(3H)-one (1300 mg, crude product) which was yellow solid. ES-API:[M+H]⁺=262.0.

Step 4: Tert-butyl (1S,2R,5R)-2-(hydroxymethyl)-3,8-diazabicyclo[3.2.1]octane-8-carboxylate (A2-2, 813.53 mg, 3.357 mmol) was added to a suspension of sodium hydride (427.29 mg, 10.682 mmol) in tetrahydrofuran (20 mL) at 0°C and stirred at this temperature for 10 minutes. The 5,7-dichloro-2-(methylthio)pyrido[4,3-d]pyrimidin-4(3H)-one (800 mg, crude product) was added and stirred with heating at 60°C for 1 hour. After completion of the reaction, the reaction was quenched with water and extracted with dichloromethane/methanol (10:1, 25 mL×3). The organic layer was dried over anhydrous sodium sulfate, filtered, and concentrated to obtain tert-butyl (1S,2R,5R)-2-(((7-chloro-4-hydroxy-2-(methylthio)pyrido[4,3-d]pyrimidin-5-yl]oxy)meth yl)-3,8-diazabicyclo[3.2.1]octane-8-carboxylate (1400 mg, 2.992 mmol, yield: 98.02%) which was yellow solid. ES-API:[M+H]⁺=468.1.

Step 5: Propylphosphonic anhydride (50% in ethyl acetate) (11422.33 mg, 17.949 mmol) was added to a solution of tert-butyl (1S,2R,5R)-2-(((7-chloro-4-hydroxy-2-(methylthio)pyrido [4,3-d]pyrimidin-5-yl]oxy)methyl)-3,8-diazabicyclo[3.2.1]octane-8-carboxylate (1400 mg, 2.992 mmol) and N,N-diisopropylethyl amine (4.957 mL, 29.916 mmol) in dichloromethane (20 mL) and stirred at room temperature for 3 hours. The reaction was quenched with water and extracted with dichloromethane. The organic layer was dried over anhydrous sodium sulfate, filtered, and concentrated. The resulting solid was suspended in acetonitrile and stirred at room temperature for 10 minutes. The obtained mixture was filtered, and the filter cake was collected to obtain tert-butyl (5aR,6S,9R)-2-chloro-12-(methylthio)5a,6,7,8,9,10-hexahydro-5H-4-oxa-3,10a,11,13,14-p entaaza-6,9-methanonaphtho[1,8-ab]heptene-14-carboxylate (600 mg, 1.333 mmol, yield: 44.57%) which was white solid. ES-API:[M+H]⁺=450.1.

Step 6: m-Chloroperoxybenzoic acid (322.61 mg, 1.589 mmol) was added to a solution of tert-butyl (5aR,6S,9R)-2-chloro-12-(methylthio)5a,6,7,8,9,10-hexahydro-5H-4-oxa-3,10a,11,13,14-pentaaza-6,9-methanonaphtho[1,8-ab]heptene-14-carboxylate (550 mg, 1.222 mmol) in dichloromethane (15 mL) at 0°C and stirred at room temperature for 1 hour. The reaction was quenched with saturated sodium sulfite and extracted with dichloromethane. The organic layer was dried over anhydrous sodium sulfate and concentrated to obtain a crude. The crude product was purified by a flash silica gel column (0-10% methanol/dichloromethane) to obtain tert-butyl (5aR,6S,9R)-2-chloro-12-(methylsulfinyl)-5a,6,7,8,9,10-hexahydro-5H-4-oxa-3,10a,11,13,14-pentaaza-6,9-methanonaphtho[1,8-ab]heptene-14-carboxylate (470 mg, 1.009 mmol, yield: 82.52%) which was white solid. ES-API:[M+H]⁺=466.1.

Step 7: ((2R,7aS)-2-fluorotetrahydro-1H-pyrrolizin-7a(5H)-yl)methanol (239.16 mg, 1.502 mmol) was added to a solution of sodium hydride (45.07 mg, 1.878 mmol) in tetrahydrofuran at 0°C and stirred at room temperature for 10 minutes. The reaction system was cooled to 0°C. A solution of tert-butyl (5aR,6S,9R)-2-chloro-12-(methylsulfinyl)-5a,6,7,8,9,10-hexahydro-5H-4-oxa-3,10a,11,13,14-pentaaza-6,9-methanonaphtho[1,8-ab]heptene-14-carboxylate (350 mg, 0.751 mmol) in tetrahydrofuran (5 mL) was added and stirred at 0°C for 1 hour. The reaction was quenched with saturated sodium bicarbonate solution and extracted with ethyl acetate. The organic phase was washed with saturated brine, dried over anhydrous sodium sulfate, filtered, and concentrated. The crude product was purified by a flash silica gel column (0-5% methanol/dichloromethane) to obtain tert-butyl (5aR,6S,9R)-2-chloro-12-(((2R,7aS)-2-fluorotetrahydro- 1H-pyrrolizine-7a(5H)-5a,6,7,8,9, 10-hexahydro-5H-4-oxa-3, 10a, 11, 13, 14-pentaaza-6,9-methanonaphtho[1,8-ab]heptene-14-carboxylate (130 mg, 0.232 mmol, yield: 30.85%) which was yellow solid. ES-API: [M+H]⁺=561.2.

Step 8: Potassium phosphate (147.55 mg, 0.695mmol) and [n-butyl di(1-adamantyl)phosphine](2-amino-1,1'-biphenyl-2-yl)palladium (II) methanesulfonate (1.95mg, 0.003mmol) were added to a mixture of tert-butyl (5aR,6S,9R)-2-chloro-12-(((2R,7aS)-2-fluorotetrahydro- 1H-pyrrolizine-7a(5H)-5a,6,7,8,9, 10-hexahydro-5H-4-oxa-3, 10a, 11, 13,1 4-pentaaza-6,9-methanonaphtho[1,8-ab]heptene-14-carboxylate and triisopropyl ((6-(methoxymethoxy)-8-(4,4,5,5-tetramethyl-1,3,2-dioxyborane-2-yl)naphthalen-1-yl)eth ynyl)silane in dioxane (6 mL) and water (1.5 mL). The reaction system was sparged with nitrogen and reacted under microwave at 80°C for 40 minutes. After completion of the reaction, the reaction was quenched with water and extracted with ethyl acetate (30 mL×2) and saturated brine (30 mL). The ethyl acetate phases were combined, dried over anhydrous sodium sulfate, and filtered. The filtrate was concentrated under reduced pressure. The crude product was purified by a flash silica gel column (0-4% methanol/dichloromethane) to obtain tert-butyl (5aR,6S,9R)-12-(((2R,7aS)-2-fluorotetrahydro-1H-pyrrolizin-7a(5H)-yl)methoxy)-2-(3-(methoxymethoxy)-8-((triisopro pylsilyl)ethynyl)naphthalen- 1-yl)-5a,6,7,8,9, 10-hexahydro-5H-4-oxa-3, 10a, 11, 13,14-penta aza-6,9-methanonaphtho[1,8-ab]heptene-14-carboxylate (160 mg, 0.179 mmol, yield: 77.31%) which was yellow solid. ES-API: [M+H]⁺=893.5.

Step 9: Cesium fluoride (127.55 mg, 0.840 mmol) was added to a solution of tert-butyl (SaR,6S,9R)-12-(((2R,7aS)-2-fluorotetrahydro-1H-pyrrolizin-7a(SH)-yl)methoxy)-2-(3-(m ethoxymethoxy)-8-((triisopropylsilyl)ethynyl)naphthalen-1-yl)-5a,6,7,8,9,10-hexahydro-5 H-4-oxa-3,10a,11,13,14-pentaaza-6,9-methanonaphtho[1,8-ab]heptene-14-carboxylate (150 mg, 0.168 mmol) in N,N-dimethylformamide (3.0 mL) and stirred at room temperature for 0.5 hours. After completion of the reaction, the reaction was quenched with water and extracted with ethyl acetate. The organic phase was washed with saturated brine, dried over anhydrous sodium sulfate, filtered, and concentrated to obtain tert-butyl (SaR,6S,9R)-2-(8-ethynyl-3-(methoxymethoxy)naphthalen-1-yl)-12-(((2R,7aS)-2-fluorotetrahydro-1H-pyrrolizin-7a(5H)-yl)methoxy)-5a,6,7,8,9,10-hexahy dro-5H-4-oxa-3,10a,11,13,14-pentaaza-6,9-methanonaphtho[1,8-ab]heptene-14-carboxylat e (120 mg, 0.163 mmol, yield: 96.97%) which was a yellow oil. ES-API: [M+H]⁺=737.3.

Step 10: Palladium on carbon (74.17 mg, 0.061 mmol) was added to a solution of tert-butyl (SaR,6S,9R)-2-(8-ethynyl-3-(methoxymethoxy)naphthalen-1-yl)-12-(((2R,7aS)-2-fluorotet rahydro- 1H-pyrrolizin-7a(5H)-yl)methoxy)-5a,6,7,8,9, 10-hexahydro-5H-4-oxa-3, 10a, 11, 1 3,14-pentaaza-6,9-methanonaphtho[1,8-ab]heptene-14-carboxylate (90 mg, 0.122 mmol) in methanol (5.0 mL). The reaction system was replaced with hydrogen and reacted at room temperature under a hydrogen atmosphere for 1 hour. The reaction mixture was filtered and concentrated. The crude product was purified by a flash silica gel column (0-3% methanol/dichloromethane) to obtain tert-butyl (5aR,6S,9R)-2-(8-ethyl-3-(methoxymethoxy)naphthalen-1-yl)-12-(((2R,7aS)-2-fluorotetra hydro-1H-pyrrolizin-7a(5H)-yl)methoxy)-5a,6,7,8,9,10-hexahydro-5H-4-oxa-3,10a,11,13, 14-pentaaza-6,9-methanonaphtho[1,8-ab]heptene-14-carboxylate (30 mg, 0.040 mmol, yield: 33.15%) which was a yellow solid. ES-API: [M+H]⁺=741.4.

Step 11: tert-butyl (SaR,6S,9R)-2-(8-ethyl-3-(methoxymethoxy)naphthalen-1-yl) -12-(((2R,7aS)-2-fluorotetrahydro-1H-pyrrolizin-7a(5H)-yl)methoxy)-5a,6,7,8,9,10-hexah ydro-5H-4-oxa-3,10a,11,13,14-pentaaza-6,9-methanonaphtho[1,8-ab]heptene-14-carboxyl ate (30 mg, 0.040 mmol) reacted with acetonitrile (2.0 mL) and 4M hydrochloric acid/dioxane solution (0.500 mL, 2.000 mmol) at 0°C for 1 hour. The reaction mixture was concentrated. The crude product was purified by high-performance liquid chromatography (ammonium bicarbonate method) to obtain 5-ethyl-4-((5aR,6S,9R)-12-(((2R,7aS)-2-fluorotetrahydro-lH-pyrrolizin-7a(5H)-yl) methoxy)-5a,6,7,8,9, 10-hexahydro-5H-4-oxa-3, 10a, 11, 13,14-pentaaza-6,9-methanonaphth o[1,8-ab]heptalen-2-yl)naphthalen-2-ol (Z170, 10.7 mg, yield: 45%) which was white solid. ES-API:[M+1]⁺=597.3. ¹HNMR(500MHz, CD₃OD): 7.57 (d, J=8.0 Hz, 1H), 7.35-7.31(m, 1 H), 7.20-7.13(m, 2 H), 7.05-6.92 (m, 1 H), 6.86-6.85(m, 1 H), 5.33-5.22 (m, 1 H), 4.80-4.77(m, 1 H), 4.57-4.46(m, 2 H), 4.28-4.20(m, 2 H), 3.88-3.84(m, 1 H), 3.78-3.77(m, 1 H), 3.74-3.67(m, 1 H), 3.51-3.44(m, 1 H), 3.30-3.18(m, 3 H), 3.02-2.97(m, 1 H), 2.52-2.01(m, 5 H), 1.98-1.84(m, 7 H), 0.95 (t, J=7.5 Hz, 3H).

### Example 5: synthesis of 5-ethyl-4-((5aS,6S,9R)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]hepten-2-yl)naphthalen-2-ol (Z140-1)

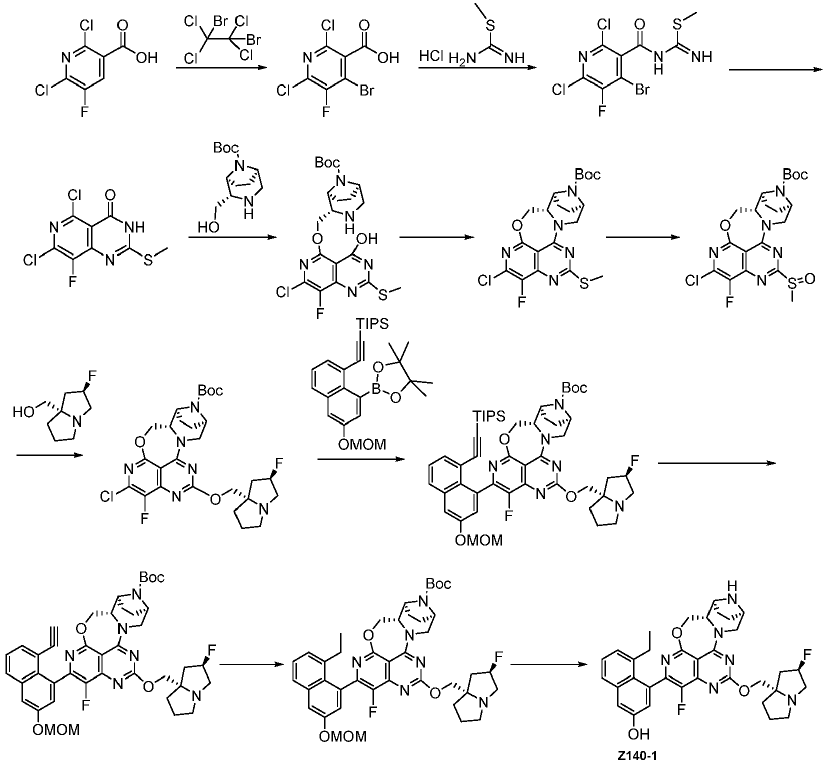

Step 1: Methyllithium (47.6 mL, 76.19 mmol, 1.6M) was added dropwise to a solution of 2,6-dichloro-5-fluoronicotinic acid (8.0 g, 38.095 mmol) in dry tetrahydrofuran (150 mL) at -78°C and then stirred at -20°C for 2 hours. After 2 hours, the reaction system was again cooled to -78°C, and a solution of 1,2-dibromo-1,1,2,2-tetrachloroethane (12.405 g, 38.095 mmol) in tetrahydrofuran (30 mL) was added dropwise over 30 minutes. Upon completion of the addition, the system reacted at 0°C for 1.5 hours. After completion of the reaction, the reaction solution was poured into ice water (500 mL) and washed with chloroform (100 mL×1). The aqueous phase was adjusted to about pH=3 with hydrochloric acid (3M), and rotoevaporated under reduced pressure. The crude product was purified by column chromatography [DCM:MeOH=100:0~90:10, (v/v)] to obtain 4-bromo-2,6-dichloro-5-fluoronicotinic acid (7.6 g, yield: 88%). ES-API:[M+H]⁺=287.9.

Step 2: 4-bromo-2,6-dichloro-5-fluoronicotinic acid (6.0 g, 20.90 mmol) was dissolved in thionyl chloride (40.0 mL) at room temperature, and 16 drops of N,N-dimethylformamide was added to the above-mentioned system. The reaction reaction system was stirred at 100°C for 1 hour. After 1 hour, the solvent was rotoevaporated under reduced pressure. The crude product was added to a mixed solvent of tetrahydrofuran (100 mL) and saturated sodium bicarbonate (100 mL) at 0°C, and finally methyl carbamimidothioate (12.91 g, 68.607 mmol) was added, The mixture was stirred at this temperature for 30 min and then extracted with ethyl acetate (200 mL). The organic layer was washed with brine, dried over anhydrous sodium sulfate, filtered, and concentrated to obtain methyl (4-bromo-2,6-dichloro-5-fluoronicotinoyl)carbamimidothioate (2.23 g, yield: 29.8%). ES-API:[M+1]⁺=359.5.

Step 3: methyl (4-bromo-2,6-dichloro-5-fluoronicotinoyl)carbamimidothioate (880 mg, 2.45 mmol) and N,N-diisopropylethyl amine (1.4 mL, 8.37 mmol) were sealed in dioxane (12 mL) and heated at 100°C for 16 hours in a nitrogen atmosphere. The reaction solution was concentrated until the volume was ~5 mL. Water (15 mL) and 2M aqueous hydrochloric acid (3 mL) were added to the residue. After a precipitate formed, the mixture was filtered. The filter cake was washed with water (10 mL) and dried under reduced pressure to obtain 5,7-dichloro-8-fluoro-2-(methylthio)pyrido[4,3-d]pyrimidin-4(3H)-one (350 mg, yield: 51%) which was yellow solid. ES-API:[M+H]⁺=280.0.

Step 4: Tert-butyl (1S,2S,5R)-2-(hydroxymethyl)-3,8-diazabicyclo[3.2.1]octane-8-carboxylate (A2-1, 813.53 mg, 3.357 mmol) was added to a suspension of sodium hydride (427.29 mg, 10.682 mmol) in tetrahydrofuran (20 mL) at 0°C and stirred at this temperature for 10 minutes. The 5,7-dichloro-8-fluoro-2-(methylthio)pyrido[4,3-d]pyrimidin-4(3H)-one (854 mg, 3.052 mmol) was added and stirred with heating at 60°C for 1 h. After completion of the reaction, the reaction was quenched with water and extracted with dichloromethane/methanol (10:1, 25 mL×3). The organic layer was dried over anhydrous sodium sulfate, filtered, and concentrated to obtain tert-butyl (1S,2S,5R)-2-(((7-chloro-8-fluoro-4-hydroxy-2-(methylthio)pyrido[4,3-d]pyrimidin-5-yl)oxy)methyl)-3,8-diazabicyclo[3.2.1]octane-8-carboxylate (1451 mg, 2.992 mmol, yield: 98.02%) which was yellow solid. ES-API:[M+H]⁺=486.1.

Step 5: Propylphosphonic anhydride (50% in ethyl acetate) (11422.33 mg, 17.949 mmol) was added to a solution of tert-butyl (1S,2S,5R)-2-(((7-chloro-8-fluoro-4-hydroxy-2-(methylthio)pyrido[4,3-d]pyrimidin-5-yl)oxy)methyl)-3,8-diazabicyclo[3.2.1]octane-8-c arboxylate (1451 mg, 2.992 mmol) and N,N-diisopropylethyl amine (4.957 mL, 29.916 mmol) in dichloromethane (20 mL) and stirred at room temperature for 3 hours. The reaction was quenched with water and extracted with dichloromethane. The organic layer was dried over anhydrous sodium sulfate, filtered, and concentrated. The resulting solid was suspended in acetonitrile and stirred at room temperature for 10 minutes. The obtained mixture was filtered, and the filter cake was collected to obtain tert-butyl (SaS,6S,9R)-2-chloro-1-fluoro-12-(methylthio)-5a,6,7,8,9,10-hexahydro-5H-4-oxa-3,10a,11,13,14-pentaaza-6,9-methylenaphtho[1,8-ab]h eptene-14-carboxylate (623 mg, yield: 45.0%) which was white solid. ES-API:[M+H]⁺=468.1.

Step 6: m-Chloroperoxybenzoic acid (322.61 mg, 1.589 mmol) was added to a solution of tert-butyl (5aS,6S,9R)-2-chloro-1-fluoro-12-(methylthio)-5a,6,7,8,9,10-hexahydro-5H-4-oxa -3,10a,11,13,14-pentaaza-6,9-methanonaphtho[1,8-ab]heptene-14-carboxylate (540 mg, 1.153 mmol) in dichloromethane (15 mL) at 0°C and stirred at room temperature for 1 hour. The reaction was quenched with saturated sodium sulfite and extracted with dichloromethane (100 mL×2). The organic layer was dried over anhydrous sodium sulfate and concentrated to obtain a crude product. The crude product was purified by a flash silica gel column (0-10% methanol/dichloromethane) to obtain tert-butyl (5aS,6S,9R)-2-chloro-1-fluoro-12-(methylsulfinyl)-5a,6,7,8,9,10-hexahydro-5H-4-oxa-3,1 0a,11,13,14-pentaaza-6,9-methylenaphtho[1,8-ab]heptene-14-carboxylate (452 mg, yield: 81.0%) which was white solid. ES-API:[M+H]⁺=484.1.

Step 7: ((2R,7aS)-2-fluorotetrahydro-1H-pyrrolizin-7a(5H)-yl)methanol (239.16 mg, 1.502 mmol) was added to a solution of sodium hydride (45.07 mg, 1.878 mmol) in tetrahydrofuran at 0°C and stirred at room temperature for 10 minutes. The reaction system was cooled to 0°C, and a solution of tert-butyl (5aS,6S,9R)-2-chloro-1-fluoro-12-(methylsulfinyl)-5a,6,7,8,9,10-hexahydro-5H-4-oxa-3,10a,11,13,14-pentaaza-6,9-methanonaphtho[1,8-ab]heptene-14-carboxylate (363.5 mg, 0.751 mmol) in tetrahydrofuran (5 mL) was added and stirred at 0°C for 1 hour. The reaction was quenched with saturated sodium bicarbonate solution and extracted with ethyl acetate. The organic phase was washed with saturated brine, dried over anhydrous sodium sulfate, filtered, and concentrated. The crude product was purified by flash column chromatography (0-5% methanol/dichloromethane) to obtain tert-butyl (5aR,6S,9R)-2-chloro-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methylenaphtho[1,8-ab]heptene-14-carboxylate (134 mg, yield: 30.85%) which was yellow solid. ES-API: [M+H]⁺=579.2.

Step 8: Potassium phosphate (147.55 mg, 0.695 mmol) and [n-butyl di(1-adamantyl)phosphine](2-amino-1,1'-biphenyl-2-yl)palladium (II) methanesulfonate (1.95 mg, 0.003 mmol) were added to a mixture of tert-butyl (5aR,6S,9R)-2-chloro-1-fluoro-12-(((2R,7aS)-2-fluorotetrahydro-1H-pyrrolizin-7a(5H)-yl)methoxy)-5a,6,7,8,9,10-hexahydro -5H-4-oxa-3, 10a, 11, 13,14-pentaaza-6,9-methanonaphtho[1,8-ab]heptene- 14-carboxylate (134.0 mg, 0.233 mmol) and triisopropyl ((6-(methoxymethoxy)-8-(4,4,5,5-tetramethyl-1,3,2-dioxaborolan-2-yl) naphthalen-1-yl)ethynyl)silane (230.0 mg, 0.466 mmol) in dioxane (6 mL) and water (1.5 mL). The reaction system was sparged with N₂ and reacted under microwave at 80°C for 40 minutes. After completion of the reaction, the reaction mixture was extracted with ethyl acetate (30 mL×2) and saturated brine (30 mL). The ethyl acetate phases were combined, dried over anhydrous sodium sulfate, and filtered. The filtrate was concentrated under reduced pressure. The crude product was purified by a flash silica gel column (0-4% methanol/dichloromethane) to obtain tert-butyl (5aS,6S,9R)-1-fluoro-12-(((2R,7aS)-2-fluorotetrahydro-1H-pyrrolizin-7a(5H)-yl)methoxy )-2-(3-(methoxymethoxy)-8-((triisopropylsilyl)ethynyl)naphthalen-1-yl)-5a,6,7,8,9,10-hex ahydro-5H-4-oxa-3,10a,11,13,14-pentaaza-6,9-methanonaphtho[1,8-ab]heptene-14-carbox ylate (164 mg, yield: 77.33%) as a yellow solid. ES-API: [M+H]⁺=911.5.

Step 9: Cesium fluoride (127.55 mg, 0.840 mmol) was added to a solution of tert-butyl (5aS,6S,9R)-1-fluoro-12-(((2R,7aS)-2-fluorotetrahydro-1H-pyrrolizin-7a(5H)-yl)methoxy )-2-(3-(methoxymethoxy)-8-((triisopropylsilyl)ethynyl)naphthalen-1-yl)-5a,6,7,8,9,10-hex ahydro-5H-4-oxa-3,10a,11,13,14-pentaaza-6,9-methanonaphtho[1,8-ab]heptene-14-carbox ylate (165 mg, 0.181 mmol) in N,N-dimethylformamide (3.0 mL) and stirred at room temperature for 0.5 hours. After completion of the reaction, the reaction was quenched with water and extracted with ethyl acetate. The organic phase was washed with saturated brine, dried over anhydrous sodium sulfate, filtered, and concentrated to obtain tert-butyl (5aS,6S,9R)-2-(8-ethynyl-3-(methoxymethoxy) naphthalen-1-yl)-1-fluoro-12-(((2R,7aS)-2-fluorotetrahydro-1H-pyrrolizin-7a(5H)-yl)meth oxy)-5a,6,7,8,9,10-hexahydro-5H-4-oxa-3,10a,11,13,14-pentaaza-6,9-methanonaphtho[1,8 -ab]heptene-14-carboxylate (150 mg, crude product) as a yellow oil. ES-API: [M+H]⁺=755.3.

Step 10: Palladium on carbon (75 mg, 0.061 mmol) was added to a solution of tert-butyl (SaS,6S,9R)-2-(8-ethynyl-3-(methoxymethoxy)naphthalen-1-yl)-1-fluoro-12-(((2R,7aS)-2 -fluorotetrahydro-1H-pyrrolizin-7a(5H)-yl)methoxy)-5a,6,7,8,9,10-hexahydro-5H-4-oxa-3 ,10a,11,13,14-pentaaza-6,9-methanonaphtho[1,8-ab]heptene-14-carboxylate (100 mg, 0.1324 mmol) in methanol (5.0 mL). The reaction system was replaced with hydrogen and reacted at room temperature under a hydrogen atmosphere for 1 hour. The reaction mixture was filtered and concentrated. The crude was purified by a flash silica gel column (0-3% methanol/dichloromethane) to obtain tert-butyl (5aS,6S,9R)-2-(8-ethyl-3-(methoxymethoxy)naphthalen-1-yl)-1-fluoro-12-(((2R,7aS)-2-fl uorotetrahydro-1H-pyrrolizin-7a(5H)-yl)methoxy)-5a,6,7,8,9,10-hexahydro-5H-4-oxa-3,1 0a,11,13,14-pentaaza-6,9-methanonaphtho[1,8-ab]heptene-14-carboxylate (70 mg, yield: 70%) as a yellow solid. ES-API: [M+H]⁺=759.4.

Step 11: tert-butyl (SaS,6S,9R)-2-(8-ethyl-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptene-14-car boxylate (80 mg, 0.105 mmol) reacted with acetonitrile (2.0 mL) and 4M hydrochloric acid/dioxane solution (0.500 mL, 2.000 mmol) at 0°C for 1 hour. The reaction mixture was concentrated. The crude product was purified by high-performance liquid chromatography (ammonium bicarbonate method) to obtain 5-ethyl-4-((SaS,6S,9R)-1-fluoro-12-(((2R,7aS)-2-fluorotetrahydro-1H-pyrrolizin-7a(5H)-yl)methoxy)-5a,6,7,8,9, 10-hexahydro-5H-4-oxa-3, 10a, 11, 13,14-pentaaz a-6,9-methanonaphtho[1,8-ab]heptalen-2-yl)naphthalen-2-ol (Z140-1, 9.65 mg, yield: 14.9%) as a yellow solid. ES-API:[M+1]⁺=615.3.

### Example 6: Synthesis of (5aS,6S,9R)-2-(8-ethynaphthalen-1-yl)-1-fluoro-12-(((2R,7aS)-2-fluorotetrahydro-1H-pyrrolizin-7a(5H)-yl)methoxy)-5a,6,7,8,9,10-hexah ydro-5H-4-oxo-3,10a,11,13,14-pentaaza-6,9-methanonaphtho[1,8-ab]heptene (Z350)

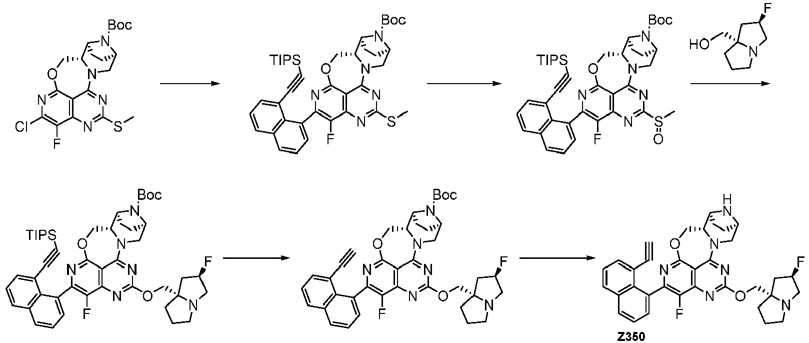

Step 1: Potassium phosphate (300 mg, 1.415 mmol) and [n-butyl di(1-adamantyl)phosphine](2-amino-1,1'-biphenyl-2-yl)palladium (II) methanesulfonate (100 mg, 0.137 mmol) were added to a solution of tert-butyl (1S,2S,5R)-2-(((7-chloro-8-fluoro-(2-methylthio)-4-oxa-3,4-dihydropyrido[4,3-d]pyrimidin-5-yl)oxy)methyl)-3,8-diazabicyc lo[3.2.1]octane-8-carboxylate (150.0 mg, 0.321 mmol) and triisopropyl ((8-(4,4,5,5-tetramethyl- 1,3,2-dioxaborolan-2-yl)naphthalen-1-yl)ethynyl)silane (220.0 mg, 0.506 mmol) in dioxane (6 mL) and water (1.5 mL). The reaction system was sparged with nitrogen and reacted under microwave at 80°C for 40 minutes. After completion of the reaction, the reaction mixture was extracted with ethyl acetate (30 mL×2) and saturated brine (30 mL). The ethyl acetate phases were combined, dried over anhydrous sodium sulfate, and filtered. The filtrate was concentrated under reduced pressure. The crude product was purified by column chromatography (methanol/dichloromethane=0-4%) to obtain tert-butyl (5aS,6S,9R)-1-fluoro-12-(methylthio)-2-(8-((triisopropylsilyl)ethynyl)naphthalen-1-yl)-5a,6,7,8,9,10-hexahydro-5H-4-oxo-3,10a,1 1,13,14-pentaaza-6,9-methanonaphtho[1,8-ab]heptene-14-carboxylate (272 mg, crude product) as a yellow solid. ES-API: [M+H]⁺=800.4.

Step 2: m-Chloroperoxybenzoic acid (67.12 mg, 0.389 mmol) was added to a solution of tert-butyl (SaS,6S,9R)-1-fluoro-12-(methylthio)-2-(8-((triisopropylsilyl)ethynyl)naphthalen-1-yl)-Sa ,6,7,8,9,10-hexahydro-5H-4-oxo-3,10a,11,13,14-pentaaza-6,9-methanonaphtho[1,8-ab]hep tene-14-carboxylate (272 mg, crude product) in dichloromethane (15 mL) at 0°C and stirred at room temperature for 1 hour. The reaction was quenched with saturated sodium sulfite and extracted with dichloromethane (100 mL×2). The organic layer was dried over anhydrous sodium sulfate and concentrated to obtain a crude product. The crude product was purified by silica gel column chromatography (methanol/dichloromethane = 0-10%) to obtain tert-butyl (5aS,6S,9R)-1-fluoro-12-(methylsulfinyl)-2-(8-((triisopropylsilyl)ethynyl)naphthalen-1-yl)-Sa,6,7,8,9,10-hexahy dro-5H-4-oxo-3,10a,11,13,14-pentaaza-6,9-methanonaphtho[1,8-ab]heptene-14-carboxyla te (230 mg, 2-step overall yield: 93.8%) which was white solid. ES-API:[M+H]⁺=756.3.

Step 3: Sodium hydride (50.0 mg, 1.25 mmol) was added to a solution of ((2R,7aS)-2-fluorotetrahydro-1H-pyrrolizin-7a(5H)-yl)methanol (100.0 mg, 0.628 mmol) in tetrahydrofuran (10.0 mL) at 0°C and stirred at this temperature for 10 minutes. Tert-butyl (5aS,6S,9R)-1-fluoro-12-(methylsulfinyl)-2-(8-((triisopropylsilyl)ethynyl)naphthalen-1-yl )-5a,6,7,8,9,10-hexahydro-5H-4-oxo-3,10a,11,13,14-pentaaza-6,9-methanonaphtho[1,8-ab ]heptene-14-carboxylate (230.0 mg, 0.304 mmol) in tetrahydrofuran (5 mL) was then added and stirred at 0°C for 1 hour. The reaction was quenched with saturated sodium bicarbonate solution and extracted with ethyl acetate. The organic phase was washed with saturated brine, dried over anhydrous sodium sulfate, filtered, and concentrated. The crude product was purified by silica gel column chromatography (methanol/dichloromethane=0-5%) to obtain tert-butyl (SaS,6S,9R)-1-fluoro-12-(((2R,7aS)-2-fluorotetrahydro-1H-pyrrolizin-7a(5H)-yl)methoxy)-2-(8-((triisopropylsilyl) ethynyl)naphthalen- 1-yl)-5a,6,7,8,9, 10-hexahydro-5H-4-oxo-3, 10a, 11, 13,14-pentaaza-6,9-methanonaphtho[1,8-ab]heptene-14-carboxylate (260 mg, crude product) which was yellow solid. ES-API: [M+H]⁺=851.5.

Step 4: Cesium fluoride (1000 mg, 6.583 mmol) was added to a solution of tert-butyl (5aS,6S,9R)-1-fluoro-12-(((2R,7aS)-2-fluorotetrahydro-1H-pyrrolizin-7a(5H)-yl)methoxy )-2-(8-((triisopropylsilyl)ethynyl)naphthalen-1-yl)-5a,6,7,8,9,10-hexahydro-5H-4-oxo-3,1 0a,11,13,14-pentaaza-6,9-methanonaphtho[1,8-ab]heptene-14-carboxylate (260 mg, crude product) in N,N-dimethylformamide (5.0 mL) and stirred at room temperature for 0.5 hours. After completion of the reaction, the reaction was quenched with water and extracted with ethyl acetate. The organic phase was washed with saturated brine, dried over anhydrous sodium sulfate, filtered, and concentrated to obtain tert-butyl (SaS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxo-3,10a,11,13,14-pentaaza-6,9-methanonaphtho[1,8-ab]heptene-14-car boxylate (120 mg, 2-step overall yield: 57%) which was yellow oil. ES-API: [M+H]⁺=695.3.

Step 5: tert-butyl (5aS,6S,9R)-2-(8-ethynaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxo-3, 10a,11,13,14-pentaaza-6,9-methanonaphtho[1,8-ab]heptene-14-carboxylate (120 mg, 0.173 mmol) reacted with acetonitrile (5.0 mL) and 4M hydrochloric acid/dioxane solution (2.0 ml, 8.000 mmol) at 0°C for 1 hour. The reaction mixture was concentrated. The crude product was purified by high-performance liquid chromatography (ammonium bicarbonate method) to obtain (5aS,6S,9R)-2-(8-ethynylnaphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaz a-6,9-methanonaphtho[1,8-ab]heptalene (Z350, 34 mg, yield: 33%) as a yellow solid. ES-API:[M+1]⁺=595.3. ¹H NMR (500 MHz, CD₃OD)δ 8.07 (d, *J* = 8.2 Hz, 1H), 8.03 (dd, *J* = 7.8, 3.6 Hz, 1H), 7.73 (ddd, *J* = 13.6, 7.2, 1.2 Hz, 1H), 7.67-7.47 (m, 3H), 5.30 (d, *J* = 54.1 Hz, 1H), 5.05 (ddd, *J* = 13.5, 6.6, 2.2 Hz, 1H), 4.59 (ddd, *J* = 13.2, 8.9, 2.0 Hz, 1H), 4.44 (ddd, *J* = 13.2, 7.4, 5.9 Hz, 1H), 4.28 (t, *J* = 10.1 Hz, 1H), 4.21 (dd, *J* = 10.5, 5.4 Hz, 1H), 4.12 (d, *J* = 6.2 Hz, 1H), 3.72 (s, 1H), 3.63 (d, *J* = 5.2 Hz, 1H), 3.27-3.16 (m, 4H), 3.02 (td, *J* = 9.8, 5.9 Hz, 1H), 2.29-2.21 (m, 1H), 2.16-2.10 (m, 1H), 1.99 (dq, *J* = 13.5, 6.7 Hz, 2H), 1.95-1.75 (m, 5H)_{∘}

### Example 7: Synthesis of 4-((5aS,6S,9R)-3-chloro-13-((2R,7aS)-2-fluorotetrahydro-1H-pyrrolizin-7a(5H)-ylmethoxy)-5a,6,7,8,9,10-hexahydro-5H-6,9-epiminoazepino[2',1':3 ,4][1,4]oxazepino[5,6,7-de]quinazolin-2-yl)-5-ethylnaphthalen-2-ol (Z381)

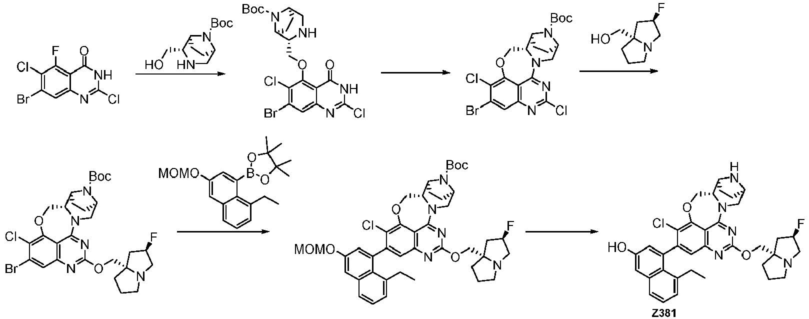

Step 1: Tert-butyl (1S,2S,5R)-2-(hydroxymethyl)-3,8-diazacyclo[3.2.1]octane-8-carboxylate (A2-1, 131 mg, 0.54 mmol) was added to a suspension of sodium hydride (60% dispersed in oil) (32 mg, 0.81 mmol) in tetrahydrofuran (10 mL) at 0°C and stirred at this temperature for 10 minutes. 7-Bromo-2,6-dichloro-5-fluoroquinazolin-4(3H)-one (170 mg, 0.54 mmol) was added and stirred with heating at 60°C for 1 hour. After completion of the reaction, the reaction was quenched with water and extracted with dichloromethane/methanol (10:1, 25 mL×3). The organic layer was dried over anhydrous sodium sulfate and rotoevaporated to obtain tert-butyl (1S,2S,5R)-2-(((7-bromo-2,6-dichloro-4-oxo-3,4-dihydroquinazolin-5-yl)oxy)methyl)-3,8-diazacyclo[3.2.1]octane-8-carboxylate (150 mg, yield: 51.0 %). ES-API:[M+H]⁺=533.2.

Step 2: 1H-Benzotriazol-1-yloxytripyrrolidinyl hexafluorophosphate (86 mg, 0.42 mmol) was added to a solution of tert-butyl (1S,2S,5R)-2-(((7-bromo-2,6-dichloro-4-oxo-3,4-dihydroquinazolin-5-yl)oxy)methyl)-3,8-diazacyclo[3.2.1]octane-8-carboxylate (110 mg, 0.21 mmol) and N,N-diisopropylethyl amine (80 mg, 0.62 mmol) in ultra-dried dichloromethane (10 mL) and stirred at room temperature for 16 hours. The reaction was quenched with water, extracted with dichloromethane (25 mL×3), and washed with saturated brine (10 mL). The organic layer was dried over anhydrous sodium sulfate, filtered, and concentrated. The obtained crude product was prepared by reverse phase HPLC (column: Waters XBridge C18, mobile phase: water/acetonitrile (0.1% trifluoroacetic acid) 0-80%, flow rate: 50 mL/min, column temperature: 25°C) to obtain tert-butyl (5aS,6S,9R)-2-bromo-3,13-dichloro-5a,6,7,8,9,10-hexahydro-5H-6,9-epiaminoazepino[2',1':3,4][1,4]oxazepino[5,6,7-de]quinazoline-15-carboxylate (64 mg, yield: 60.3 %). ES-API:[M+H]⁺= 515.1.

Step 3: ((2R,7aS)-2-Fluorotetrahydro-1H-pyrrolizin-7a(5H)-yl)methanol (29 mg, 0.18 mmol), potassium fluoride (51 mg, 0.9 mmol) and 4A molecular sieves were added to a solution of tert-butyl (5aS,6S,9R)-2-bromo-3,13-dichloro-5a,6,7,8,9,10-hexahydro-5H-6,9-epiminoazepino[2',1':3,4][1,4]oxazepino[5,6,7-de]quinazoline-15-carboxylate (45 mg, 0.09 mmol) in ultra-dried N,N-dimethylformamide (10 mL) and stirred at 120°C for 16 hours in a nitrogen atmosphere. After completion of the reaction, the reaction was quenched with water, extracted with dichloromethane (25 mL×3), and washed with saturated brine (10 mL). The organic layer was dried over anhydrous sodium sulfate, filtered, and concentrated to obtain a crude product. The crude product was prepared by reverse phase HPLC (column: Waters XBridge C18, mobile phase: water/acetonitrile (0.1% trifluoroacetic acid) (0-50%), flow rate: 50 mL/min, column temperature: 25°C) to obtain tert-butyl (5aS,6S,9R)-2-bromo-3-chloro-13-(((2R,7aS)-2-fluorotetrahydro-1H-pyrrolizin-7a(5H)-yl)methoxy)-5a,6,7,8,9,10-hexahydro-5H-6,9-epiminoazepino[2',1':3,4] [1,4]oxazepino[5,6,7-de]quinazoline-15-carboxylate (22 mg, yield: 38.4%). ES-API:[M+H]⁺= 638.2.

Step 4: Potassium phosphate (21 mg, 0.1mmol) and [n-butyl di(1-adamantyl)phosphine](2-amino-1,1'-biphenyl-2-yl)palladium (II) methanesulfonate (15 mg) were added to a solution of tert-butyl (5aS,6S,9R)-2-bromo-3-chloro-13-(((2R,7aS) -2-fluorotetrahydro-1H-pyrrolizin-7a(5H)-yl)methoxy)-5a,6,7,8,9,10-hexahydro-5H-6,9-e piminoazepino[2',1':3,4][1,4]oxazepino[5,6,7-de]quinazoline-15-carboxylate (22 mg, 0.03 mmol) and 2-(8-ethyl-3-(methoxymethoxy)naphthalen-1-yl)-4,4,5,5-tetramethyl-1,3,2-didioxaborolan e (17 mg, 0.05 mmol) in tetrahydrofuran (6 mL) and water (1.0 mL), and the system reacted at 60°C for 2 hours in a nitrogen atmosphere. After completion of the reaction, the reaction mixture was extracted with ethyl acetate (30 mL×2) and saturated brine (10 mL). The ethyl acetate phases were combined, dried over anhydrous sodium sulfate, and filtered. The filtrate was concentrated under reduced pressure to obtain a crude product. The crude product was purified by thin layer chromatography (methanol/dichloromethane=1:15) to obtain tert-butyl (5aS,6S,9R)-3-chloro-2-(8-ethyl-3-(methoxymethoxy)naphthalen-1-yl)-13-(((2R,7aS)-2-fluorotetrahydro-1H-pyrro lin-7a(5H)-ylmethoxy)-5a,6,7,8,9,10-hexahydro-5H-6,9-epiminoazepino[2',1':3,4][1,4]oxa zepino[5,6,7-de]quinazoline-15-carboxylate (10 mg, yield: 43%). ES-API: [M+H]⁺=774.1.

Step 5: tert-butyl (5aS,6S,9R)-3-chloro-2-(8-ethyl-3-(methoxymethoxy)naphthalen-1-yl)-13-(((2R,7aS)-2-fluorotetrahydro-1H-pyrrolin-7a(5H)-ylmethoxy)-5a,6,7,8,9,10-hexahydr o-5H-6,9-epiminoazepino[2',1':3,4][1,4]oxazepino[5,6,7-de]quinazoline-15-carboxylate (10 mg, 0.013 mmol) reacted with acetonitrile (2.0 mL) and 4M hydrochloric acid/dioxane solution (0.5 mL, 2.000 mmol) at 0°C for 1 hour. The reaction solution was concentrated. The obtained crude product was purified by high-performance liquid chromatography (formic acid method) to obtain 4-((5aS,6S,9R)-3-chloro-13-(((2R,7aS)-2-fluorotetrahydro-1H-pyrrolizin-7a(5H)-yl)metho xy)-5a,6,7,8,9,10-hexahydro-5H-6,9-epiminoazepino[2',1':3,4][1,4]oxazepino[5,6,7-de]qui nazolin-2-yl)-5-ethylnaphthalen-2-ol (Z381, formate, 5.0 mg, yield: 61.1%) as a yellow solid. ES-API:[M+1]⁺=630.3. ¹H NMR (400 MHz, CD₃OD)δ 8.51 (s, 2H), 7.59 (d, J = 8.1 Hz, 1H), 7.37-7.27 (m, 1H), 7.24-7.07 (m, 2H), 6.97 (d, J = 2.7 Hz, 1H), 6.79 (dd, J = 12.2, 2.6 Hz, 1H), 5.44 (d, J = 52.4 Hz, 1H), 5.09 (dd, J = 29.8, 11.5 Hz, 3H), 4.66-4.44 (m, 1H), 4.24-4.08 (m, 1H), 3.92-3.57 (m, 5H), 3.29-3.22 (m, 2H), 2.63-2.31 (m, 5H), 2.27-2.13 (m, 2H), 1.99 (dd, J = 30.6, 18.8 Hz, 5H), 0.91 (dt, J = 14.5, 7.4 Hz, 3H).

### Example 8: Synthesis of 5-ethynyl-6-fluoro-4-((5aS,6S,9R)-1-fluoro-12-(((2R,7aS)-2-fluorotetrahydro-1H-pyrrolizin-7a(5H)-ylmethoxy)-5a,6,7,8,9,10-hexahydro-5H-4-ox o-3,10a,11,13,14-pentaaza-6,9-methanonaphtho[1,8-ab]hepten-2-yl)naphthalen-2-ol (Z131-1)

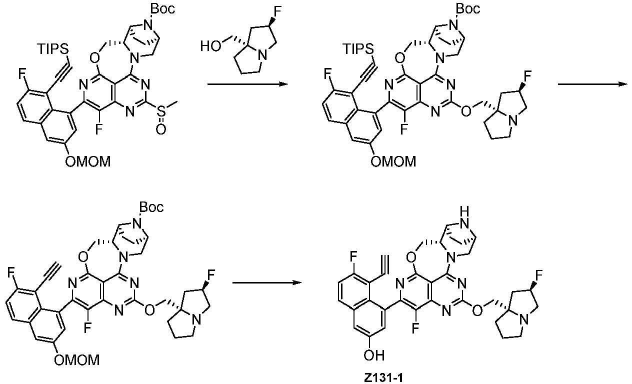

Step 1: Sodium hydride (50.0 mg, 1.25 mmol) was added to a solution of ((2R,7aS)-2-fluorotetrahydro-1H-pyrrolizin-7a(5H)-yl)methanol (100.0 mg, 0.628 mmol) in tetrahydrofuran (10.0 mL) at 0°C and stirred at this temperature for 10 minutes. A solution of tert-butyl (SaS,6S,9R)-1-fluoro-2-(7-fluoro-3-(methoxymethoxy)-8-((triisopropylsilyl) ethynyl)naphthalen-1-yl)-12-(methylsulfinyl)-5a,6,7,8,9,10-hexahydro-5H-4-oxo-3,10a,11 ,13,14-pentaaza-6,9-methanonaphtho[1,8-ab]heptene-14-carboxylate (210.0 mg, 0.252 mmol) in tetrahydrofuran (5 mL) was then added and stirred at 0°C for 1 hour. The reaction was quenched with saturated sodium bicarbonate solution and extracted with ethyl acetate. The organic phase was washed with saturated brine, dried over anhydrous sodium sulfate, filtered, and concentrated. The crude product was purified by silica gel column chromatography (0-5% methanol/dichloromethane) to obtain tert-butyl (SaS,6S,9R)-1-fluoro-2-(7-fluoro-3-(methoxymethoxy)-8-((triisopropylsilyl)ethynyl)naphthalen-1-yl)-12-(((2R,7aS)-2-fluorot etrahydro-1H-pyrrolizin-7a(5H)-yl)methoxy)-5a,6,7,8,9,10-hexahydro-5H-4-oxo-3,10a,11 ,13,14-pentaaza-6,9-methanonaphtho[1,8-ab]heptene-14-carboxylate (170.0 mg, yield: 72.6%) as a yellow solid. ES-API: [M+H]⁺=929.3.

Step 2: Cesium fluoride (1000 mg, 6.583 mmol) was added to a solution of tert-butyl (SaS,6S,9R)-1-fluoro-2-(7-fluoro-3-(methoxymethoxy)-8-((triisopropylsilyl)ethynyl)napht halen-1-yl)-12-(((2R,7aS)-2-fluorotetrahydro-1H-pyrrolizin-7a(5H)-yl)methoxy)-5a,6,7,8, 9,10-hexahydro-5H-4-oxo-3,10a, 11,13,14-pentaaza-6,9-methanonaphtho[1,8-ab]heptene-14-carboxylate (170 mg, 0.183 mmol) in N,N-dimethylformamide (5.0 mL) and stirred at room temperature for 0.5 hours. After completion of the reaction, the reaction was quenched with water and extracted with ethyl acetate. The organic phase was washed with saturated brine, dried over anhydrous sodium sulfate, filtered, and concentrated to obtain tert-butyl (5aS ,6S ,9R)-2-(8-ethynyl-7-fluoro-3 - (methoxymethoxy)naphthalen-1-yl)-1-fluoro-12-(((2R,7aS)-2-fluorotetrahydro-1H-pyrroli zin-7a(5H)-yl)methoxy)-5a,6,7,8,9,10-hexahydro-5H-4-oxo-3,10a,11,13,14-pentaaza-6,9-methanonaphtho[1,8-ab]heptene-14-carboxylate (200 mg, crude product) as a yellow oil. ES-API: [M+H]⁺=773.3.

Step 3: tert-butyl (5aS,6S,9R)-2-(8-ethynyl-7-fluoro-3-(methoxymethoxy)naphthalen-1-yl) -1-fluoro-12-(((2R,7aS)-2-fluorotetrahydro-1H-pyrrolizin-7a(5H)-yl)methoxy)-5a,6,7,8,9, 10-hexahydro-5H-4-oxo-3,10a,11,13,14-pentaaza-6,9-methanonaphtho[1,8-ab]heptene-14 -carboxylate (200 mg, crude product) reacted with acetonitrile (10.0 mL) and 4M hydrochloric acid/dioxane solution (2.0 mL, 8.000 mmol) at 0°C for 1 hour. The reaction mixture was concentrated. The crude product was purified by high-performance liquid chromatography (ammonium bicarbonate method) to obtain 5-ethynyl-6-fluoro-4-((SaS,6S,9R)-1-fluoro-12-(((2R,7aS)-2-fluorotetrahydro-1H-pyrrolizin-7a(5H)-yl)methoxy)-5a,6,7,8,9,10-hexahydro-5H-4-oxa-3, 10a,11,13,14-pentaaza-6,9-methanonaphtho[1,8-ab]heptalen-2-yl)naphthalen-2-ol (Z131-1, 8.95 mg, 2-step overall yield: 7.8%) as a yellow solid. ES-API:[M+1]⁺=629.3.

### Example 9: Synthesis of 5-ethyl-4-((5aS,6S,9R)-12-((1-methyl-1-azaspiro[4.4]nonan-6-yl) oxy)-5a,6,7,8,9,10-hexahydro-5H-4-oxa-3,10a,11,13,14-pentaaza[6,9]methanonaphtho [1,8-ab]hepten-2-yl)naphthalen-2-ol (Z260-1)

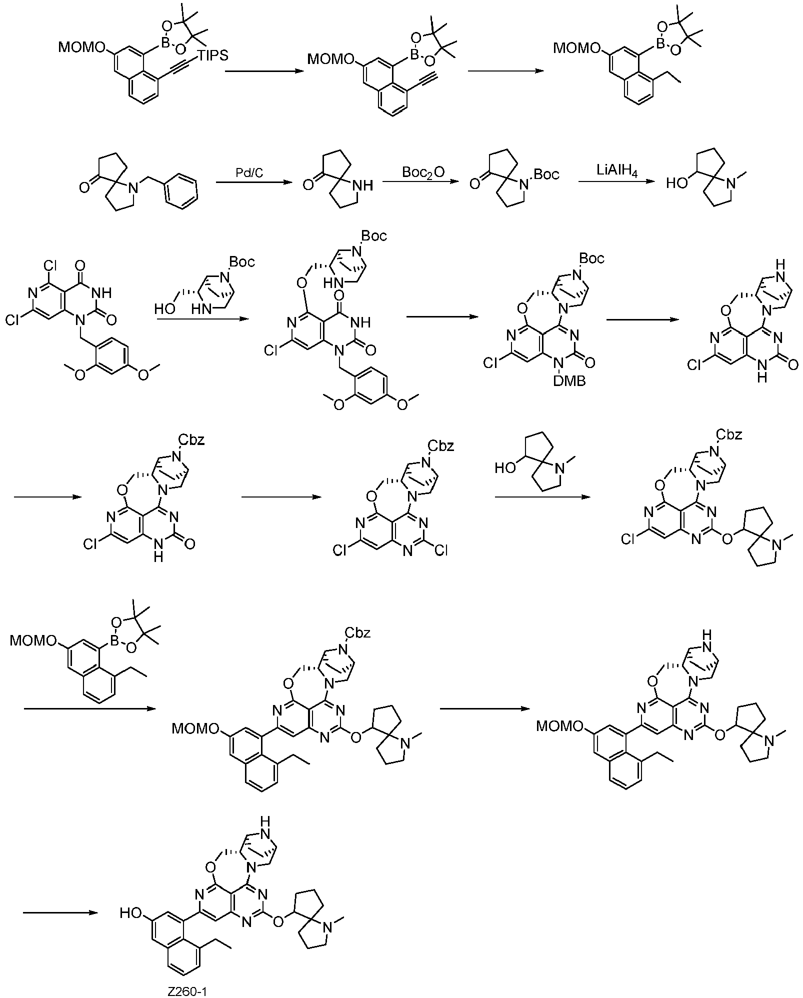

Step 1: Cesium fluoride (3.0 g, 20.24 mmol) was added to a solution of triisopropyl ((6-methoxymethoxy)-8-(4,4,5,5-tetramethyl-1,3,2-dioxaborolane-2-yl)naphthalen-1-yl)et hynyl)silane (500 mg, 1.012 mmol) in N,N-dimethylformamide (5.0 mL), and the system reacted at room temperature for 2 hours. After completion of the reaction, the reaction mixture was extracted with ethyl acetate (100 mL×2). The ethyl acetate phases were combined, washed with saturated brine (100 mL×3), dried over anhydrous sodium sulfate, filtered, and rotoevaporated to obtain 2-(8-ethynyl-3-(methoxymethoxy)naphthalen-1-yl)-4,4,5,5-tetramethyl-1,3,2-dioxaborola ne (558 mg, crude product). ES-API:[M+H]⁺=339.3.

Step 2: Palladium on carbon with a mass fraction of 10% was added to a solution of 2-(8-ethynyl-3-(methoxymethoxy)naphthalen-1-yl)-4,4,5,5-tetramethyl-1,3,2-dioxaborina ne (558 mg, crude product) in methanol (10.0 mL). The reaction system was replaced with hydrogen 4 times and reacted at room temperature for 2 hours in the presence of protective hydrogen. After completion of the reaction, the reaction mixture was filtered. The solvent was rotoevaporated under reduced pressure to obtain 2-(8-ethyl-3-(methoxymethoxy) naphthalen-1-yl)-4,4,5,5-tetramethyl-1,3,2-dioxaborolane (284 mg, 2-step overall yield: 82%). ES-API:[M+1]⁺=343.3.

Step 3: 1-Benzyl-1-azaspiro[4.4]nonan-6-one (1.5 g, 6.5 mmol) was dissolved in methanol (10 mL), and palladium on carbon catalyst (150 mg) was added. The reaction system was replaced with hydrogen 3 times and reacted at room temperature for 4 hours. The reaction mixture was filtered, washed with methanol, and concentrated to obtain 1-azaspiro[4.4]nonan-6-one (800 mg, yield: 88.5%). ES-API:[M+H]⁺=140.1.

Step 4: 1-azaspiro[4.4]nonan-6-one (800 mg, 5.7 mmol) was dissolved in tetrahydrofuran (10 mL). Triethylamine (0.86 mg, 8.5 mmol) and di-tert-butyl dicarbonate (1.8 g, 8.5 mmol) were added at room temperature and stirred at room temperature for 8 hours. The reaction was monitored by LCMS for completion, and water (10 ml) was added. The reaction mixture was extracted with ethyl acetate, dried over anhydrous sodium sulfate, and concentrated to obtain a crude product. The crude product was purified by silica gel column chromatography (ethyl acetate/petroleum ether=0~80%) to obtain tert-butyl 6-oxo-1-azaspiro[4.4]nonane-1-carboxylate (900 mg, yield: 66%). ES-API:[M+H]⁺=240.1.

Step 5: tert-butyl 6-oxo-1-azaspiro[4.4]nonane-1-carboxylate (900 mg, 3.7 mmol) was dissolved in dry tetrahydrofuran (10 mL). Lithium aluminum hydride (11 ml, 11 mmol, 1M tetrahydrofuran) was added under an ice-water bath condition and stirred at 60°C for 2 hours. Water (10 mL) was added. The obtained mixture was extracted with ethyl acetate, dried over anhydrous sodium sulfate, and concentrated to obtain 1-methyl-1-azaspiro[4.4]nonan-6-ol (450 mg), which was used directly in the next step. ES-API:[M+Na]⁺=156.1.

Step 6: Sodium hydride (80 mg, 2.0 mmol) was added to a solution of tert-butyl (1S,2S,5R)-2-(hydroxymethyl)-3,8-diazacyclo[3.2.1]octane-8-carboxylate (A2-1, 242 mg, 1.0 mmol) in tetrahydrofuran (10 mL) at 0°C. After reaction for 10-20 minutes in a nitrogen atmosphere, 5,7-dichloro-1-(2,4-dimethoxybenzyl)pyrido[4,3-d]pyrimidine-2,4(1H,3H)-dione (381 mg, 1.0 mmol) was added in batches. The reaction was continually carried out at 0~5°C for 20-30 minutes and monitored by LCMS for completion. The reaction solution was poured into ice water (300 mL), adjusted to the pH 7-8 with 6M aqueous hydrochloric acid under an ice-water bath condition, and extracted with ethyl acetate (100 mL×2). The organic phases were combined, washed with saturated brine (100 mL×1), dried over anhydrous sodium sulfate, filtered, and rotoevaporated to obtain a target compound tert-butyl (1S,2S,5R)-2-(((7-chloro-1-(2,4-dimethoxybenzyl)-2,4-dioxa-1,2,3,4-tetrahydropyrido[4,3-d]pyrimidin-5-yl)oxy)meth yl)-3,8-diazabicyclo[3.2.1]octane-8-carboxylate (560 mg, yield: 87%). ES-API: [M+H]⁺=588.2.

Step 7: Dried N,N-dimethylformamide (15 mL) and tert-butyl (1S,2S,5R)-2-(((7-chloro-1-(2,4-dimethoxybenzyl)-2,4-dioxa-1,2,3,4-tetrahydropyrido[4,3-d]pyrimidin-5-yl)oxy)meth yl)-3,8-diazabicyclo[3.2.1]octane-8-carboxylate (510 mg, 0.87 mmol) were added to a 250 mL single-neck flask at room temperature followed by BOP reagent (1.9 g, 4.35 mmol). After the reaction for 5 minutes at room temperature, 1,8-diazabicyclo[5.4.0]undec-7-ene (661 mg, 4.35 mmol) was added dropwise, and the system reacted at room temperature for 1-2 hours. After completion of the reaction, the above-mentioned solution was slowly added dropwise into ice water (100 mL). A large amount of solid was precipitated and filtered. The filter cake was rotoevaporated under reduced pressure and purified by column chromatography (petroleum ether/ethyl acetate=1/1) to obtain tert-butyl (5aS ,6S ,9R)-2-chloro-13 -(2,4-dimethoxybenzyl)-12-oxo-5a,6,7,8,9,10,12,13-octahydro-5H-4-oxa-3, 10a, 11, 13,14-pentaaza-6,9-methanonap htho[1,8-ab]heptene-14-carboxylate (300 mg, yield: 61%). ES-API: [M+H]⁺=568.2.

Step 8: tert-butyl (5aS,6S,9R)-2-chloro-13-(2,4-dimethoxybenzyl)-12-oxo-5a,6,7,8,9,10,12,13-octahydro-5H-4-oxa-3,10a,11,13,14-pentaaza-6,9-methanonaphtho[1, 8-ab]heptene-14-carboxylate (300 mg, 0.53 mmol) was added to trifluoroacetic acid (5 mL) and stirred at 55°C for 1 hour. After completion of the reaction, the solvent was rotoevaporated under reduced pressure to obtain a compound (5aS,6S,9R)-2-chloro-5a,6,7,8,9,10-hexahydro-5H-4-oxa-3,10a,11,13,14-pentaaza-6,9-methylenaphtho[1,8-ab]hepten-12(13H)-one (500 mg, crude product). ES-API: [M+H]⁺=320.1.

Step 9: Sodium carbonate (168 mg, 1.59 mmol) was added to a mixed solution of (5aS,6S,9R)-2-chloro-5a,6,7,8,9,10-hexahydro-5H-4-oxa-3,10a,11,13,14-pentaaza-6,9-met hanonaphtho[1,8-ab]hepten-12(13H)-one (500 mg, crude product) in tetrahydrofuran (5.0 mL) and water (5.0 mL), and the reaction system was cooled in an ice-water bath. Carbobenzoxysuccinimide (171 mg, 0.68 mmol) was added, and the system reacted at room temperature for 3 hours. After completion of the reaction, dichloromethane (80 mL) was added to the reaction solution. The obtained mixture was washed with saturated aqueous sodium bicarbonate solution (100 mL) and saturated brine (80 mL), dried over anhydrous sodium sulfate, and concentrated to obtain a crude product. The crude product was purified by silica gel column chromatography (ethyl acetate/petroleum ether: 0-50%) to obtain a target product of benzyl (5aS,6S,9R)-2-chloro-12-oxa-5a,6,7,8,9,10,12,13-octahydro-5H-4-oxa-3,10a,11,13,14-pen taaza-6,9-methanonaphtho[1,8-ab]heptene-14-carboxylate (396 mg, yield: 93%). ES-API: [M+H]⁺=454.1.

Step 10: Phosphorus oxychloride (365 mg, 2.4 mmol) and N,N-diisopropylethyl amine (310 mg, 2.4 mmol) were added successively to a solution of benzyl (5aS,6S,9R)-2-chloro-12-oxa-5a,6,7,8,9,10,12,13-octahydro-5H-4-oxa-3,10a,11,13,14-pentaaza-6,9-methanonaphtho[1, 8-ab]heptene-14-carboxylate (390 mg, 0.8 mmol) in toluene (5.0 mL) at 0°C, and the system reacted at 105°C for 12 hours. After completion of the reaction, the reaction system was cooled to room temperature, and the solvent was rotoevaporated under reduced pressure. The crude product was purified by high-performance liquid chromatography (ammonium bicarbonate method) to obtain benzyl (5aS,6S,9R)-2,12-dichloro-5a,6,7,8,9,10-hexahydro-5H-4-oxa-3,10a, 11,13,14-pentaaza-6,9-methanonaphtho[1,8-ab]heptene-14-carboxylate (70 mg, yield: 17%). ES-API: [M+H]⁺=472.1.

Step 11: Potassium fluoride (29 mg, 0.5 mmol), N,N-diisopropylethylene diamine (39 mg, 0.3 mmol), 1-methyl-1-azaspiro[4.4]nonan-6-ol (31 mg, 0.2 mmol) and 4A molecular sieves were added to a solution of benzyl (5aS,6S,9R)-2,12-dichloro-5a,6,7,8,9,10-hexahydro-5H-4-oxa-3,10a,11,13,14-pentaaza-6,9-methanonaphtho[1,8-ab]heptene-14-carboxylate (50 mg, 0.1 mmol) in dimethyl sulfoxide (3.0 mL), and the system reacted at 110°C for 4 hours in a nitrogen atmosphere. After completion of the reaction, the reaction system was cooled to room temperature and extracted with ethyl acetate (50 mL×2). The ethyl acetate phases were combined, washed with saturated brine (50 mL×4), dried over anhydrous sodium sulfate, filtered, and rotoevaporated. The crude product was purified by silica gel column chromatography (petroleum ether/ethyl acetate=2:1) to obtain benzyl (5aS,6S,9R)-2-chloro-12-((1-methyl-1-azaspiro[4.4]nonan-6-yl) oxy)-5a,6,7,8,9,10-hexahydro-5H-4-oxa-3,10a,11,13,14-pentaaza-6,9-methanonaphtho[1,8 -ab]heptene-14-carboxylate (28 mg, yield: 44%). ES-API: [M+H]⁺=591.2.

Step 12: benzyl (5aS,6S,9R)-2-chloro-12-((1-methyl-1-azaspiro[4.4]nonan-6-yl)oxy)-5a,6,7,8,9,10-hexahydro-5H-4-oxa-3,10a,11,13,14-pentaaza-6,9-methanonaphtho[1,8-ab]h eptene-14-carboxylate (28 mg, 0.047 mmol) was dissolved in tetrahydrofuran/water (2 mL/0.5 mL). The 2-(8-ethyl-3-(methoxymethoxy)naphthalen-1-yl)-4,4,5,5-tetramethyl-1,3,2-dioxaborolane (24 mg, 0.07 mmol), potassium phosphate (30 mg, 0.14 mmol) and [n-butyl di(1-adamantyl)phosphine](2-amino-1,1'-biphenyl-2-yl)palladium (II) methanesulfonate (10 mg, 0.014 mmol) were added. The reaction system was replaced with nitrogen 4 times and reacted at 60°C for 1 hour. The reaction solution was cooled to room temperature and extracted with ethyl acetate (80 mL) and water (60 mL). The organic phase was dried over anhydrous sodium sulfate and filtered. The filtrate was rotoevaporated under reduced pressure to obtain a crude product. The crude product was purified by silica gel column chromatography (ethyl acetate/petroleum ether: 0-70%) to obtain benzyl (5aS,6S,9R)-2-(8-ethyl-3-(methoxymethoxy) naphthalen-1-yl)-12-((1-methyl-1-azaspiro[4.4]nonan-6-yl)oxy)-5a,6,7,8,9,10-hexahydro-5H-4-oxy-3,10a,11,13,14-pentaaza-6,9-methanonaphtho[1,8-ab]heptene-14-carboxylate (26 mg, yield: 73%). ES-API: [M+H]⁺=771.4.

Step 13: Palladium on carbon with a mass fraction of 10% (50 mg) was added to a solution of benzyl (5aS,6S,9R)-2-(8-ethyl-3-(methoxymethoxy)naphthalen-1-yl)-12-((1-methyl-1-azaspiro[4. 4]nonan-6-yl)oxy)-5a,6,7,8,9,10-hexahydro-5H-4-oxy-3,10a,11,13,14-pentaaza-6,9-metha nonaphtho[1,8-ab]heptene-14-carboxylate (26 mg, 0.034 mmol) in methanol (10.0 mL). The reaction system was replaced with hydrogen 4 times and reacted at room temperature under a hydrogen atmosphere for 2 hours. The reaction mixture was filtered to give a crude target compound (5aS,6S,9R)-2-(8-ethyl-3-(methoxymethoxy)naphthalen-1-yl)-12-((1-methyl-1-azaspiro[4. 4]nonan-6-yl)oxy)-5a,6,7,8,9,10-hexahydro-5H-4-oxy-3,10a,11,13,14-pentaaza-6,9-metha nonaphtho[1,8-ab]heptene (17 mg, yield: 80%). ES-API: [M+H]⁺=637.3.

Step 14: (5aS,6S,9R)-2-(8-ethyl-3-(methoxymethoxy)naphthalen-1-yl)-12-((1-methyl-1-azaspiro[4.4]nonan-6-yl)oxy)-5a,6,7,8,9,10-hexahydro-5H-4-oxy-3,10a,11,1 3,14-pentaaza-6,9-methanonaphtho[1,8-ab]heptene (17 mg, 0.027 mmol) was dissolved in acetonitrile (6.0 mL). 4M Hydrochloric acid/dioxane solution (1.0 mL, 4.0 mmol) was added under an ice-water bath condition, and the system reacted in an ice-water bath for 0.5 hours. After completion of the reaction, the solvent was rotoevaporated under reduced pressure. Dichloromethane (20 mL) was then added, and triethylamine (3.0 mL) was added under an ice-water bath condition. After stirring for 10 minutes, the obtained mixture was extracted with dichloromethane (100 mL×1) and water (50 mL×1). The dichloromethane phase was dried over anhydrous sodium sulfate and filtered. The filtrate was rotoevaporated under reduced pressure to obtain a crude product. The crude product was purified by high-performance liquid chromatography (formic acid method) to obtain 5-ethyl-4-((5aS,6S,9R)-12-((1-methyl-1-azaspiro[4.4]nonan-6-yl)oxy)-5a,6,7,8,9,10-hexahydro-5H-4-oxa-3,10a,11,13,14-pentaa za[6,9]methanonaphtho[1,8-ab]heptalen-2-yl)naphthalen-2-ol (Z260-1, formate, 1.6 mg, yield: 10%). ES-API: [M+H]⁺=593.3.

### Example 10: Synthesis of (5aS,6S,9R)-1-fluoro-12-(((2R,7aS)-2-fluorotetrahydro-1H-pyrrolizin-7a(5H)-yl)methoxy)-2-(5-methyl-1H-indazol-4-yl)-5a,6,7,8,9,10-hexahydro-5H-4-oxo-3,10a,11,13,14-pentaaza-6,9-methanonaphtho[1,8-ab]heptene (Z382)

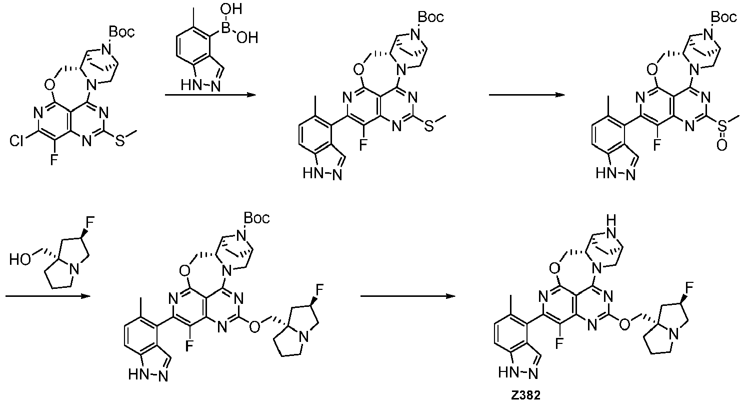

Step 1: Cesium carbonate (560 mg, 1.719 mmol) and tetrakis triphenylphosphine palladium (100 mg, 0.87 mmol) were added to a solution of tert-butyl (1S,2S,5R)-2-(((7-chloro-8-fluoro-(2-methylthio)-4-oxa-3,4-dihydropyrido[4,3-d]pyrimidin-5-yl)oxy)methyl)-3,8-diazabicyc lo[3.2.1]octane-8-carboxylate (200 mg, 0.427 mmol) and (5-methyl-1H-indazol-4-yl)boronic acid (230.0 mg, 1.307 mmol) in tetrahydrofuran (10.0 mL) and water (2.0 mL). The reaction system was sparged with nitrogen and heated at 115°C for 6 hours. After completion of the reaction, the reaction mixture was extracted with ethyl acetate (30 mL×2) and saturated brine (30 mL). The ethyl acetate phases were combined, dried over anhydrous sodium sulfate, and filtered. The filtrate was concentrated under reduced pressure. The crude product was purified by silica gel column chromatography (methanol/dichloromethane=0-4%) to obtain tert-butyl (5aS,6S,9R)-1-fluoro-2-(5-methyl-1H-indazol-4-yl)-12-(methylthio)-5a,6,7,8,9,10-hexahydro-5H-4-oxo-3,10a,11, 13,14-pentaaza-6,9-methanonaphtho[1,8-ab]heptene-14-carboxylate (400 mg, crude product) as a yellow solid. ES-API: [M+H]⁺=564.4.

Step 2: m-Chloroperoxybenzoic acid (85 mg, 0.493 mmol) was added to a solution oftert-butyl (SaS,6S,9R)-1-fluoro-2-(5-methyl-1H-indazol-4-yl)-12-(methylthio)-Sa,6,7,8,9,10-hexahy dro-5H-4-oxo-3,10a,11,13,14-pentaaza-6,9-methanonaphtho[1,8-ab]heptene-14-carboxyla te (180 mg, 0.319 mmol) in dichloromethane (15 mL) at 0°C and stirred at room temperature for 1 hour. The reaction was quenched with saturated sodium sulfite and extracted with dichloromethane (100 mL×2). The organic layer was dried over anhydrous sodium sulfate and concentrated to obtain a crude product. The crude product was purified by silica gel column chromatography (methanol/dichloromethane=0-10%) to obtain tert-butyl (5aS,6S,9R)-1-fluoro-2-(5-methyl-1H-indazol-4-yl)-12-(methylsulfinyl)-5a,6,7,8,9,10-hexahydro-5H-4-oxo-3,10a, 11,13,14-pentaaza-6,9-methanonaphtho[1,8-ab]heptene-14-carboxylate (200 mg, crude product) which was white solid. ES-API:[M+H]⁺=580.3.

Step 3: Sodium hydride (55.0 mg, 1.382 mmol) was added to a solution of ((2R,7aS)-2-fluorotetrahydro-1H-pyrrolizin-7a(5H)-yl)methanol (110.0 mg, 0.691 mmol) in tetrahydrofuran (10.0 mL) at 0°C and stirred at this temperature for 10 minutes. A solution oftert-butyl (SaS,6S,9R)-1-fluoro-2-(5-methyl-1H-indazol-4-yl)-12-(methylsulfinyl)-5a,6,7,8,9,10-hexahydro-5H-4-oxo-3,10a,11,13,14-pentaaza-6,9-methanonaphtho[1,8-ab]h eptene-14-carboxylate (200 mg, crude product) in tetrahydrofuran (5 mL) was added and stirred at 0°C for 1 hour. The reaction was quenched with saturated sodium bicarbonate solution and extracted with ethyl acetate. The organic phase was washed with saturated brine, dried over anhydrous sodium sulfate, filtered, and concentrated to obtain a crude product. The crude product was purified by silica gel column chromatography (methanol/dichloromethane=0-5%) to obtain tert-butyl (SaS,6S,9R)-1-fluoro-12-(((2R,7aS)-2-fluorotetrahydro-1H-pyrrolizin-7a(5H)-yl)methoxy)-2-(5-methyl-1H-indazol-4-yl)-5a,6,7,8,9,10-hexahydro-5H -4-oxo-3,10a,11,13,14-pentaaza-6,9-methylenaphtho[1,8-ab]heptene-14-carboxylate (220 mg, 3-step overall yield: 94.5%) which was yellow solid. ES-API: [M+H]⁺=675.3.

Step 4: At 0°C, 4M hydrochloric acid/dioxane solution (2.0 mL, 8.000 mmol) was added to a solution of tert-butyl (SaS,6S,9R)-1-fluoro-12-(((2R,7aS)-2-fluorotetrahydro-1H-pyrrolizin-7a(5H)-yl)methoxy)-2-(5-methyl-1H-indazol-4-yl)-5a,6,7,8,9,10-hexahydro-5H -4-oxo-3,10a,11,13,14-pentaaza-6,9-methanonaphtho[1,8-ab]heptene-14-carboxylate (200 mg, 0.296 mmol) in acetonitrile (5.0 mL), and the reaction solution reacted at 0°C for 1 hour. The reaction mixture was concentrated. The crude product was purified by high-performance liquid chromatography (ammonium bicarbonate method) to obtain (5aS,6S,9R)-1-fluoro-12-(((2R,7aS)-2-fluorotetrahydro-1H-pyrrolizin-7a(5H)-yl)methoxy)-2-(5-methyl-1H-indazol -4-yl)-5a,6,7,8,9,10-hexahydro-5H-4-oxa-3,10a,11,13,14-pentaaza-6,9-methanonaphtho[1, 8-ab]heptalene (Z382, 30 mg, yield: 17.6%) which was yellow solid. ES-API:[M+1]⁺=575.3. ¹H NMR (500 MHz, CD₃OD)δ 8.39 (s, 2H), 7.74 (d, *J* = 27.1 Hz, 1H), 7.59 (d, *J* = 8.6 Hz, 1H), 7.40 (dd, *J* = 8.6, 3.2 Hz, 1H), 5.52 (d, *J* = 52.0 Hz, 1H), 5.20 (t, *J* = 12.5 Hz, 1H), 4.82-4.48 (m, 4H), 4.33 (d, *J* = 5.9 Hz, 1H), 4.20-3.67 (m, 4H), 3.37 (dd, *J* = 23.7, 13.9 Hz, 2H), 2.73-1.87 (m, 12H).

### Example 11: Synthesis of 5-ethynyl-6-fluoro-4-((5aS,6S,9R)-1-fluoro-12-((hexahydro-1H-pyrrolizin-3-yl)methoxy)-5a,6,7,8,9,10-hexahydro-5H-4-oxo-3,10a,11 ,13,14-pentaaza-6,9-methanonaphtho[1,8-ab]hepten-2-yl)naphthalen-2-ol (Z383)

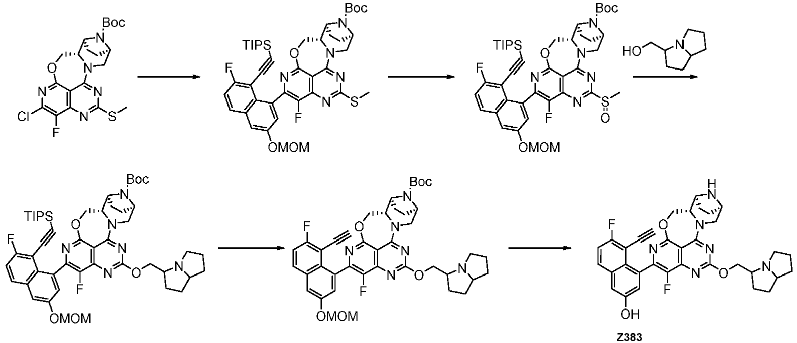

Step 1: Potassium phosphate (200 mg, 0.943 mmol) and [n-butyl di(1-adamantyl)phosphine](2-amino-1,1'-biphenyl-2-yl)palladium (II) methanesulfonate (50 mg, 0.069 mmol) were added to a solution of tert-butyl (1S,2S,5R)-2-(((7-chloro-8-fluoro-(2-methylthio)-4-oxa-3,4-dihydropyrido[4,3-d]pyrimidin-5-yl)oxy)methyl)-3,8-diazabicyc lo[3.2.1]octane-8-carboxylate (100 mg, 0.214 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (200.0 mg, 0.390 mmol) in tetrahydrofuran (6 mL) and water (1.5 mL). The reaction system was sparged with nitrogen and reacted under microwave at 80°C for 40 minutes. After completion of the reaction, the reaction mixture was extracted with ethyl acetate (30 mL×2) and saturated brine (30 mL). The ethyl acetate phases were combined, dried over anhydrous sodium sulfate, and filtered. The filtrate was concentrated under reduced pressure to obtain a crude product. The crude product was purified by silica gel column chromatography (methanol/dichloromethane=0-4%) to obtain tert-butyl (SaS,6S,9R)-1-fluoro-2-(7-fluoro-3-(methoxymethoxy)-8-((triisopropylsilyl) ethynyl)naphthalen-1-yl)-12-(methylthio)-5a,6,7,8,9,10-hexahydro-5H-4-oxo-3,10a,11,13, 14-pentaaza-6,9-methanonaphtho[1,8-ab]heptene-14-carboxylate (210 mg, crude product) as a yellow solid. ES-API: [M+H]⁺=818.3.

Step 2: m-Chloroperoxybenzoic acid (55 mg, 0.320 mmol)was added to a solution of tert-butyl (SaS,6S,9R)-1-fluoro-2-(7-fluoro-3-(methoxymethoxy)-8-((triisopropylsilyl)ethynyl)napht halen-1-yl)-12-(methylthio)-5a,6,7,8,9,10-hexahydro-5H-4-oxo-3,10a,11,13,14-pentaaza-6 ,9-methanonaphtho[1,8-ab]heptene-14-carboxylate (210 mg, crude product) in dichloromethane (15 mL) at 0°C and stirred at room temperature for 1 hour. The reaction was quenched with saturated sodium sulfite and extracted with dichloromethane (100 mL×2). The organic layer was dried over anhydrous sodium sulfate and concentrated to obtain a crude product. The crude product was purified by silica gel column chromatography (methanol/dichloromethane=0-10%) to obtain tert-butyl (SaS,6S,9R)-1-fluoro-2-(7-fluoro-3-(methoxymethoxy)-8-((triisopropylsilyl) ethynyl)naphthalen-1-yl)-12-(methylsulfinyl)-5a,6,7,8,9,10-hexahydro-5H-4-oxo-3,10a,11 ,13,14-pentaaza-6,9-methanonaphtho[1,8-ab]heptene-14-carboxylate (210 mg, crude product) which was white solid. ES-API:[M+H]⁺=834.4.

Step 3: Sodium hydride (54.0 mg, 1.34 mmol) was added to a solution of (hexahydro-1H-pyrrolizin-3-yl)methanol (95.0 mg, 0.673 mmol) in tetrahydrofuran (10.0 mL) at 0°C and stirred at this temperature for 10 minutes. A solution of tert-butyl (SaS,6S,9R)-1-fluoro-2-(7-fluoro-3-(methoxymethoxy)-8-((triisopropylsilyl)ethynyl)napht halen-1-yl)-12-(methylsulfinyl)-5a,6,7,8,9,10-hexahydro-5H-4-oxo-3,10a,11,13,14-pentaa za-6,9-methanonaphtho[1,8-ab]heptene-14-carboxylate (270.0 mg, 0.324 mmol) in tetrahydrofuran (5 mL) was added and stirred at 0°C for 1 hour. The reaction was quenched with saturated sodium bicarbonate solution and extracted with ethyl acetate. The organic phase was washed with saturated brine, dried over anhydrous sodium sulfate, filtered, and concentrated to obtain a crude product. The crude product was purified by silica gel column chromatography (methanol/dichloromethane=0-5%) to obtain tert-butyl (SaS,6S,9R)-1-fluoro-2-(7-fluoro-3-(methoxymethoxy)-8-((triisopropylsilyl)ethynyl)naphthalen-1-yl)-12-((hexahydro-1H-pyrr olizin-3-yl)methoxy)-5a,6,7,8,9,10-hexahydro-5H-4-oxo-3,10a,11,13,14-pentaaza-6,9-met hanonaphtho[1,8-ab]heptene-14-carboxylate (300 mg, crude product) as a yellow solid. ES-API: [M+H]⁺=911.5.

Step 4: Cesium fluoride (1000 mg, 6.583 mmol) was added to the solution of tert-butyl (SaS,6S,9R)-1-fluoro-2-(7-fluoro-3-(methoxymethoxy)-8-((triisopropylsilyl)ethynyl)napht halen-1-yl)-12-((hexahydro-1H-pyrrolizin-3-yl)methoxy)-5a,6,7,8,9,10-hexahydro-5H-4-o xo-3,10a,11,13,14-pentaaza-6,9-methanonaphtho[1,8-ab]heptene-14-carboxylate (300 mg, crude product) in N,N-dimethylformamide (5.0 mL) and stirred at room temperature for 0.5 hours. After completion of the reaction, the reaction was quenched with water and extracted with ethyl acetate. The organic phase was washed with saturated brine, dried over anhydrous sodium sulfate, filtered, and concentrated to obtain tert-butyl (5aS,6S,9R)-2-(8-ethynyl-7-fluoro-3-(methoxymethoxy) naphthalen-1-yl)-1-fluoro-12-((hexahydro-1H-pyrrolizin-3-yl)methoxy)-5 a,6,7,8,9,10-hex ahydro-5H-4-oxo-3,10a,11,13,14-pentaaza-6,9-methanonaphtho[1,8-ab]heptene-14-carbox ylate (150 mg, 3-step overall yield: 60.3%) which was yellow oil. ES-API: [M+H]⁺=755.3.

Step 5: tert-butyl (5aS,6S,9R)-2-(8-ethynyl-7-fluoro-3-(methoxymethoxy)naphthalen-1-yl)-1-fluoro-12-((hexahydro-1H-pyrrolizin-3-yl)methoxy)-5a,6,7,8,9,10-hexahydro-5H-4-oxo -3,10a,11,13,14-pentaaza-6,9-methoxynaphtho[1,8-ab]heptene-14-carboxylate (150 mg, 0.199 mmol) reacted with acetonitrile (5.0 mL) and 4M hydrochloric acid/dioxane solution (2.0 mL, 8.000 mmol) at 0°C for 1 hour. The reaction was concentrated. The crude product was purified by high-performance liquid chromatography (formic acid method) to obtain 5-ethynyl-6-fluoro-4-((5aS,6S,9R)-1-fluoro-12-((hexahydro-1H-pyrrolizin-3-yl)methoxy)-5a,6,7,8,9,10-hexahy dro-5H-4-oxa-3,10a,11,13,14-pentaaza-6,9-methanonaphtho[1,8-ab]heptalen-2-yl)naphtha len-2-ol (Z383, formate, 49.0 mg, yield: 41%) as a yellow solid. ES-API:[M+1]⁺=611.3. ¹H NMR (500 MHz, CD₃OD) δ 8.43 (s, 2H), 7.88-7.83 (m, 1H), 7.35-7.16 (m, 3H), 5.07 (d, *J* = 13.7 Hz, 1H), 4.72-4.60 (m, 2H), 4.57-4.46 (m, 1H), 4.37-4.19 (m, 2H), 4.11 (s, 1H), 3.86 (d, *J* = 21.8 Hz, 2H), 3.66-3.40 (m, 3H), 2.47-1.66 (m, 13H).

### Example 12: Synthesis of 1-(8-((5aS,6S,9R)-1-fluoro-12-((2R,7aS)-2-fluorotetrahydro-1H-pyrrolizin-7a(5H)-yl)methoxy)-5a,6,7,8,9,10-hexahydro-5H-4-oxo-3,10a,11,13,14-pentaaza-6,9-methanonaphtho[1,8-ab]hepten-2-yl)naphthalen-1-yl)propanol (Z385)

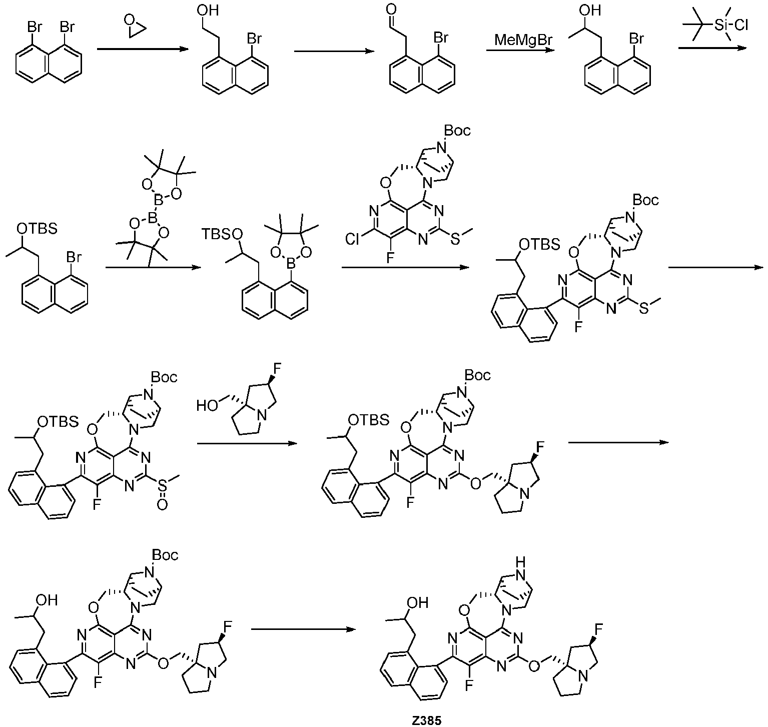

Step 1: 1,8-Dibromonaphthalene (4 g, 13.99 mmol) and tetrahydrofuran (40 mL) were added to a round-bottom flask in a nitrogen atmosphere. The reaction system was cooled to -70°C, and 2.5M butyllithium in n-hexane (6.15 mL, 15.39 mmol) was added dropwise to the above-mentioned solution. Upon completion of the addition, the solution was stirred at -70°C for 30 minutes, and a 3M solution of ethylene oxide in tetrahydrofuran (28 mL, 84 mmol) was added dropwise. Upon completion of the addition, the obtained solution was warmed to 0°C and stirred for 1 hour, and ice water (100 mL) was added. The reaction mixture was extracted with ethyl acetate (50 mL×3). The organic phase was washed with saturated brine, dried over anhydrous sodium sulfate, and concentrated to obtain a crude product. The crude product was purified by a flash silica gel column (ethyl acetate/petroleum ether: 0-40%) to obtain 2-(8-bromonaphthalen-1-yl)ethan-1-ol (2 g, yield: 56.9%) as a yellow solid. ES-API: [M+Na]⁺=273.1.

Step 2: 2-(8-bromonaphthalen-1-yl) ethan-1-ol (1.4 g, 5.75 mmol) and dichloromethane (30 mL) were added to a round-bottom flask, and the reaction system was cooled to 0°C in a nitrogen atmosphere. Dess-Martin periodinane (3.55 g, 8.36 mmol) was added to the above-mentioned reaction solution with stirring. The reaction solution was stirred at 0°C for 1 hour. Saturated sodium thiosulfate solution (50 mL) and saturated aqueous sodium dicarbonate solution (50 mL) were added to the above-mentioned reaction solution and stirred at room temperature for 5 minutes. The reaction mixture was extracted with ethyl acetate (100 mL×3). The organic phase was washed with saturated brine, dried over anhydrous sodium sulfate, and concentrated. The crude product was purified by a flash silica gel column (ethyl acetate/petroleum ether: 0-20%) to obtain 2-(8-bromonaphthalen-1-yl)acetaldehyde (1.1 g, yield: 79.2%) as a yellow solid. ES-API: [M+H]⁺=249.1.

Step 3: 2-(8-bromonaphthalen-1-yl)acetaldehyde (1.8 g, 7.23 mmol) and tetrahydrofuran (40 mL) were added to a reaction flask in a nitrogen atmosphere, and the reaction system was cooled to -30°C. A 3*M* solution of methylmagnesium bromide in tetrahydrofuran (2.65 mL, 7.95 mmol) was added dropwise to the above-mentioned reaction solution with stirring. The reaction solution was stirred at -20°C for 30 minutes. The ice-water bath was removed, and saturated aqueous ammonium chloride (50 mL) was added to the reaction solution. The obtained mixture was extracted with ethyl acetate (50 mL×3). The organic phase was washed with saturated brine, dried over anhydrous sodium sulfate, and concentrated. The crude product was purified by a flash silica gel column (ethyl acetate/petroleum ether: 0-30%) to obtain 1-(8-bromonaphthalen-1-yl) propan-2-ol (1.4 g, yield: 73%). ES-API: [M+H]⁺=287.0.

Step 4: A solution of 1-(8-bromonaphthalen-1-yl)propan-2-ol (1.4 g, 5.28 mmol) and imidazole (0.719 g, 10.56 mmol) in anhydrous N,N-dimethylformamide (15 mL) was added to a reaction flask followed by tert-butyldimethylsilyl chloride (1.03 g, 6.86 mmol), and stirred at room temperature for 2 hours. Saturated aqueous ammonium chloride (100 mL) was added to the reaction solution, and the obtained mixture was extracted with ethyl acetate (50 mL×2). The organic phase was washed with saturated brine, dried over anhydrous sodium sulfate, and concentrated. The crude product was purified by a flash silica gel column (ethyl acetate/petroleum ether: 0-15%) to obtain ((1-(8-bromonaphthalen-1-yl)propan-2-yl)oxy)(tert-butyl)dimethylsilane (1.9 g, yield: 94.8%). ES-API: [2M+Na]⁺=779.5.

Step 5: ((1-(8-bromonaphthalen-1-yl)propan-2-yl)oxy)(tert-butyl)dimethylsilane (1.8 g, 4.74 mmol), bis(pinacolato)diboron (2.41 g, 9.49 mmol), [1,1'-bis(diphenylphosphino)ferrocene] dichloropalladium (347 mg, 0.47 mmol), potassium acetate (1.40 g, 14.23 mmol) and 1,4-dioxane (30 mL) were added to a round-bottom flask. The reaction system was heated and stirred in an oil bath at 95°C for 12 hours in a nitrogen atmosphere. Water (50 mL) was added to the reaction solution, and the obtained mixture was extracted with ethyl acetate (30 mL×3). The organic phase was dried over anhydrous sodium sulfate and concentrated. The crude product was purified by a flash silica gel column (ethyl acetate/petroleum ether: 0-10%) to obtain tert-butyldimethyl ((1-(8-(4,4,5,5-tetramethyl-1,3,2-dioxaborolan-2-yl)naphthalen-1-yl)propan-2-yl)oxy)silan e (1.3 g, crude product) as a yellow oil. ES-API: [M+Na]⁺=449.4.

Step 6: tert-butyldimethyl ((1-(8-(4,4,5,5-tetramethyl-1,3,2-dioxaborolan-2-yl)naphthalen-1-yl) propan-2-yl)oxy)silane (260 mg, 0.43 mmol), tert-butyl (5aS,6S,9R)-2-chloro-1-fluoro-12-(methylthio)-5a,6,7,8,9,10-hexahydro-5H-4-oxido-3,10a,11,13,14-pentaaza-6,9-methanonaphthalene[1,8-ab]heptene-14-carboxylate (80 mg, 0.17 mmol), [n-butyl di(1-adamantyl) phosphino](2-amino-1,1'-biphenyl-2-yl)palladium (II) methanesulfonate (12.4 mg, 0.017 mmol), potassium phosphate (109 mg, 0.51 mmol), tetrahydrofuran (5 mL) and water (1 mL) were added to a round-bottom flask. The reaction system was heated in a microwave reactor at 80°C for 40 minutes in a nitrogen atmosphere. Water (30 mL) was added to the reaction solution, and the obtained mixture was extracted with ethyl acetate (30 mL×3). The organic phase was dried over anhydrous sodium sulfate and concentrated. The crude product was purified by a flash silica gel column (ethyl acetate/petroleum ether: 0-30%) to obtain tert-butyl (SaS,6S,9R)-2-(8-(2-((tert-butyldimethylsilyl)oxy)propyl)naphthalen-1-yl)-1-fluoro-12-(m ethylthio)-5a,6,7,8,9,10-hexahydro-5H-4-oxo-3,10a,11,13,14-pentaaza-6,9-methanonaphth o[1,8-ab]heptene-14-carboxylate (100 mg, yield: 79.9%) which was yellow solid. ES-API: [M+H]⁺=732.3.

Step 7: tert-butyl (5aS,6S,9R)-2-(8-(2-((tert-butyldimethylsilyl)oxy)propyl) naphthalen-1-yl)-1-fluoro-12-(methylthio)-5a,6,7,8,9,10-hexahydro-5H-4-oxo-3,10a,11,13 ,14-pentaaza-6,9-methanonaphtho[1,8-ab]heptene-14-carboxylate (100 mg, 0.14 mmol) and dichloromethane (8 mL) were added to a round-bottom flask. The reaction system was cooled to 0°C, and m-chloroperoxybenzoic acid (36 mg, 0.20 mmol) was added to the above-mentioned reaction solution. The solution was stirred at 0°C for 1 hour. Water (30 mL) was added to the reaction solution, and the obtained mixture was extracted with ethyl acetate (30 mL×3). The organic phase was dried over anhydrous sodium sulfate and concentrated. The crude product was purified by a flash silica gel column (ethyl acetate/petroleum ether: 0-100%) to obtain tert-butyl (SaS,6S,9R)-2-(8-(2-((tert-butyldimethylsilyl)oxy)propyl)naphthalen-1-yl)-1-fluoro-12-(m ethylsulfoxide)-5a,6,7,8,9, 10-hexahydro-5H-4-oxo-3, 10a, 11, 13,14-pentaaza-6,9-methanon aphtho[1,8-ab]heptene-14-carboxylate (100 mg, yield: 97.8%) as a yellow solid. ES-API: [M+H]⁺=748.3.

Step 8: Sodium hydride (16 mg, 0.40 mmol) and tetrahydrofuran (5 mL) were added to a round-bottom flask. The reaction system was cooled to 0°C and ((2R,7aS)-2-fluorotetrahydro-1H-pyrrolizin-7a(5H)-yl)methanol (64 mg, 0.40 mmol) was added to the above-mentioned reaction. The reaction solution was stirred at 0°C for 10 minutes, and a solution of tert-butyl (5aS,6S,9R)-2-(8-(2-((tert-butyldimethylsilyl)oxy)propyl) naphthalen-1-yl)-1-fluoro-12-(methylsulfoxide)-5a,6,7,8,9,10-hexahydro-5H-4-oxo-3,10a, 11,13,14-pentaaza-6,9-methanonaphtho[1,8-ab]heptene-14-carboxylate (100 mg, 0.13 mmol) in tetrahydrofuran (2 mL) was added dropwise. Upon completion of the addition, the solution was stirred at 0°C for 30 minutes. Water (30 mL) was added to the reaction solution, and the obtained mixture was extracted with ethyl acetate (30 mL×3). The organic phase was dried over anhydrous sodium sulfate and concentrated. The crude product was purified by a preparative chromatographic plate (ethyl acetate) to obtain tert-butyl (5aS,6S,9R)-2-(8-(2-((tert-butyldimethylsilyl)oxy)propyl)naphthalen-1-yl)-1-fluoro-12-((2R,7aS)-2-fluorotetra hydro-1H-pyrrolizin-7a(5H)-yl)methoxy)-5a,6,7,8,9,10-hexahydro-5H-4-oxy-3,10a,11,13, 14-pentaaza-6,9-methanonaphtho[1,8-ab]heptene-14-carboxylate (20 mg, yield: 17.7%), ES-API: [M+H]⁺=843.3.

Step 9: tert-butyl (SaS,6S,9R)-2-(8-(2-((tert-butyldimethylsilyl)oxy)propyl)naphthalen-1-yl)-1-fluoro-12-((2R,7aS)-2-fluorotetrahydro-1H-pyrrolizin-7a(5H)-yl)methoxy)-5a,6,7,8,9,10-hexahydro-5H-4-oxy-3,10a,11,13,14-pentaaza-6,9-methanonaphtho[1,8-ab]heptene-14-car boxylate (20 mg, 0.024 mmol), tetrabutylammonium fluoride (1 mL, 1 mmol, 1M tetrahydrofuran solution) and tetrahydrofuran (1 mL) were added to a round-bottom flask. The solution was stirred at room temperature for 24 hours. Water (30 mL) was added to the reaction solution, and the obtained mixture was extracted with ethyl acetate (30 mL×2). The organic phase was washed for 3 times with saturated brine, dried over anhydrous sodium sulfate, and concentrated to obtain tert-butyl (5aS,6S,9R)-1-fluoro-12-((2R,7aS)-2-fluorotetrahydro-1H-pyrrolizin-7a(5H)-ylmethoxy)-2-(8-(2-hydroxypropyl)naphthalen-1-yl)-5a,6,7,8,9,10-hexahydro-5H-4-oxo-3,10a,11,13,1 4-pentaaza-6,9-methanonaphtho[1,8-ab]heptene-14-carboxylate (16 mg, crude product), which was used directly in the next reaction. ES-API: [M+H]⁺=729.3.

Step 10: tert-butyl (SaS,6S,9R)-1-fluoro-12-((2R,7aS)-2-fluorotetrahydro-1H-pyrrolizin-7a(5H)-ylmethoxy)-2-(8-(2-hydroxypropyl)naphthalen-1-yl)-5a,6,7,8,9,10-hexahydro-5H-4-oxo-3,10a,11,13,14-pentaaza-6,9-methanonaphtho[1,8-ab]heptene-14-carboxylate (16 mg, 0.022 mmol) and acetonitrile (1 mL) were added to a round-bottom flask. 4*M* of Hydrogen chloride/dioxane solution (1 mL) was added to the mixture. The reaction solution was stirred at room temperature for 1 hour. The reaction solution was concentrated to dryness. The crude product was purified by high-performance liquid chromatography (ammonium bicarbonate method) to obtain 1-(8-((SaS,6S,9R)-1-fluoro-12-(((2R,7aS)-2-fluorotetrahydro-1H-pyrrolizin-7a(SH)-yl)me thoxy)-5a,6,7,8,9,10-hexahydro-5H-4-oxa-3,10a,11,13,14-pentaaza-6,9-methanonaphtho[1 ,8-ab]heptalen-2-yl)naphthalen-1-yl)propan-2-ol (Z385, 6 mg, yield: 43.4%) which was white solid. ES-API:[M+H]⁺=629.3.

The compounds of Table A, Table B or Table C, respectively, were prepared by modifying some of the starting materials with reference to the preparation methods of the foregoing examples.

**Table A**

| Structure | MS [M+H]⁺ | Structure | MS [M+H]⁺ |
|---|---|---|---|
| 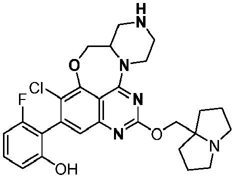 | 526.2 | 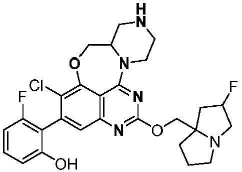 | 544.2 |
| 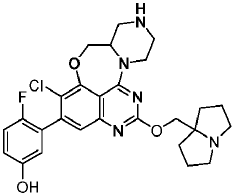 | 526.2 | 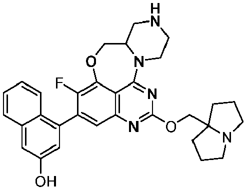 | 542.2 |
| 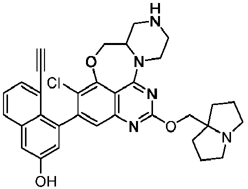 | 582.2 | 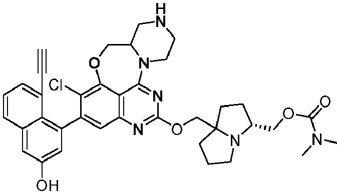 | 683.2 |
| 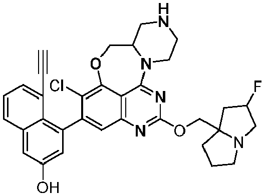 | 600.2 | 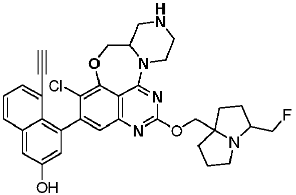 | 614.2 |
| 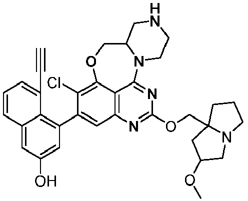 | 612.2 | 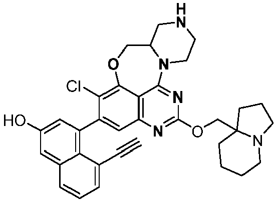 | 596.2 |
| 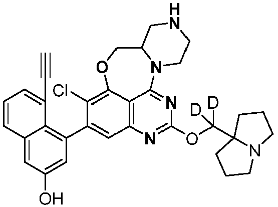 | 583.2 | 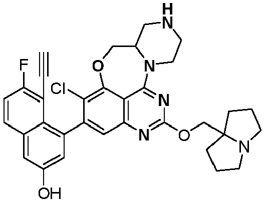 | 600.2 |
| 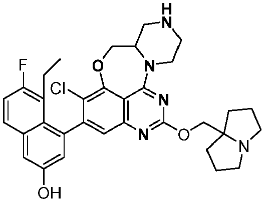 | 604.2 | 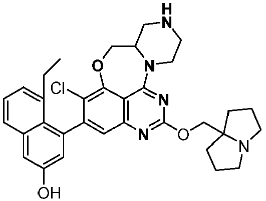 | 586.2 |
| 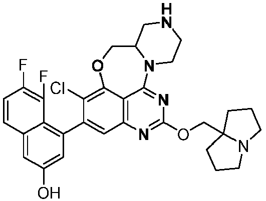 | 594.2 | 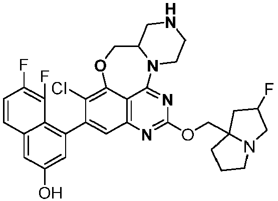 | 612.2 |
| 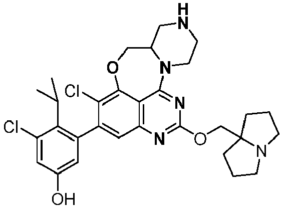 | 584.2 | 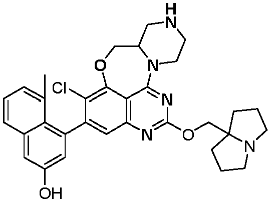 | 572.2 |
| 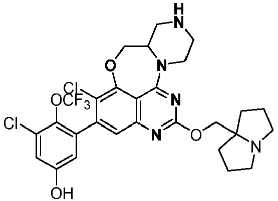 | 266.2 | 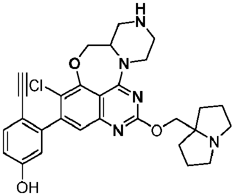 | 531.2 |
| 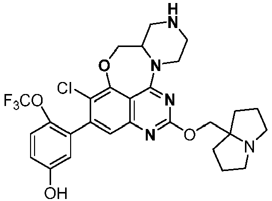 | 591.2 | 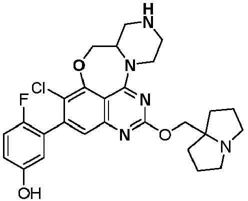 | 526.2 |
| 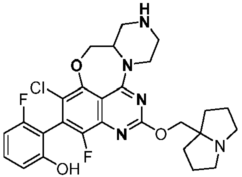 | 544.3 | 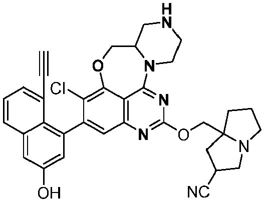 | 607.1 |
| 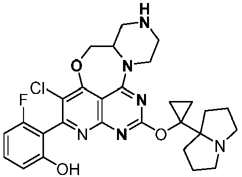 | 553.2 | 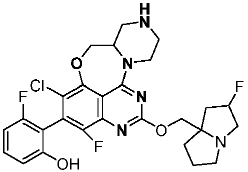 | 562.2 |
| 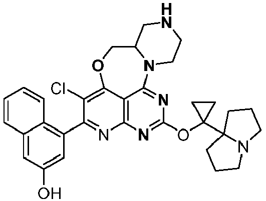 | 585.3 | 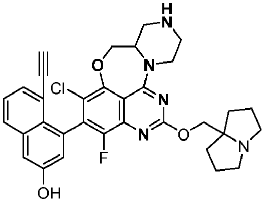 | 600.1 |
| 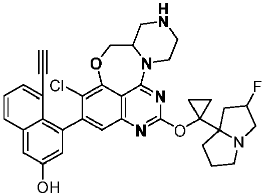 | 626.3 | 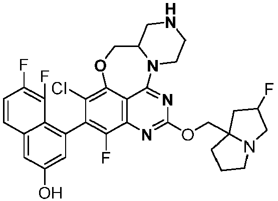 | 630.2 |
| 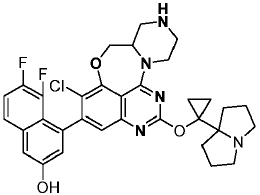 | 620.3 | 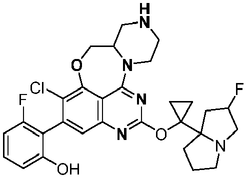 | 570.2 |
| 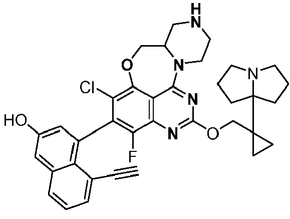 | 640.2 | 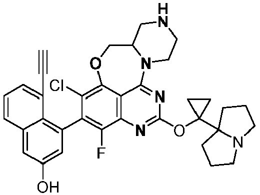 | 626.3 |
| 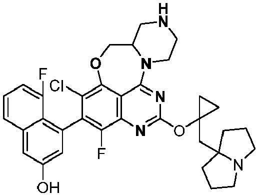 | 634.2 | 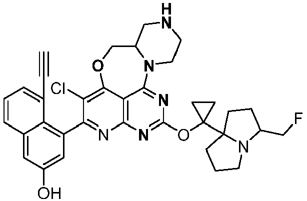 | 641.2 |
| 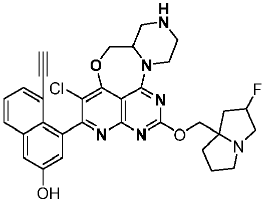 | 614.2 | 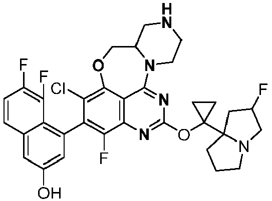 | 656.2 |
| 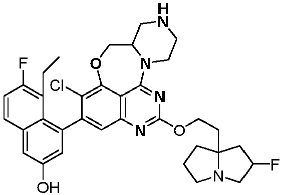 | 636.2 | 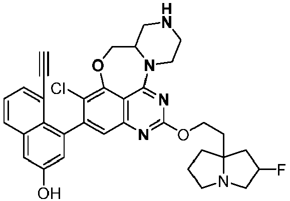 | 614.2 |
| 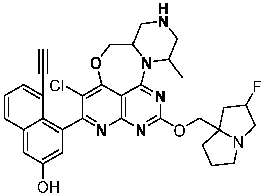 | 614.2 | 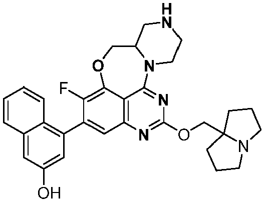 | 542.2 |

**Table B**

| Structure | MS [M+H]⁺ | Structure | MS [M+H]⁺ |
|---|---|---|---|
| 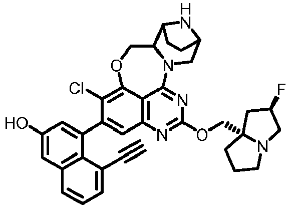 | 626.2 | 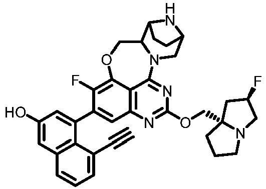 | 610.3 |
| 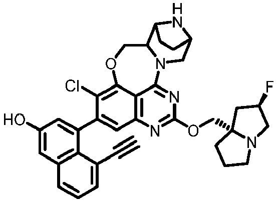 | 626.2 | 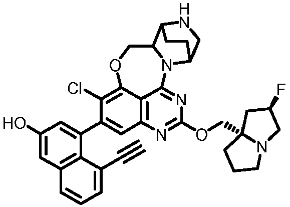 | 626.2 |
| 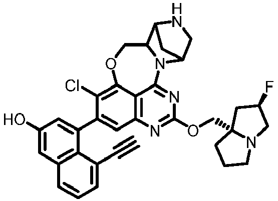 | 612.2 | 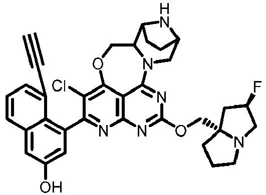 | 627.2 |
| 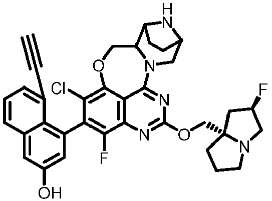 | 644.2 | 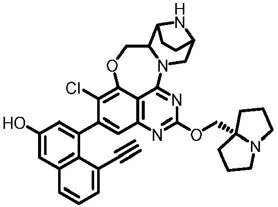 | 608.2 |
| 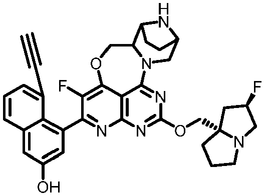 | 611.3 | 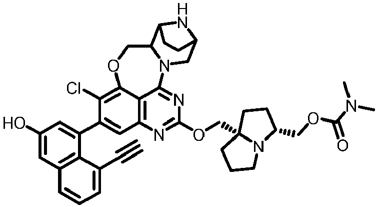 | 709.3 |
| 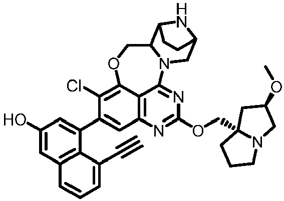 | 638.2 | 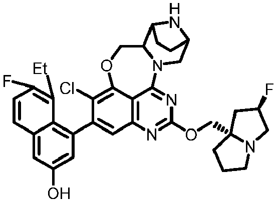 | 648.3 |
| 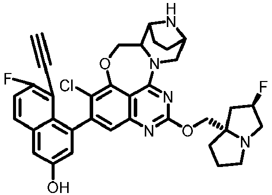 | 644.2 | 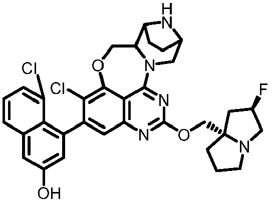 | 636.2 |
| 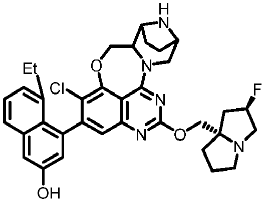 | 630.3 | 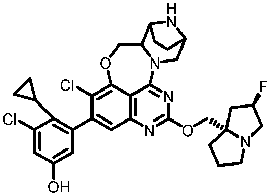 | 626.2 |
| 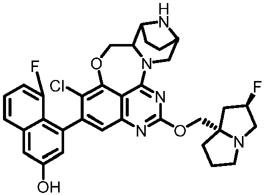 | 620.2 | 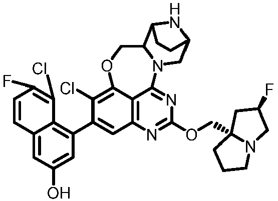 | 654.2 |
| 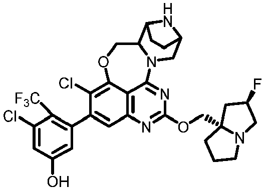 | 654.2 | 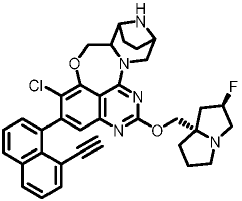 | 610.2 |
| 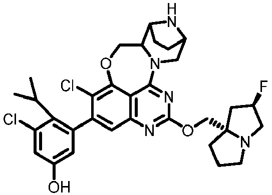 | 628.2 | 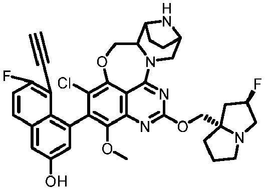 | 674.2 |
| 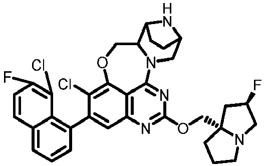 | 638.2 | 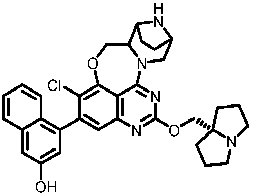 | 584.2 |
| 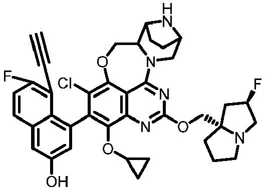 | 700.2 | 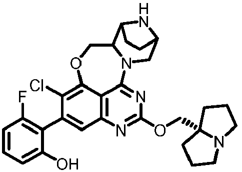 | 552.2 |
| 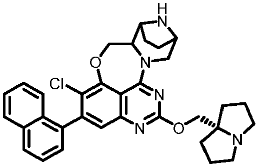 | 568.2 | 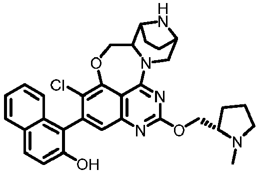 | 558.2 |
| 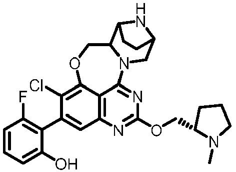 | 526.2 | 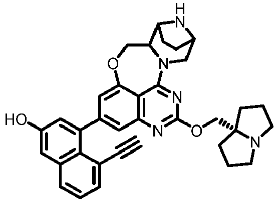 | 574.3 |

**Table C**

| Structure | MS [M+H]⁺ | Structure | MS [M+H]⁺ |
|---|---|---|---|
| 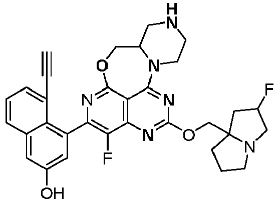 | 585.3 | 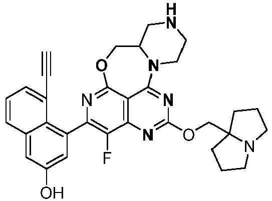 | 567.2 |
| 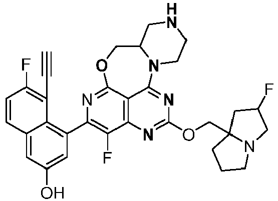 | 603.3 | 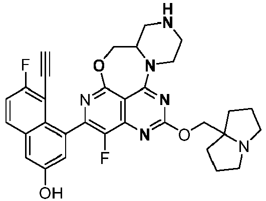 | 585.3 |
| 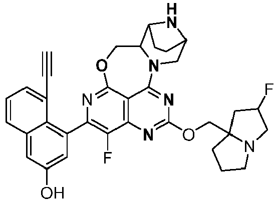 | 611.3 | 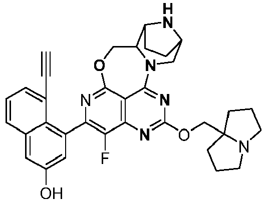 | 593.3 |
| 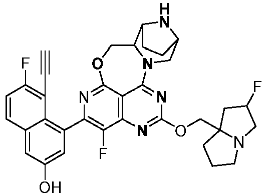 | 629.3 | 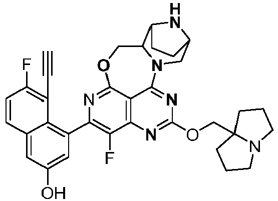 | 611.3 |
| 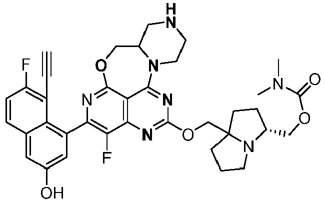 | 686.3 | 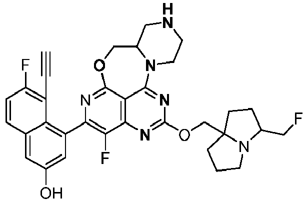 | 617.2 |
| 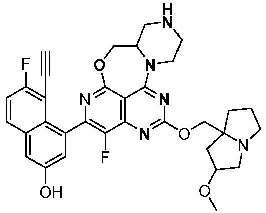 | 615.2 | 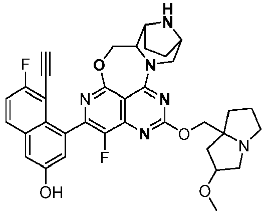 | 641.1 |
| 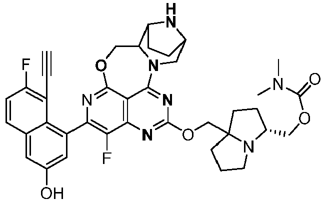 | 711.2 | 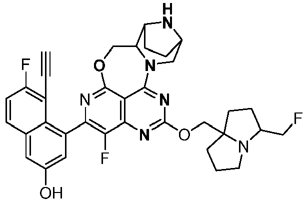 | 643.2 |
| 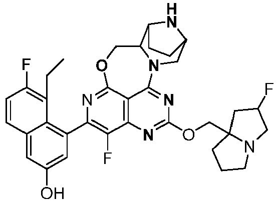 | 633.2 | 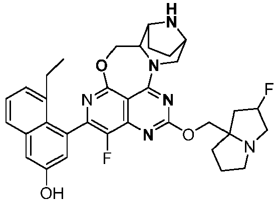 | 615.3 |
| 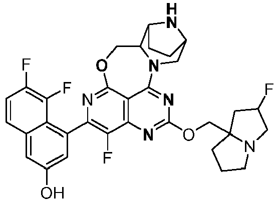 | 622.3 | 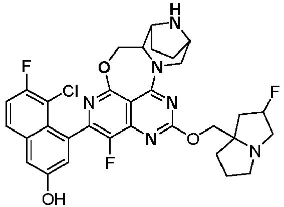 | 638.2 |
| 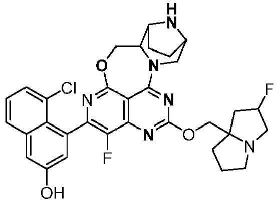 | 620.2 | 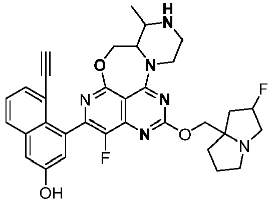 | 599.1 |
| 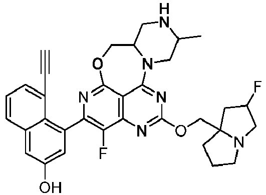 | 599.2 | 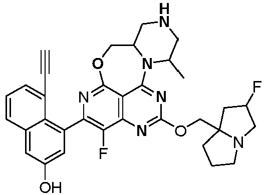 | 599.3 |
| 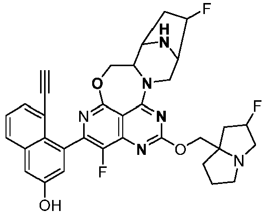 | 629.2 | 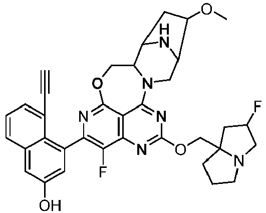 | 641.1 |
| 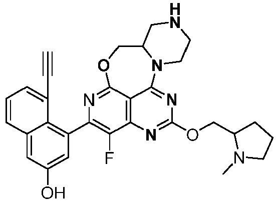 | 541.1 | 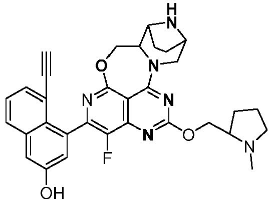 | 567.2 |
| 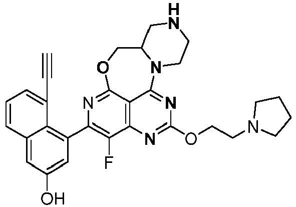 | 541.2 | 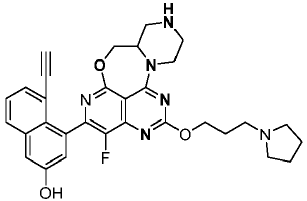 | 555.3 |
| 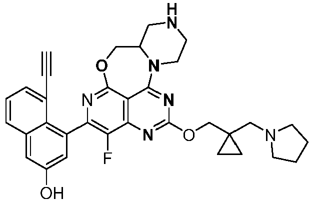 | 581.3 | 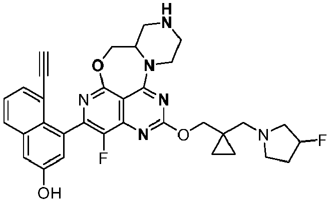 | 599.2 |
| 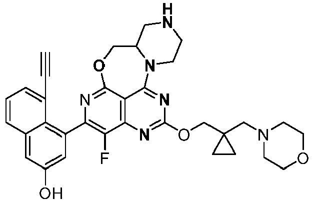 | 597.2 | 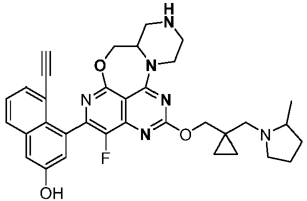 | 595.2 |
| 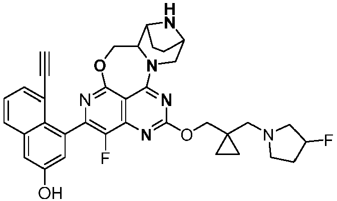 | 625.2 | 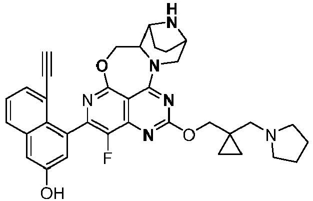 | 607.2 |
| 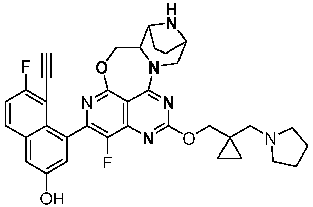 | 624.3 | 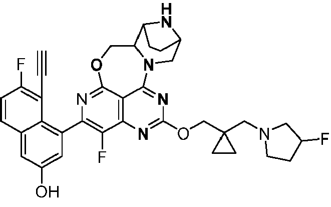 | 643.2 |
| 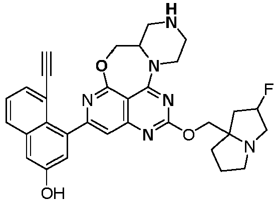 | 567.2 | 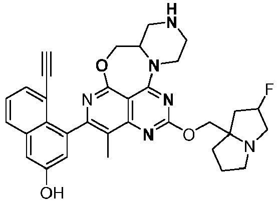 | 581.3 |
| 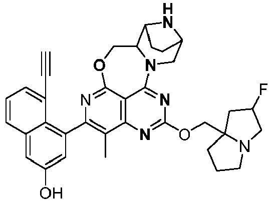 | 607.2 | 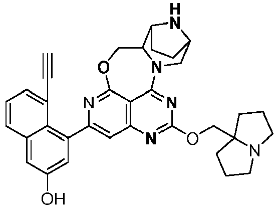 | 575.3 |
| 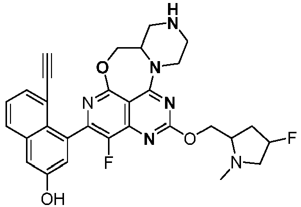 | 559.3 | 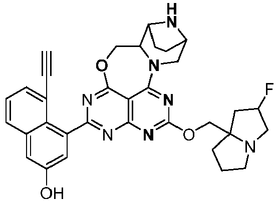 | 594.2 |
| 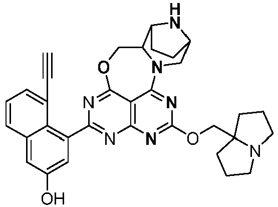 | 575.3 | 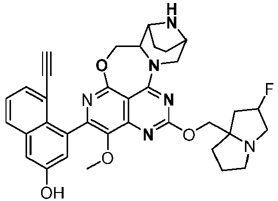 | 623.2 |
| 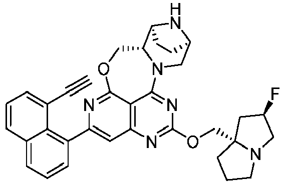 | 577.3 | 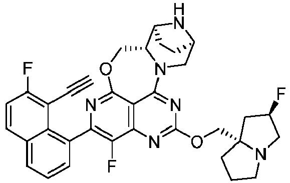 | 613.2 |
| 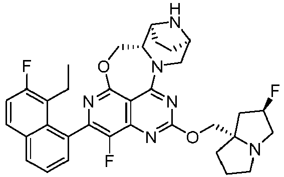 | 617.3 | 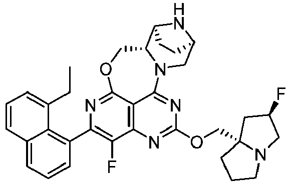 | 599.3 |
| 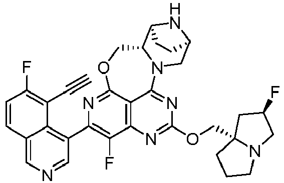 | 614.2 | | |

### Test Example 1: Cell p-ERK Assay

AGS was purchased from ATCC; catalog No: CRL-1739; F12K was purchased from Gibco; catalog No: 21127-022; FBS was purchased from Gibco; catalog No: 10099-141C; trypsin (containing EDTA) was purchased from Gibco; catalog No: 25200-072; DMSO was purchased from VWRAMRESCO; catalog No: 0231-500ML; 96-well cell culture plates were purchased from Coming; catalog No: 3599; opaque 96-well plates with a white background were purchased from Cisbio; catalog No: 66PL96025; Advance ERK phospho-T202/Y204 kit was purchased from Cisbio; catalog No: 64AERPEH; cell counters were purchased from CHEMOMETEC; model No: NC-200; microplate reader was purchased from PerkinElmer; model No: Envison.

AGS is an endogenous human gastric adenocarcinoma cell line containing the KRAS^{G12D} mutation, cultured in F12K+10% FBS medium. On the first day of the experiment, cells in the logarithmic growth phase were taken, digested with trypsin (containing EDTA), collected, and counted. Approximately 20000 cells/well were seeded in a 96-well cell culture plate and cultured overnight in an incubator with 5% CO₂. 1000X stock solutions of compounds at a 3.16-fold concentration gradient were prepared using DMSO, and diluted 200-fold into a 5X compound stock solution using the medium described above. The 5X compound stock solution was added to each cell culturing well the day after cell seeding at a final concentration of 1X and a DMSO content of 0.1%. DMSO was used as an experimental control. After the compound was added and the incubation was kept for 3 hours, the medium in the culture wells was removed. 50 µl of Cell lysis solution (from Advance ERK phospho-T202/Y204 kit, the same below) was added to each well, mixed well, and incubated for 30 minutes and then 16 µl of the mixed solution was transferred to an opaque 96-well plate with a white background. In addition, 16 µl of cell lysis solution was added to a well as a blank well. After transferring, 4 µl of p-ERK HTRF antibody mixture (from Advance ERK phospho-T202/Y204 kit) was added to each well. After incubation for 4 hours, HTRF fluorescence value (RLU) was read by a microplate reader, and p-ERK level inhibition rate (IR(%)=(RLU control-RLU compound)/(RLU control-RLU blank)×100%) of the compounds was calculated. The concentration of the compounds and corresponding cell p-ERK level inhibition rate were fitted using Prism 8 four-parameters method, and the IC₅₀ value was calculated. The results showed that the compounds of the present disclosure have high inhibitory activity on p-ERK. Some compounds inhibit with an IC₅₀ of less than 1 µM, or some compounds have an IC₅₀ of less than 500 nM, even less than 100 nM or 50 nM. The results of some exemplary compounds are shown in Table 1.

**Table 1**

| Number | AGS p-ERK IC₅₀ (nM) | Number | AGS p-ERK IC₅₀ (nM) |
|---|---|---|---|
| Z2 | 10.65 | Z131-1 | 1.58 |
| Z140-1 | 2.85 | Z350 | 46.98 |
| Z381 | 435.88 | | |

### Test Example 2: Cell Proliferation Inhibition Assay

AsPC-1 was purchased from ATCC; catalog No: CRL-1682; AGS was purchased from ATCC; catalog No: CRL-1739; RPMI1640 was purchased from Gibco; catalog No: 11875-093 2235123; F12K was purchased from Gibco; catalog No: 21127-022; FBS was purchased from Gibco; catalog No: 10099-141C; trypsin (containing EDTA) was purchased from Gibco; catalog No: 25200-072; CellTiter Glo-3D was purchased from Progema; catalog No: G9683; 384-well sphere plates were purchased from Coming; catalog No: 3830; opaque 384-well plates with a white background were purchased from Corning; catalog No: 3570; cell counters were purchased from CHEMOMETEC; model: NC-200; microplate reader was purchased from PerkinElmer; model: Envison;

AsPC-1 is an endogenous human pancreatic cancer cell containing KRAS*^{G12D}* mutation, cultured in RPMI-1640 medium containing 10% FBS; AGS is a human gastric cancer cell line containing KRAS*^{G12D}*, cultured in F-12K medium containing 10% FBS. Cells in the logarithmic growth phase were taken, digested with trypsin (containing EDTA), collected, and counted. 800 AsPC-1 cells/well or 400 AGS cells/well, respectively, were seeded in a 384-well sphere plate and cultured overnight at 5% CO₂ to establish a 3D cell model. 1000X stock solutions of compounds at a 3.16-fold concentration gradient of were prepared using DMSO, diluted 100-fold into a 10X compound stock solution using the medium described above. The 10X compound stock solution was added to each cell on 2nd day after cell seeding at a final concentration of 1X and a DMSO content of 0.1%. DMSO was used as an experimental control, and the medium was used as blank. After the addition of the compounds, the cells were incubated for 5 days. 30 µl of CellTiter-Glo work solution was added to each well, mixed well, and incubated for 30 minutes. After standing at room temperature for 30 minutes, 40 µl of the mixed solution was transferred to an opaque 384-well plate with a white clear bottom. Luminescence chemiluminescence value was read by a microplate reader, and the cell proliferation inhibition rate (IR(%)=(RLU control-RLU compound)/(RLU control-RLU blank)×100%) was calculated. The gradiently diluted concentration of the compounds and corresponding cell proliferation inhibition rate were fitted using XLFit four-parameters method, and the IC₅₀ value was calculated. The results showed that the compounds of the present disclosure have high inhibitory activity on cells having KRAS*^{G12D}* mutantion. Some compounds have an IC₅₀ of less than 1000 nM; or an IC₅₀ of less than 500 nM; and even less than 100 nM or 50 nM. The results of some exemplary compounds are shown in Table 2.

**Table 2**

| Number | AGS IC₅₀ (nM) | AsPC-1 IC₅₀ (nM) |
|---|---|---|
| Z2 | 10.35 | 127.31 |
| Z131-1 | 2.53 | 32.80 |
| Z140-1 | 4.00 | 21.11 |
| Z350 | 181.24 | 202.81 |

While particular embodiments of the present disclosure have been described in detail, it will be apparent to those skilled in the art that various modifications and alternatives to those details could be made in light of the teachings of the present disclosure, and shall fall in the protection scope of the present disclosure. The full scope of the present disclosure is given by the appended claims and any equivalents thereof.

## Claims

1. A compound, or a pharmaceutically acceptable salt, a stereoisomer, a solvate or a prodrug thereof, wherein,
the compound is a compound shown in formula (I): 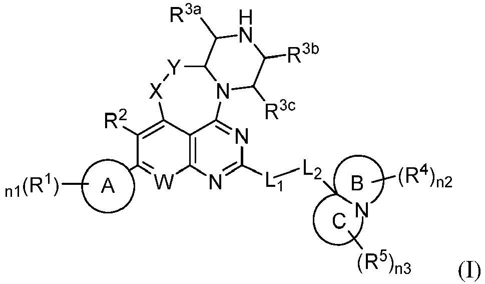 in the formula (I),
X is O or NR¹¹; where R¹¹ is selected from H and C1-C6 alkyl;
Y is CR¹²R¹³, CR¹⁴R¹⁵CR¹⁶R¹⁷, C(O) or C(O)CR¹⁸R¹⁹, where R¹², R¹³, R¹⁴ and R¹⁵ are each independently selected from H, C1-C3 alkyl, C1-C3 deuterated alkyl, and C1-C3 halogenated alkyl; R¹⁶, R¹⁷, R¹⁸, R¹⁹ are each independently selected from H, C1-C3 alkyl and halogen;
W is N or CR²⁰; where R²⁰ is H, C1-C6 alkyl, halogen, C1-C6 halogenated alkyl, C1-C6 alkoxyl, cycloalkyl, cycloalkyl-O-, heterocyclyl or heterocyclyl-O-; where the cycloalkyl, the heterocyclyl are each independently optionally substituted with halogen;
ring A is selected from a group consisting of: aryl, heteroaryl;
R¹ is a substituent at any position on ring A, each R¹ is independently selected from: C1-C6 alkyl, halogen, hydroxyl, C1-C6 alkoxyl, C1-C6 halogenated alkyl, C1-C6 halogenated alkoxyl, cyano, NR²¹R²², C(O)NR²³R²⁴, CH₂R²⁵, N=S(O) (C1-C6 alkyl)₂, S (O) C1-C6 alkyl, S (O)₂R²⁶, -S-C1-C6 alkyl, C2-C6 alkenyl, C2-C6 alkynyl, C2-C6 hydroxyalkynyl, C1-C6 cyanoalkyl, triazolyl, -S-C1-C6 halogenated alkyl, C1-C6 hydroxyalkyl, -CH₂C(O)NR²⁷R²⁸, -C2-C6 alkynyl NR²⁹R³⁰, C2-C6 deuterated alkynyl, (C1-C6 alkoxyl) C1-C6 halogenated alkyl- or cycloalkyl; where the cycloalkyl is optionally substituted with halogen or C1-C6 alkyl, where,
R²¹ is H, C1-C6 alkyl, C1-C6 halogenated alkyl, C(O)C1-C6 alkyl or C(O)₂C1-C6 alkyl; R²² is H, C1-C6 alkyl or C1-C6 halogenated alkyl; or R²¹ and R²², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²³, R²⁴ are each independently H, C1-C6 alkyl or C1-C6 halogenated alkyl; or R²³ and R²⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C6 alkyl; where R²⁵¹, R²⁵², R²⁵³, R²⁵⁴ are each independently H or C1-C6 alkyl; R²⁵¹ and R²⁵², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy; R²⁵³ and R²⁵⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁶ is C1-C6 alkyl, C1-C6 halogenated alkyl or NR²⁶¹R²⁶², where R²⁶¹, R²⁶² are each independently H or C1-C6 alkyl; or R²⁶¹ and R²⁶², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁷, R²⁸ are each independently H, C1-C6 alkyl or C1-C6 halogenated alkyl; or R²⁷ and R²⁸, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁹, R³⁰ are each independently H, C1-C6 alkyl or C1-C6 halogenated alkyl; or R²⁹ and R³⁰, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
n1 is 0, 1, 2 or 3;
R² is H, cyano, halogen, C1-C6 alkyl, C1-C6 alkoxyl, C1-C6 halogenated alkyl, C1-C6 halogenated alkoxyl, C1-C6 deuterated alkoxyl, NR³¹R³², C2-C4 alkynyl or CH₂OR³³; where R³¹, R³², R³³ are each independently hydrogen or C1-C6 alkyl;
R^{3a}, R^{3b}, R^{3c} are each independently hydrogen, halogen, hydroxyl, cyano, C1-C3 alkyl, C1-C3 deuterated alkyl or C1-C3 halogenated alkyl;
L₁ is a bond, O or NR³⁴;
L₂ is C1-C4 alkylene or heteroaryl, where one or two hydrogen atoms on any carbon atom or on any same carbon atom of the C1-C4 alkylene are independently and optionally substituted with deuterium, C1-C6 alkyl, or C1-C6 halogenated alkyl; or two hydrogen atoms on any same carbon atom of the C1-C4 alkylene are optionally simultaneously substituted with -(CH₂)ₘ₃-, -(CH₂)ₘ₁-O-(CH₂)ₘ₂-, -(CH₂)ₘ₁-N-(CH₂)ₘ₂- to form a cyclic substituent; m3 is 1 or 2; each m1 is respectively and independently 0, 1, 2, or 3; each m2 is respectively and independently 0, 1, 2, or 3; and m1 and m2 are not 0 semultaneously;
ring B is a 3-to 6-membered nitrogen-containing heterocyclyl;
ring C is a 3-to 6-membered nitrogen-containing heterocyclyl;
R⁴ is a substituent at any position on ring B; n2 is 0, 1, 2 or 3;
R⁵ is a substituent at any position on ring C; n3 is 0, 1, 2 or 3;
R⁴, R⁵ are defined as follows:
(a) R⁴, R⁵ are each independently halogen, hydroxyl, C1-C3 hydroxyalkyl, C1-C3 alkyl, C1-C3 halogenated alkyl, C1-C3 alkoxyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxyl) C1-C3 alkyl, (C1-C3 alkyl)C(O), oxo, (C1-C3 halogenated alkyl)C(O)-, -SO₂F, (C1-C3 alkoxyl) C1-C3 alkoxyl, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl) phenyl, -OC (O) heterocyclyl or -CH₂ heterocyclyl; wherein, the phenyl in -OC(O)NH(C1-C3 alkyl)O(C1-C3 alkyl) phenyl or -NHC (O) phenyl is optionally substituted with -C(O)H or OH; the heterocyclyl in -CH₂ heterocyclyl is optionally substituted with oxo; or
(b) one R⁴ is -CH₂CH₂- or -CH₂CH₂CH₂-, and, the R⁵ together with a carbon atom to which it is attached, forms a cyclopropyl or cyclobutyl; the remaining R⁴, R⁵ are each independently halogen, hydroxyl, C1-C3 hydroxyalkyl, C1-C3 alkyl, C1-C3 halogenated alkyl, C1-C3 alkoxyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxyl) C1-C3 alkyl, (C1-C3 alkyl)C(O), oxo, (C1-C3 halogenated alkyl)C(O)-, -SO₂F, (C1-C3 alkoxyl) C1-C3 alkoxyl, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl) phenyl, -OC (O) heterocyclyl or -CH₂ heterocyclyl; wherein, the phenyl in -OC(O)NH(C1-C3 alkyl)O(C1-C3 alkyl) phenyl or -NHC (O) phenyl is optionally substituted with -C(O)H or OH; the heterocyclyl in -CH₂ heterocyclyl is optionally substituted with oxo; or
(c) one R⁵ is -CH₂CH₂- or -CH₂CH₂CH₂-, and the R⁵ together with a carbon atom to which it is attached, forms a cyclopropyl or cyclobutyl; the remaining R⁵, and R⁴ are each independently halogen, hydroxyl, C1-C3 hydroxyalkyl, C1-C3 alkyl, C1-C3 halogenated alkyl, C1-C3 alkoxyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxyl) C1-C3 alkyl, (C1-C3 alkyl)C(O), oxo, (C1-C3 halogenated alkyl)C(O)-, -SO₂F, (C1-C3 alkoxyl) C1-C3 alkoxyl, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl) phenyl, -OC(O) heterocyclyl or -CH₂ heterocyclyl; wherein, the phenyl in -OC(O)NH(C1-C3 alkyl)O(C1-C3 alkyl) phenyl or -NHC(O)phenyl is optionally substituted with -C(O)H or OH; the heterocyclyl in -CH₂ heterocyclyl is optionally substituted with oxo; or
(d) one R⁴ is -CH₂CH₂- or -CH₂CH₂CH₂-, and the R⁴ together with a carbon atom to which it is attached, forms a cyclopropyl or cyclobutyl; one R⁵ is -CH₂CH₂- or -CH₂CH₂CH₂-, and the R⁵ together with a carbon atom to which it is attached, forms a cyclopropyl or cyclobutyl; the remaining R⁴, the remaining R⁵ are each independently halogen, hydroxyl, C1-C3 hydroxyalkyl, C1-C3 alkyl, C1-C3 halogenated alkyll, C1-C3 alkoxyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxyl) C1-C3 alkyl, (C1-C3 alkyl)C(O), oxo, (C1-C3 halogenated alkyl)C(O)-, -SO₂F, (C1-C3 alkoxyl) C1-C3 alkoxyl, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl) phenyl, -OC (O) heterocyclyl or -CH₂ heterocyclyl; wherein, the phenyl in -OC (O)NH(C1-C3 alkyl)O(C1-C3 alkyl) phenyl or -NHC (O) phenyl is optionally substituted with -C(O)H or OH; the heterocyclyl in -CH₂ heterocyclyl is optionally substituted with oxo;
each R³⁴ is respectively and independently hydrogen, C1-C3 alkyl, or C1-C3 halogenated alkyl; or two R³⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
or
the compound is a compound shown in formula (II-1) or formula (II-2): 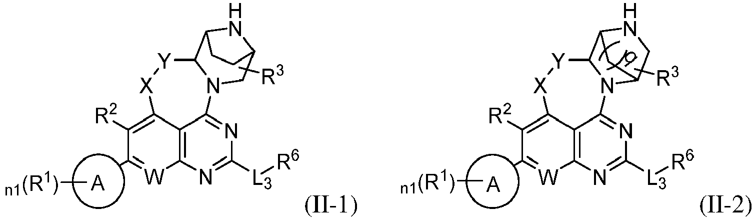 where in formula (II-1) or formula (II-2),
q is 1 or 2;
X is O or NR¹¹; where R¹¹ is selected from H and C1-C6 alkyl;
Y is CR¹²R¹³, CR¹⁴R¹⁵CR¹⁶R¹⁷, C(O) or C(O)CR¹⁸R¹⁹, where R¹², R¹³, R¹⁴ and R¹⁵ are each independently selected from H, C1-C3 alkyl, C1-C3 deuterated alkyl, and C1-C3 halogenated alkyl; R¹⁶, R¹⁷, R¹⁸, R¹⁹ are each independently selected from H, C1-C3 alkyl and halogen;
W is N or CR²⁰; where R²⁰ is H, C1-C6 alkyl, halogen, C1-C6 halogenated alkyl, C1-C6 alkoxyl, cycloalkyl, cycloalkyl-O-, heterocyclyl or heterocyclyl-O-; where the cycloalkyl, the heterocyclyl are each independently optionally substituted with halogen;
ring A is selected from a group consisting of: aryl, heteroaryl;
R¹ is a substituent at any position on ring A, each R¹ is independently selected from: C1-C6 alkyl, halogen, hydroxyl, C1-C6 alkoxyl, C1-C6 halogenated alkyl, C1-C6 halogenated alkoxyl, cyano, NR²¹R²², C(O)NR²³R²⁴, CH₂R²⁵, N=S(O) (C1-C6 alkyl)₂, S (O) C1-C6 alkyl, S (O)₂R²⁶, -S-C1-C6 alkyl, C2-C6 alkenyl, C2-C6 alkynyl, C2-C6 hydroxyalkynyl, C1-C6 cyanoalkyl, triazolyl, -S-C1-C6 halogenated alkyl, C1-C6 hydroxyalkyl, -CH₂C(O)NR²⁷R²⁸, -C2-C6 alkynyl NR²⁹R³⁰, C2-C6 deuterated alkynyl, (C1-C6 alkoxyl) C1-C6 halogenated alkyl- or cycloalkyl; where the cycloalkyl is optionally substituted with halogen or C1-C6 alkyl, where,
R²¹ is H, C1-C6 alkyl, C1-C6 halogenated alkyl, C(O)C1-C6 alkyl or C(O)₂C1-C6 alkyl; R²² is H, C1-C6 alkyl or C1-C6 halogenated alkyl; or R²¹ and R²², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²³, R²⁴ are each independently H, C1-C6 alkyl or C1-C6 halogenated alkyl; or R²³ and R²⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C6 alkyl; where R²⁵¹, R²⁵², R²⁵³, R²⁵⁴ are each independently H or C1-C6 alkyl; R²⁵¹ and R²⁵², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy; R²⁵³ and R²⁵⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁶ is C1-C6 alkyl, C1-C6 halogenated alkyl or NR²⁶¹R²⁶², where R²⁶¹, R²⁶² are each independently H or C1-C6 alkyl; or R²⁶¹ and R²⁶², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁷, R²⁸ are each independently H, C1-C6 alkyl or C1-C6 halogenated alkyl; or R²⁷ and R²⁸,
together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁹, R³⁰ are each independently H, C1-C6 alkyl or C1-C6 halogenated alkyl; or R²⁹ and R³⁰, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
n1 is 0, 1, 2 or 3;
R² is H, cyano, halogen, C1-C6 alkyl, C1-C6 alkoxyl, C1-C6 halogenated alkyl, C1-C6 halogenated alkoxyl, C1-C6 deuterated alkoxyl, NR³¹R³², C2-C4 alkynyl or CH₂OR³³; where R³¹, R³², R³³ are each independently hydrogen or C1-C6 alkyl;
R³ is a substituent at any position on a bridge ring, and is hydrogen, halogen, hydroxyl, cyano, C1-C3 alkyl, C1-C3 deuterated alkyl or C1-C3 halogenated alkyl;
L₃ is a bond, O or NR³⁴;
R⁶ is hydrogen, -N(R³⁴)₂, heterocyclyl, C1-C6 alkyl, -L-heterocyclyl, -L-aryl, -L-heteroaryl, -L-cycloalkyl, -L-N(R³⁴)₂, -L-NHC(=NH)NH₂, -L-C(O)N(R³⁴)₂, -L-C1-C6 halogenated alkyl, -L-OR³⁴, -L-(CH₂OR³⁴)(CH₂)ₙOR³⁴, -L-NR³⁴C(O)-aryl, -L-COOH, or -L-C(O)OC1-C6 alkyl; where each of the heterocyclyl, the aryl in the -L-NR³⁴C(O)-aryl, the heterocyclyl in the -L-heterocyclyl, the cycloalkyl in the -L-cycloalkyl may be optionally substituted with one or more R³⁵, or optionally two hydrogen atoms on the same carbon atom are simultaneously substituted with -CH₂CH₂- or -CH₂CH₂CH₂- as cyclopropyl or cyclobutyl; each of the aryl in the -L-aryl, the heteroaryl in the -L-heteroaryl may be optionally substituted with one or more R³⁶;
where each L is respectively and independently C1-C4 alkylene or heteroaryl, where one or two hydrogen atoms on any carbon atom or on any same carbon atom of the C1-C4 alkylene are independently and optionally substituted with deuterium, C1-C6 alkyl, or C1-C6 halogenated alkyl; or two hydrogen atoms on any same carbon atom of the C1-C4 alkylene are optionally simultaneously substituted with -(CH2)m3-, -(CH2)ml-O-(CH2)m2-, -(CH₂)ₘ₁-N-(CH₂)ₘ₂- to form a cyclic substituent; m3 is 1 or 2; each m1 is respectively and independently 0, 1, 2, or 3; each m2 is respectively and independently 0, 1, 2, or 3; and m1 and m2 are not 0 semultaneously;
each R³⁵ is respectively and independently halogen, hydroxyl, C1-C3 hydroxyalkyl, C1-C3 alkyl, C1-C3 halogenated alkyl, C1-C3 alkoxyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxyl) C1-C3 alkyl, (C1-C3 alkyl)C(O), oxo, (C1-C3 halogenated alkyl)C(O)-, -SO₂F, (C1-C3 alkoxyl) C1-C3 alkoxyl, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl) phenyl, -OC (O) heterocyclyl or -CH₂ heterocyclyl; wherein, the phenyl in -OC (O)NH(C1-C3 alkyl)O(C1-C3 alkyl) phenyl or -NHC (O) phenyl is optionally substituted with -C(O)H or OH; the heterocyclyl in -CH₂ heterocyclyl is optionally substituted with oxo;
each R³⁶ is respectively and independently halogen, hydroxyl, HC (O)-, C1-C4 alkyl, C1-C4 alkoxyl, C1-C4 halogenated alkyl, C1-C4 hydroxyalkyl, or -N(R³⁴)₂;
each R³⁴ is respectively and independently hydrogen, C1-C3 alkyl, or C1-C3 halogenated alkyl; or two R³⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
or
the compound is a compound shown in formula (III): 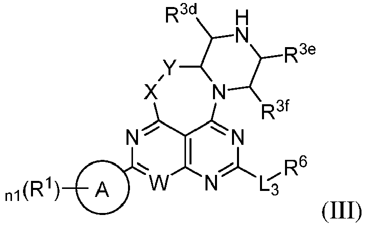 in formula (III),
X is O or NR¹¹; where R¹¹ is selected from H and C1-C6 alkyl;
Y is CR¹²R¹³, CR¹⁴R¹⁵CR¹⁶R¹⁷, C(O) or C(O)CR¹⁸R¹⁹, where R¹², R¹³, R¹⁴ and R¹⁵ are each independently selected from H, C1-C3 alkyl, C1-C3 deuterated alkyl, and C1-C3 halogenated alkyl; R¹⁶, R¹⁷, R¹⁸, R¹⁹ are each independently selected from H, C1-C3 alkyl and halogen;
W is N or CR²⁰; where R²⁰ is H, C1-C6 alkyl, halogen, C1-C6 halogenated alkyl, C1-C6 alkoxyl, cycloalkyl, cycloalkyl-O-, heterocyclyl or heterocyclyl-O-; where the cycloalkyl, the heterocyclyl are each independently optionally substituted with halogen;
ring A is selected from a group consisting of: aryl, heteroaryl;
R¹ is a substituent at any position on ring A, each R¹ is independently selected from: C1-C6 alkyl, halogen, hydroxyl, C1-C6 alkoxyl, C1-C6 halogenated alkyl, C1-C6 halogenated alkoxyl, cyano, NR²¹R²², C(O)NR²³R²⁴, CH₂R²⁵, N=S(O) (C1-C6 alkyl)₂, S (O) C1-C6 alkyl, S (O)₂R²⁶, -S-C1-C6 alkyl, C2-C6 alkenyl, C2-C6 alkynyl, C2-C6 hydroxyalkynyl, C1-C6 cyanoalkyl, triazolyl, -S-C1-C6 halogenated alkyl, C1-C6 hydroxyalkyl, -CH₂C(O)NR²⁷R²⁸, -C2-C6 alkynyl NR²⁹R³⁰, C2-C6 deuterated alkynyl, (C1-C6 alkoxyl) C1-C6 halogenated alkyl- or cycloalkyl; where the cycloalkyl is optionally substituted with halogen or C1-C6 alkyl, where,
R²¹ is H, C1-C6 alkyl, C1-C6 halogenated alkyl, C(O)C1-C6 alkyl or C(O)₂C1-C6 alkyl; R²² is H, C1-C6 alkyl or C1-C6 halogenated alkyl; or R²¹ and R²², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²³, R²⁴ are each independently H, C1-C6 alkyl or C1-C6 halogenated alkyl; or R²³ and R²⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C6 alkyl; where R²⁵¹, R²⁵², R²⁵³, R²⁵⁴ are each independently H or C1-C6 alkyl; R²⁵¹ and R²⁵², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy; R²⁵³ and R²⁵⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁶ is C1-C6 alkyl, C1-C6 halogenated alkyl or NR²⁶¹R²⁶², where R²⁶¹, R²⁶² are each independently H or C1-C6 alkyl; or R²⁶¹ and R²⁶², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁷, R²⁸ are each independently H, C1-C6 alkyl or C1-C6 halogenated alkyl; or R²⁷ and R²⁸, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁹, R³⁰ are each independently H, C1-C6 alkyl or C1-C6 halogenated alkyl; or R²⁹ and R³⁰, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
n1 is 0, 1, 2 or 3;
R ^{3d} , R^{3e}, R^{3f} are each independently hydrogen, halogen, hydroxyl, cyano, C1-C3 alkyl, C1-C3 deuterated alkyl or C1-C3 halogenated alkyl; or
R^{3d} is linked to R^{3e} to form -CHR^{3d1}- or -CHR^{3d2}CHR^{3d3}-; R^{3f} is hydrogen, halogen, hydroxyl, cyano, C1-C3 alkyl, C1-C3 deuterated alkyl or C1-C3 halogenated alkyl; R^{3a1}, R^{3d2}, R^{3d3} are each independently hydrogen, halogen, hydroxyl, cyano, C1-C3 alkyl, C1-C3 deuterated alkyl or C1-C3 halogenated alkyl; or
R^{3d} is linked to R^{3f} to form -CHR^{3f1}- or -CHR^{3f2}CHR^{3f3}-; R^{3e} is hydrogen, halogen, hydroxyl, cyano, C1-C3 alkyl, C1-C3 deuterated alkyl or C1-C3 halogenated alkyl; R^{3f1}, R^{3f2}, R^{3f3} are each independently hydrogen, halogen, hydroxyl, cyano, C1-C3 alkyl, C1-C3 deuterated alkyl or C1-C3 halogenated alkyl;
L₃ is a bond, O or NR³⁴;
R⁶ is hydrogen, -N(R³⁴)₂, heterocyclyl, C1-C6 alkyl, -L-heterocyclyl, -L-aryl, -L-heteroaryl, -L-cycloalkyl, -L-N(R³⁴)₂, -L-NHC(=NH)NH₂, -L-C(O)N(R³⁴)₂, -L-C1-C6 halogenated alkyl, -L-OR³⁴, -L-(CH₂OR³⁴)(CH₂)ₙOR³⁴, -L-NR³⁴C(O)-aryl, -L-COOH, or -L-C(O)OC1-C6 alkyl; where each of the heterocyclyl, the aryl in the -L-NR³⁴C(O)-aryl, the heterocyclyl in the -L-heterocyclyl, the cycloalkyl in the -L-cycloalkyl may be optionally substituted with one or more R³⁵, or optionally two hydrogen atoms on the same carbon atom are simultaneously substituted with -CH₂CH₂- or -CH₂CH₂CH₂- as cyclopropyl or cyclobutyl; each of the aryl in the -L-aryl, the heteroaryl in the -L-heteroaryl may be optionally substituted with one or more R³⁶;
where each L is respectively and independently C1-C4 alkylene or heteroaryl, where one or two hydrogen atoms on any carbon atom or on any same carbon atom of the C1-C4 alkylene are independently and optionally substituted with deuterium, C1-C6 alkyl, or C1-C6 halogenated alkyl; or two hydrogen atoms on any same carbon atom of the C1-C4 alkylene are optionally simultaneously substituted with -(CH₂)ₘ₃-, -(CH₂)ₘ₁-O-(CH₂)ₘ₂-, -(CH₂)ₘ₁-N-(CH₂)ₘ₂- to form a cyclic substituent; m3 is 1 or 2; each m1 is respectively and independently 0, 1, 2, or 3; each m2 is respectively and independently 0, 1, 2, or 3; and m1 and m2 are not 0 semultaneously;
each R³⁵ is respectively and independently halogen, hydroxyl, C1-C3 hydroxyalkyl, C1-C3 alkyl, C1-C3 halogenated alkyl, C1-C3 alkoxyl, cyano, -phenyl, -phenyl-SO₂F, -O-phenyl, -O-phenyl-SO₂F, -NHC(O)phenyl, -NHC(O)phenyl-SO₂F, C1-C3 alkyl substituted pyrazolyl, aryl C1-C3 alkyl-, tert-butyldimethylsilyl CH₂-, -N(R³⁴)₂, (C1-C3 alkoxyl) C1-C3 alkyl, (C1-C3 alkyl)C(O), oxo, (C1-C3 halogenated alkyl)C(O)-, -SO₂F, (C1-C3 alkoxyl) C1-C3 alkoxyl, -CH₂OC(O)N(R³⁴)₂, -CH₂NHC(O)OC1-C6 alkyl, -CH₂NHC(O)N(R³⁴)₂, -CH₂NHC(O)C1-C6 alkyl, -CH₂(pyrazolyl), -CH₂NHSO₂C1-C6 alkyl, -CH₂OC(O) heterocyclyl, -OC(O)N(R³⁴)₂, -OC(O)NH(C1-C3 alkyl)O(C1-C3 alkyl), -OC(O)NH(C1-C3 alkyl)O(C1-C3 alkyl)phenyl(C1-C3 alkyl)N(CH₃)₂, -OC(O)NH(C1-C3 alkyl)O(C1-C3 alkyl) phenyl, -OC (O) heterocyclyl or -CH₂ heterocyclyl; wherein, the phenyl in -OC(O)NH(C1-C3 alkyl)O(C1-C3 alkyl) phenyl or -NHC (O) phenyl is optionally substituted with -C(O)H or OH; the heterocyclyl in -CH₂ heterocyclyl is optionally substituted with oxo;
each R³⁶ is respectively and independently halogen, hydroxyl, HC (O)-, C1-C4 alkyl, C1-C4 alkoxyl, C1-C4 halogenated alkyl, C1-C4 hydroxyalkyl, or -N(R³⁴)₂;
each R³⁴ is respectively and independently hydrogen, C1-C3 alkyl, or C1-C3 halogenated alkyl; or two R³⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy.

2. The compound, or pharmaceutically acceptable salt, stereoisomer, solvate or prodrug thereof according to claim 1, wherein the compound is a compound shown in formula (II-1C1) or formula (II-1C2): 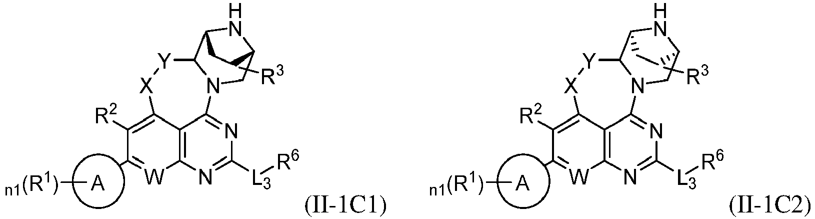 in either formula, X, Y, W, R¹, R², R³, R⁶, n1, L₃, and ring A are each as defined for formula (II-1) or formula (II-2).

3. The compound, or pharmaceutically acceptable salt, stereoisomer, solvate or prodrug thereof according to claim 1, wherein, the compound is a compound shown in formula (III-1c1) or formula (III-1c2): 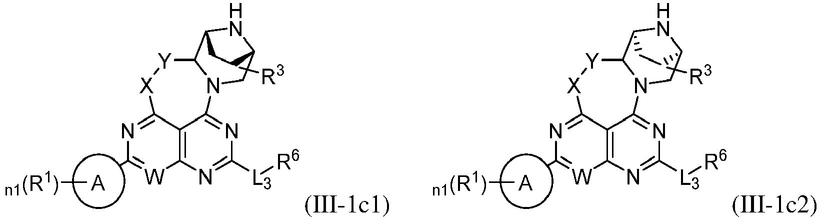 in either formula, R³ is a substituent at any position on a bridge ring, and is hydrogen, halogen, hydroxyl, cyano, C1-C3 alkyl, C1-C3 deuterated alkyl or C1-C3 halogenated alkyl; X, Y, W, ring A, R¹, n1, L₃, R⁶ are as defined for formula (III).

4. The compound, or pharmaceutically acceptable salt, stereoisomer, solvate or prodrug thereof according to claim 1, wherein W is N or CR²⁰; R²⁰ is hydrogen, fluorine, chlorine, methyl, cyclopropyl, methoxyl or cyclopropoxyl.

5. The compound, or pharmaceutically acceptable salt, stereoisomer, solvate or prodrug thereof according to claim 1, wherein X is O.

6. The compound, or pharmaceutically acceptable salt, stereoisomer, solvate or prodrug thereof according to claim 1, wherein Y is -CH₂-.

7. The compound, or pharmaceutically acceptable salt, stereoisomer, solvate or prodrug thereof according to claim 1, wherein R² is fluorine, chlorine, hydroxyl, methoxyl or cyano.

8. The compound, or pharmaceutically acceptable salt, stereoisomer, solvate or prodrug thereof according to claim 1, wherein,
ring A is phenyl, naphthyl, pyridyl, pyrimidinyl, pyrazinyl, pyridazinyl, pyrrolyl, pyrazolyl, imidazolyl, 1,2,3,4-tetrahydronaphthyl, 2,3-dihydro-1H-indenyl, quinolinyl, quinazolinyl, isoquinolinyl, indolyl, indazolyl, or benzo[d][1,3]dioxolane;
and/or
R¹ is a substituent at any position on ring A; n1 is 0, 1, 2 or 3;
each R¹ is respectively and independently selected from: C1-C3 alkyl, halogen, hydroxyl, C1-C3 alkoxyl, C1-C3 halogenated alkyl, C1-C3 halogenated alkoxyl, cyano, NR²¹R²², C(O)NR²³R²⁴, CH₂R²⁵, N=S(O) (C1-C3 alkyl)₂, S (O) C1-C3 alkyl, S (O)₂R²⁶, -S-C1-C3 alkyl, C2-C4 alkenyl, C2-C4 alkynyl, C2-C4 hydroxyalkynyl, C1-C3 cyanoalkyl, triazolyl, -S-C1-C3 halogenated alkyl, C1-C3hydroxyalkyl, -CH₂C(O)NR²⁷R²⁸, -C2-C4 alkynyl NR²⁹R³⁰, C2-C4 deuterated alkynyl, (C1-C3 alkoxyl) C1-C3 halogenated alkyl- or cycloalkyl; where the cycloalkyl is optionally substituted with halogen or C1-C3 alkyl,
where,
R²¹ is H, C1-C3 alkyl, C1-C3 halogenated alkyl, C(O)C1-C3 alkyl or C(O)₂C1-C3 alkyl; R²² is H, C1-C3 alkyl or C1-C3 halogenated alkyl; or R²¹ and R²², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²³, R²⁴ are each independently H, C1-C3 alkyl or C1-C3 halogenated alkyl; or R²³ and R²⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁵ is hydroxyl, cyano, heterocyclyl, NR²⁵¹R²⁵², C(O)NR²⁵³R²⁵⁴ or SO₂C1-C3 alkyl; where R²⁵¹, R²⁵², R²⁵³, R²⁵⁴ are each independently H or C1-C3 alkyl; R²⁵¹ and R²⁵², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy; R²⁵³ and R²⁵⁴, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁶ is C1-C3 alkyl, C1-C3 halogenated alkyl or NR²⁶¹R²⁶², where R²⁶¹, R²⁶² are each independently H or C1-C3 alkyl; or R²⁶¹ and R²⁶², together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁷, R²⁸ are each independently H, C1-C3 alkyl or C1-C3 halogenated alkyl; or R²⁷ and R²⁸, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy;
R²⁹, R³⁰ are each independently H, C1-C3 alkyl or C1-C3 halogenated alkyl; or R²⁹ and R³⁰, together with a nitrogen atom to which they are attached, form a 3-to 6-membered nitrogen-containing heterocyclyl, the 3-to 6-membered nitrogen-containing heterocyclyl is optionally substituted with one or two groups selected from halogen, methyl, trifluoromethyl, methoxy, trifluoromethoxy.

9. The compound, or pharmaceutically acceptable salt, stereoisomer, solvate or prodrug thereof according to claim 1, wherein L₃ is a single bond or O.

10. The compound, or pharmaceutically acceptable salt, stereoisomer, solvate or prodrug thereof according to claim 1, wherein each R⁶ is respectively and independently 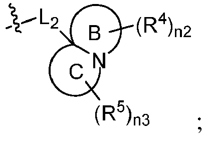 R⁴, R⁵, L₂, ring B, ring C, n2, n3 are each as defined for formula (I).

11. The compound, or pharmaceutically acceptable salt, stereoisomer, solvate or prodrug thereof according to claim 1, wherein R⁶ is selected from a group consisting of:

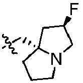

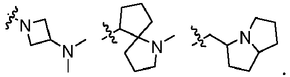

12. The compound, or pharmaceutically acceptable salt, stereoisomer, solvate or prodrug thereof according to claim 1, wherein the compound is selected from a group consisting of:

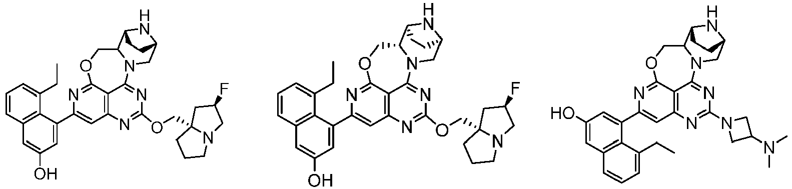

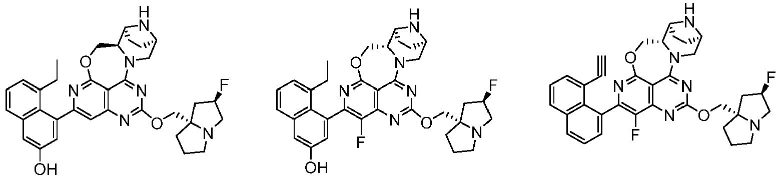

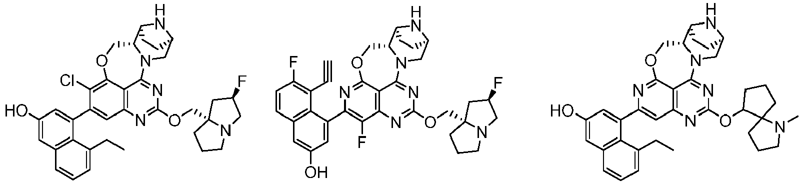

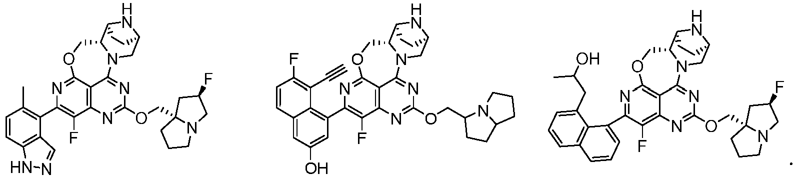

13. A pharmaceutical composition comprising a therapeutically effective amount of the compound, or pharmaceutically acceptable salt, stereoisomer, solvate or prodrug thereof according to any one of claims 1-12; and a pharmaceutically acceptable excipient thereof.

14. Use of the compound, or pharmaceutically acceptable salt, stereoisomer, solvate or prodrug thereof according to any one of claims 1-12 or the pharmaceutical composition according to claim 13 in preparation of a medicament for preventing or treating of a disease or condition, the disease or condition being a KRAS G12D associated disease or disorder.

15. The use according to claim 14, wherein the disease or disorder is KRAS G12D associated cancer; the KRAS G12D associated cancer is selected from a group consisting of: non-small cell lung cancer, small cell lung cancer, colorectal cancer, rectal cancer or pancreatic cancer.
